# EUROPEAN PATENT APPLICATION

(11) **EP 2 003 196 A2**
(43) Date of publication of application: **17.12.2008**
(21) Application number: 08014571.7
(22) Date of filing: 09.06.2004
(51) Int. Cl.: C12N 5/08

(54) **Compositions and methods for treating and diagnosing cancer**

(30) Priority: 09.06.2003 US 477228 P; 09.06.2003 US 477235 P; 09.06.2004 US 864207
(62) Divisional of application: 04776395.8
(71) Applicant: The Regents of the University of Michigan, Ann Arbor, MI 48104-2592 (US)
(72) Inventor: Clarke, Michael F., Palo Alto, CA 94304 (US); Liu, Rui, Ann Arbor, MI 48105 (US)
(74) Representative: King, Hilary Mary

(57) **Abstract**

The present invention relates to compositions and methods for treating, characterizing, and diagnosing cancer. In particular, the present invention provides gene expression profiles associated with solid tumor stem cells, as well as novel stem cell cancer markers useful for the diagnosis, characterization, and treatment of solid tumor stem cells.

## Description

This application claims priority to U.S. Provisional Application Serial No. 60/477,228 filed 06/09/03 and U.S. Provisional Application Serial no. 60/477,235 filed 06/09/03.

This invention was made with government support under Grant No. 5P01CA07513606 awarded by the National Institutes of Health. The Government has certain rights in the invention.

### FIELD OF THE INVENTION

The present invention relates to compositions and methods for treating, characterizing, and diagnosing cancer. In particular, the present invention provides gene expression profiles associated with solid tumor stem cells, as well as novel stem cell cancer markers useful for the diagnosis, characterization, and treatment of solid tumor stem cells.

### BACKGROUND OF THE INVENTION

Breast cancer is the most common female malignancy in most industrialized countries, as it is estimated to affect about 10% of the female population during their lifespan. Although its mortality has not increased along with its incidence, due to earlier diagnosis and improved treatment, it is still onè125.7e of the predominant causes of death in middle-aged women. Despite earlier diagnosis of breast cancer, about 1-5% of women with newly diagnosed breast cancer have a distant metastasis at the time of the diagnosis. In addition, approximately 50% of the patients with local disease who are primarily diagnosed eventually relapse with the metastasis. Eighty-five percent of these recurrences take place within the first five years after the primary manifestation of the disease.

On presentation, most patients with metastatic breast cancer have only one or two organ systems involved. As the disease progresses over time, multiple sites usually become involved. Indeed, metastases may be found in nearly every organ of the body at autopsy. The most common sites of metastatic involvement observed are locoregional recurrences in the skin and soft tissues of the chest wall, as well as in axilla, and supraclavicular area. The most common site for distant metastasis is the bone (30 - 40% of distant metastasis), followed by lung and liver. Metastatic breast cancer is generally considered to be an incurable disease. However, the currently available treatment options often prolong the disease-free state and overall survival rate, as well as increase the quality of the life. The median survival from the manifestation of distant metastases is about three years.

Although great strides have been made understanding the genetic changes that lead to cancer (e.g. breast cancer), the lack of reliable tumor assay for de novo human cancer cells has hindered the ability to undertand the effects of these mutations at the cellular level. Also, the lack of identified cancer markers for solid tumor stem cells has hindered the development of diagnostics and thereapeutics for cancer patients (e.g. breast cancer patients). As such, what is needed is a reliable tumor assay as well as the identification of cancer markers for solid tumor stem cells.

### SUMMARY OF THE INVENTION

The present invention relates to compositions and methods for treating, characterizing, and diagnosing cancer. In particular, the present invention provides gene expression profiles associated with solid tumor stem cells, as well as novel stem cell cancer markers useful for the diagnosis, characterization, and treatment of solid tumor stem cells.

In some embodiments, the present invention provides methods of detecting solid tumor stem cells, comprising; a) providing a tissue sample from a subject, and b) detecting at least one stem cell cancer marker (e.g., 1, 2, 3, 5, 10, ... etc.) from Tables 4-8 in the tissue sample under conditions such that the presence or absence of solid tumor stem cells in the tissue sample is determined. In particular embodimnets, the detecting comprises determining the presence of (or absence of), or an expression level for the at least one stem cell cancer marker. In other embodiments, the detecting comprises detecting mRNA expression of the at least one stem cell cancer marker. In particular embodiments, the detecting comprises exposing the stem cell cancer marker mRNA to a nucleic acid probe complementary to the stem cell cancer marker mRNA.

In certain embodiments, the detecting comprises detecting polypeptide expression of the at least one stem cell cancer marker. In other embodiments, the detecting comprises exposing the stem cell cancer marker polypeptide to an antibody specific to the stem cell cancer marker polypeptide and detecting the binding of the antibody to the stem cell cancer polypeptide. In further embodiments, the subject comprises a human subject. In additional embodiments, the tissue sample comprises tumor tissue. In some embodiments, the tumor tissue sample is a post-surgical tumor tissue sample (e.g. tumor biopsy).

In other embodiments, the methods further comprise c) providing a prognosis to the subject. In some embodiments, the at least one stem cell cancer marker is from Table 8. In preferred embodiments, the at least one stem cell cancer marker comprises: Bmi-1, eed, easyh1, easyh2, mf2, yyl, smarcA3, smarcA5, smarcD3, smarcE1, mllt3, FZD1, FZD2, FZD3, FZD4, FZD6, FZD7, FZD8, FZD9, FZD10, WNT2, WNT2B, WNT3, WNT5A, WNT10B, WNT16, AXIN1, BCL9, MYC, and (TCF4).

In particular embodiments, the present invention provides methods for reducing the size of a solid tumor (e.g. in research drug screening, or therapeutic applications) comprising contacting cells of a solid tumor with a biologically (e.g. therapeutically) effective amount of a composition comprising at least one agent directed against at least one stem cell cancer marker shown in Tables 4-8. In some embodiments, the biologically effective amount is an amount sufficient to cause cell death of or inhibit proliferation of solid tumor stem cells in the solid tumor. In other embodiments, the biologically effective amount is an amount interferences with the survival pathyways (e.g. notch related genes) or self-renewal pathaways (e.g. WNT pathways) of the solid tumor stem cell.

Examples of solid tumors from which solid tumor stem cells can be isolated or enriched for according to the invention include, but are not limited to, sarcomas and carcinomas such as, but not limited to: fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma. The invention is applicable to sarcomas and epithelial cancers, such as ovarian cancers and breast cancers.

In additional embodiments, the at least one agent is an antibody, peptide or small molecule. In further embodiments, the antibody, peptide, anti-sense, siRNA, or small molecule is directed against an extracellular domain of the at least one stem cell cancer marker. In some embodiments, the at least one stem cell cancer marker is selected from the group consisting of: Bmi-1, eed, easyh1, easyh2, mf2, yyl, smarcA3, smarcA5, smarcD3, smarcE1, mllt3, FZD1, FZD2, FZD3, FZD4, FZD6, FZD7, FZD8, FZD9, FZD10, WNT2, WNT2B, WNT3, WNT5A, WNT10B, WNT16, AXINI, BCL9, MYC, and (TCF4).

In other embodiments, the present invention provides methods for reducing the size of a solid tumor, comprising contacting cells of a solid tumor with a biologically (e.g. therapeutically) effective amount of a composition comprising at least one agent that modulates the activity of at least one stem cell cancer marker shown in Tables 4-8. In some embodiments, the present invention provides methods for killing or inhibiting the proliferation of solid tumor stem cells comprising contacting the solid tumor stem cells with a biologically effective amount of a composition comprising at least one agent targeted to at least one stem cell cancer marker shown in Tables 4-8. In certain embodiments, the methods further comprise identifying the death of or the prevention of the growth of the solid tumor stem cells following the contacting. In additional embodiments, the cell death is caused by apoptosis. In other embodiments, the biologically effective amount is an amount interferences with the survival pathyways (e.g. notch related genes) or self-renewal pathaways (e.g. WNT pathways) of the solid tumor stem cell. In other embodiments, the at least one stem cell cancer marker is selected from the group consisting of: Bmi-1, eed, easyh1, easyh2, mf2, yy1, smarcA3, smarcA5, smarcD3, smarcE1, mllt3, FZD1, FZD2, FZD3, FZD4, FZD6, FZD7, FZD8, FZD9, FZD10, WNT2, WNT2B, WNT3, WNT5A, WNT10B, WNT16, AXIN1, BCL9, MYC, and (TCF4).

In particular embodiments, the solid tumor stem cells express cell surface marker CD44, ESA, or B38.1. In other embodiments, the solid tumor stem cells fail to express at least one LINEAGE marker selected from the group consisting of CD2, CD3, CD IO, CD 14, CD16, CD31, CD45, CD64, and CD140b (see,e.g., US Pat. Pub. US20040037815A1, and US20020119565, both of which are herein incorporated by reference).

In other embodiments, the present invention provides methods for selectively targeting a solid tumor stem cell comprising, (a) identifying at least one stem cell cancer marker from Tables 4-8 present on a solid tumor stem cell; and (b) obtaining an agent or set of agents that selectively binds to or regulates the at least one stem cell cancer marker. In some embodiments, the agent genetically modifies the solid tumor stem cell. In particular embodiments, the agent comprises a bi-specific conjugate. In further embodiments, the agent comprises an adenoviral vector.

In some embodiments, the present invention provides methods for forming a tumor in an animal, comprising: introducing purified solid tumor stem cells (e.g. a cell dose of) into an animal, wherein: (a) the solid tumor stem cells are derived from a solid tumor; and (b) the solid tumor stem cells are enriched at least 2-fold relative to unfractionated tumor cells based on the presence of at least one stem cell cancer marker in Tables 4-8. In other embodiments, the animal is an immunocompromised animal. In certain embodiments, the animal is an immunocompromised mammal, such as a mouse (e.g., a nude mouse, SCID mouse, NOD/SCID mouse, Beige/SCID mouse; and microglobin deficient NOD/SCID mouse). In particular embodiments, the number of cells in the cell dose is between about 100 cells and about 5x10⁵ cells.

In certain embodiments, the present invention provides kits for detecting solid tumor stem cells in a subject, comprising: a) a reagent capable of specifically detecting at least one stem cell cancer marker from Tables 4-8 in a tissue or cell sample from a subject, and, optionally, b) instructions for using the reagent for detecting the presence or absence of solid tumor stem cells in the tissue sample. In further embodiments, the reagent comprises a nucleic acid probe complementary to mRNA from the at least one stem cell cancer marker. In other embodiments, the reagent comprises an antibody or antibody fragment.

In some embodiments, the present invention provides methods of screening compounds, comprising: a) providing; i) a solid tumor stem cell; and ii) one or more test compounds; and b) contacting the solid tumor stem cell with the test compound; and c) detecting a change in expression of at least one stem cell cancer marker shown in Tables 4-8 in the presence of the test compound relative to the absence of the test compound. In particular embodiments, the detecting comprises determining an expression level for the at least one stem cell cancer marker. In particular embodiments, the detecting comprises detecting mRNA expression of the at least one stem cell cancer marker. In some embodiments, the detecting comprises detecting polypeptide expression of the at least one stem cell cancer marker. In additional embodiments, the solid tumor stem cell is *in vitro.* In other embodiments, the solid tumor stem cell is *in vivo.* In further embodiments, the test compound comprises a drug (e.g. small molecule, antibody, antibody-toxin conjugate, siRNA, etc.).

In some embodiments, the present invention provides compositions comprising at least two agents (e.g. small molecule, antibody, antibody-toxin conjugate, siRNA, etc.), wherein each of the agents modulates the activity of at least one stem cell cancer marker shown in Tables 4-8. In additional embodiments, the composition comprises at least three agents.

In particular embodiments, the present invention provides methods of distinguishing tumorigenic from non-tumorigenic cancer cells, comprising: detecting the presence of β-catenin in a cancer cell such that the localization of β -catenin in the cancer cell is determined to be primarily nuclear or primarily cytoplasmic. In some embodiments, the method further comprises identifying the cancer cell as tumorigenic if the β -catenin localization is primarily nuclear, or identifying the cancer cell as non-tumorigenic if the β -catenin localization is primarily cytoplasmic.

In certain embodiments, the present invention provides methods of distinguishing a tumorigenic from a non-tumorigenic cancer cell, comprising; a) providing; i) a cancer cell, and ii) a composition comprising an agent configured to bind β-catenin; and b) contacting the cancer cell with the composition under conditions such that the localization of β -catenin in the cancer cell is determined to be primarily nuclear or primarily cytoplasmic, and c) identifying the cancer cell as tumorigenic if the β -catenin localization is primarily nuclear, or identifying the cancer cell as non-tumorigenic if the β -catenin localization is primarily cytoplasmic.

### DESCRIPTION OF THE FIGURES

Figure 1 shows isolation of tumorigenic cells.
Figure 2 shows the DNA content of tumorigenic and non-tumorigenic breast cancer cells.

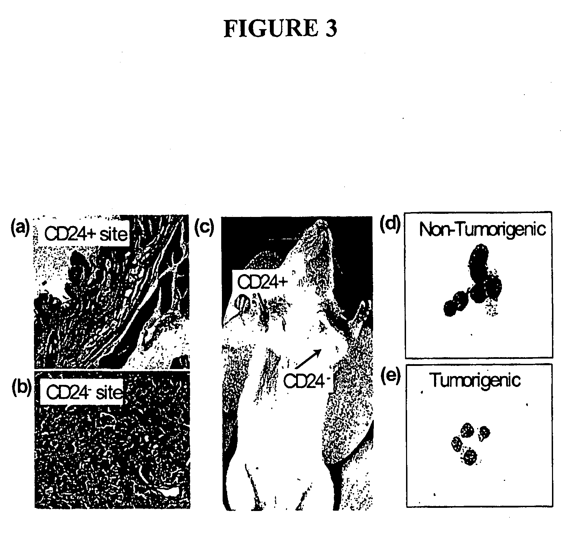

Figure 3 shows histology from the CD24⁺ injection site (a), (20x objective magnification) revealed only normal mouse tissue while the CD24⁻ /low injection site (b), (40x objective magnification) contained malignant cells. (c) A representative tumor in a mouse at the CD44⁺CD24^{-/low}Lineage⁻ injection site, but not at the CD44⁺CD24⁺Lineage⁻ injection site. T3 cells were stained with Papanicolaou stain and examined microscopically (100x objective). Both the non-tumorigenic (c) and tumorigenic (d) populations contained cells with a neoplastic appearance, with large nuclei and prominent nucleoli.
Figure 4 shows the phenotypic diversity in tumors arising from CD44+CD24-/lowLineage- cells.
Figure 5 shows the expression of Wnt (left panel) and Frizzled (right panel).
Figure 6 shows the isolation of normal tumor fibroblasts and endothelial cells.
Figures 7 shows infection of breast cancer stem cells with an adenovirus vector.
Figure 8 shows subcellular localization of β-catenin.
Figure 9 shows inhibition of β-catenin signaling in cancer cells.

### GENERAL DESCRIPTION

The present invention relates to compositions and methods for treating, characterizing and diagnosing cancer. In particular, the present invention provides gene expression profiles associated with solid tumor stem cells, as well as novel markers useful for the diagnosis, characterization, and treatment of solid tumor stem cells. Suitable markers that may be targeted (e.g. for diagnostic or therapeutic purposes) are the genes and peptides encoded by the genes that are differentially expressed in solid tumor stem cells as shown in Tables 4-8. The differentially expressed genes, and the peptides encoded thereby, may be detected (e.g. quantitatively) in order to identify the presence of solid tumor stem cells, and to determine and screen molecules suitable for reducing the proliferation (or killing), interfering with self-renewal pathways, or interfering with survival pathways of any solid tumor stem cells that are present. The differentially expressed genes, and peptides encoded thereby, shown in these tables are also useful for generating therapeutic agents targeted to one or more of these markers (e.g. to inhibit or promote the activity of the marker).

In order to identify solid tumor stem cell markers, cells from 5 patients, AML stem cells and non-tumorigenic cancer cells from 6 patients, normal hematopoietic stem cells (HSC5), normal hematopoietic cells, normal colon epithelial cells, and normal breast epithelial cells were analyszed for differential expression.

The present invention also provides solid tumor stem cells that differentially express from other cells one or more of the markers provided in Tables 4-8. The solid tumor stem cells can be human or other animal. The expression can be either to a greater extent or to a lesser extent. The other cells can be selected from normal cells, hematopoietic stem cells, acute myelogenous leukemia (AML) stem cells, or any other class of cells.

The invention provides a method of selecting cells of a population, which results in a purified population of solid tumor stem cells (e.g. from a patient to select or test therapetuic agents are preferred for the patient). The present invention also provides a method of selecting a purified population of tumor cells other than solid tumor stem cells, such as a population of non-tumorigenic (NTG) tumor cells. The present invention provides methods of raising antibodies to the selected cells. The invention provides diagnostic methods using the selected cells. The invention also provides therapeutic methods, where the therapeutic is directed to a solid tumor stem cell (e.g. directed to one of the stem cells cancer markers identified herein directly or indirectly).

Accordingly, the invention provides methods of selecting cells, diagnosing disease, conducting research studies, and treating solid tumors using selection methods, diagnostic methods and therapeutics directed to specific genes on a given pathway. Included are one or more of the following genes and gene products: Bmi-1, eed, easyhi, easyh2, mf2, yyl, smarcA3, smarcA5, smarcD3, smarcE 1 and mllt3, as well as those shown in Tables 4-8. Many of these genes are differentially expressed in solid tumor stem cells as compared with normal cells and non-tumorigenic cancer cells, as shown herein.

The invention provides in vivo and in vitro assays of solid tumor stem cell function and cell function by the various populations of cells isolated from a solid tumor. The invention provides methods for using the various populations of cells isolated from a solid tumor (such as a population of cells enriched for solid tumor stem cells) to identify factors influencing solid tumor stem cell proliferation. By the methods of the present invention, one can characterize the phenotypically heterogeneous populations of cells within a solid tumor. In particular, one can identify, isolate, and characterize a phenotypically distinct cell population within a tumor having the stem cell properties of extensive proliferation and the ability to give rise to all other tumor cell types. Solid tumor stem cells are the tumorigenic cells that are capable of re-establishing a tumor following treatment.

The invention thus provides a method for selectively targeting diagnostic or therapeutic agents to solid tumor stem cells. The invention also provides an agent, such as a biomolecule, that is selectively targeted to solid tumor stem cells (e.g. directed to one of the solid tumor stem cell cancer markers disclosed herein). In preferred embodiments, the stem cell cancer marker this targeted is part of a self-renewal or cell survival pathway. One example of such a marker is Bmi-1, which was shown to be required for maintenance of adult self-renewing heamatopoietic stem cells (see, e.g., Park et al., Nature, 2003 May 15;423(6937):302-5, herein incorporated by reference).

In certain embodiments, the present invention provides methods for screening for anti-cancer agents; for the testing of anti-cancer therapies; for the development of drugs targeting novel pathways; for the identification of new anti-cancer therapeutic targets; the identification and diagnosis of malignant cells in pathology specimens; for the testing and assaying of solid tumor stem cell drug sensitivity; for the measurement of specific factors that predict drug sensitivity; and for the screening of patients (e.g., as an adjunct for mammography).

Other features, objects, and advantages of the invention will be apparent from the detailed description below. Additional guidance is provided in Published PCT patent application WO 02/12447 by the Regents of the University of Michigan and PCT patent application PCT/USO2/39191 by the Regents of the University of Michigan, both of which are incorporated herein by reference.

### DEFINITIONS

To facilitate an understanding of the present invention, a number of terms and phrases are defined below:

As used here, the term "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity (e.g. able to bind a stem cell cancer marker as described herein). Antibodies may be conjugated to other molecules (e.g., toxins).

As used herein, the term "antibody fragments" refers to a portion of an intact antibody. Examples of antibody fragments include, but are not limited to, linear antibodies; single-chain antibody molecules; Fc or Fc' peptides, Fab and Fab fragments, and multispecific antibodies formed from antibody fragments.

As used herein, "humanized" forms of non-human (e.g., murine) antibodies are chimeric antibodies that contain minimal sequence, or no sequence, derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues that are not found in the recipient antibody or in the donor antibody. These modifications are generally made to further refine antibody performance. In general, the humanized antibody will comprise substantially all of at least one, and typically two, variable domains, in which all or substantially all of the hypervariable loops correspond to those of a nonhuman immunoglobulin and all or substantially all of the FR residues are those of a human immunoglobulin sequence. The humanized antibody may also comprise at least a portion of an immunoglobulin constant region (Fc), typically that of a human immunoglobulin. Examples of methods used to generate humanized antibodies are described in U.S. Pat. 5,225,539 to Winter et al. (herein incorporated by reference).

"Enriched", as in an enriched population of cells, can be defined phenotypically based upon the increased number of cells having a particular marker (e.g. as shown in Tables 4-8) in a fractionated set of cells as compared with the number of cells having the marker in the unfractionated set of cells. However, the term "enriched can be preferably defined fucntionally by tumorigenic function as the minimum number of cells that form tumors at limit dilution frequency in test mice. For example, if 500 tumor stem cells form tumors in 63% of test animals, but 5000 unfractionated tumor cells are required to form tumors in 63% of test animals, then the solid tumor stem cell population is 10-fold enriched for tumorigenic activity. The stem cell cancer markers of the present invention can be used to generate enriched populations of cancer stem cells. In preferred embodiments, the stem cell population is enriched at least 1.4 fold relative to unfractioned tumor cells (e.g. 1.4 fold, 1.5 fold, 2 fold, 5 fold ....20 fold).

"Isolated" in regard to cells, refers to a cell that is removed from its natural environment (such as in a solid tumor) and that is isolated or separated, and is at least about 30%, 50%, 75% free, and most preferably about 90% free, from other cells with which it is naturally present, but which lack the marker based on which the cells were isolated. The stem cell cancer markers of the present invention can be used to generate isolated populations of cancer stem cells.

As used herein, the term "receptor binding domain" refers to any native ligand for a receptor, including cell adhesion molecules, or any region or derivative of such native ligand retaining at least a qualitative receptor binding ability of a corresponding native ligand.

As used herein, the term "antibody-immunoadhesin chimera" comprises a molecule that combines at least one binding domain of an antibody with at least one immunoadhesin. Examples include, but are not limited to, the bispecific CD4-IgG chimeras described in Berg et al., PNAS (USA) 88:4723-4727 (1991) and Charnow et al., J. Immunol., 153:4268 (1994), both of which are hereby incorporated by reference.

As used herein, the terms "cancer" and "cancerous" refer to or describe the physiological condition in mammals that is typically characterized by unregulated cell growth. Examples of cancer include, but are not limited to, carcinoma, lymphoma, blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma and various types of head and neck cancer.

The term "epitope" as used herein refers to that portion of an antigen that makes contact with a particular antibody.

When a protein or fragment of a protein is used to immunize a host animal, numerous regions of the protein may induce the production of antibodies which bind specifically to a given region or three-dimensional structure on the protein; these regions or structures are referred to as "antigenic determinants". An antigenic determinant may compete with the intact antigen (i.e., the "immunogen" used to elicit the immune response) for binding to an antibody.

The terms "specific binding" or "specifically binding" when used in reference to the interaction of an antibody and a protein or peptide means that the interaction is dependent upon the presence of a particular structure (i.e., the antigenic determinant or epitope) on the protein; in other words the antibody is recognizing and binding to a specific protein structure rather than to proteins in general. For example, if an antibody is specific for epitope "A," the presence of a protein containing epitope A (or free, unlabelled A) in a reaction containing labeled "A" and the antibody will reduce the amount of labeled A bound to the antibody.

As used herein, the terms "non-specific binding" and "background binding" when used in reference to the interaction of an antibody and a protein or peptide refer to an interaction that is not dependent on the presence of a particular structure (i.e., the antibody is binding to proteins in general rather that a particular structure such as an epitope).

As used herein, the term "subject" refers to any animal (*e.g*., a mammal), including, but not limited to, humans, non-human primates, rodents, and the like, which is to be the recipient of a particular treatment. Typically, the terms "subject" and "patient" are used interchangeably herein in reference to a human subject.

As used herein, the term "subject suspected of having cancer" refers to a subject that presents one or more symptoms indicative of a cancer (e.g., a noticeable lump or mass) or is being screened for a cancer (e.g., during a routine physical). A subject suspected of having cancer may also have one or more risk factors. A subject suspected of having cancer has generally not been tested for cancer. However, a "subject suspected of having cancer" encompasses an individual who has received an initial diagnosis but for whom the stage of cancer is not known. The term further includes people who once had cancer (*e.g*., an individual in remission).

As used herein, the term "subject at risk for cancer" refers to a subject with one or more risk factors for developing a specific cancer. Risk factors include, but are not limited to, gender, age, genetic predisposition, environmental expose, previous incidents of cancer, preexisting non-cancer diseases, and lifestyle.

As used herein, the term "characterizing cancer in subject" refers to the identification of one or more properties of a cancer sample in a subject, including but not limited to, the presence of benign, pre-cancerous or cancerous tissue, the stage of the cancer, and the subject's prognosis. Cancers may be characterized by the identification of the expression of one or more cancer marker genes, including but not limited to, the cancer markers disclosed herein.

As used herein, the term "stem cell cancer markers" refers to a gene or peptide expressed by the gene whose expression level, alone or in combination with other genes, is correlated with the presence of tumorigenic cancer cells. The correlation may relate to either an increased or decreased expression of the gene (e.g. increased or decreased levels of mRNA or the peptide encoded by the gene).

As used herein, the term "a reagent that specifically detects expression levels" refers to reagents used to detect the expression of one or more genes (e.g., including but not limited to, the cancer markers of the present invention). Examples of suitable reagents include but are not limited to, nucleic acid probes capable of specifically hybridizing to the gene of interest, aptamers, PCR primers capable of specifically amplifying the gene of interest, and antibodies capable of specifically binding to proteins expressed by the gene of interest. Other non-limiting examples can be found in the description and examples below.

As used herein, the term "detecting a decreased or increased expression relative to non-cancerous control" refers to measuring the level of expression of a gene (*e.g*., the level of mRNA or protein) relative to the level in a non-cancerous control sample. Gene expression can be measured using any suitable method, including but not limited to, those described herein.

As used herein, the term "detecting a change in gene expression in a a cell sample in the presence of said test compound relative to the absence of said test compound" refers to measuring an altered level of expression (*e.g*., increased or decreased) in the presence of a test compound relative to the absence of the test compound. Gene expression can be measured using any suitable method.

As used herein, the term "instructions for using said kit for detecting cancer in said subject" includes instructions for using the reagents contained in the kit for the detection and characterization of cancer in a sample from a subject.

As used herein, the term "providing a prognosis" refers to providing information regarding the impact of the presence of cancer (*e.g*., as determined by the diagnostic methods of the present invention) on a subject's future health (*e.g*., expected morbidity or mortality, the likelihood of getting cancer, and the risk of metastasis).

As used herein, the term "post surgical tumor tissue" refers to cancerous tissue (*e.g*., biopsy tissue) that has been removed from a subject (*e.g*., during surgery).

As used herein, the term "subject diagnosed with a cancer" refers to a subject who has been tested and found to have cancerous cells. The cancer may be diagnosed using any suitable method, including but not limited to, biopsy, x-ray, blood test, and the diagnostic methods of the present invention.

As used herein, the term "biopsy tissue" refers to a sample of tissue that is removed from a subject for the purpose of determining if the sample contains cancerous tissue. In some embodiment, biopsy tissue is obtained because a subject is suspected of having cancer. The biopsy tissue is then examined for the presence or absence of cancer.

As used herein, the term "gene transfer system" refers to any means of delivering a composition comprising a nucleic acid sequence to a cell or tissue. For example, gene transfer systems include, but are not limited to, vectors (*e.g*., retroviral, adenoviral, adeno-associated viral, and other nucleic acid-based delivery systems), microinjection of naked nucleic acid, polymer-based delivery systems (*e.g*., liposome-based and metallic particle-based systems), biolistic injection, and the like. As used herein, the term "viral gene transfer system" refers to gene transfer systems comprising viral elements (*e.g*., intact viruses, modified viruses and viral components such as nucleic acids or proteins) to facilitate delivery of the sample to a desired cell or tissue. As used herein, the term "adenovirus gene transfer system" refers to gene transfer systems comprising intact or altered viruses belonging to the family Adenoviridae.

As used herein, the term "site-specific recombination target sequences" refers to nucleic acid sequences that provide recognition sequences for recombination factors and the location where recombination takes place.

As used herein, the term "nucleic acid molecule" refers to any nucleic acid containing molecule, including but not limited to, DNA or RNA. The term encompasses sequences that include any of the known base analogs of DNA and RNA including, but not limited to, 4-acetylcytosine, 8-hydroxy-N6-methyladenosine, aziridinylcytosine, pseudoisocytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

The term "gene" refers to a nucleic acid (e.g., DNA) sequence that comprises coding sequences necessary for the production of a polypeptide, precursor, or RNA (e.g., rRNA, tRNA). The polypeptide can be encoded by a full length coding sequence or by any portion of the coding sequence so long as the desired activity or functional properties (*e.g*., enzymatic activity, ligand binding, signal transduction, immunogenicity, etc.) of the full-length or fragment are retained. The term also encompasses the coding region of a structural gene and the sequences located adjacent to the coding region on both the 5' and 3' ends for a distance of about 1 kb or more on either end such that the gene corresponds to the length of the full-length mRNA. Sequences located 5' of the coding region and present on the mRNA are referred to as 5' non-translated sequences. Sequences located 3' or downstream of the coding region and present on the mRNA are referred to as 3' non-translated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "heterologous gene" refers to a gene that is not in its natural environment. For example, a heterologous gene includes a gene from one species introduced into another species. A heterologous gene also includes a gene native to an organism that has been altered in some way (*e.g*., mutated, added in multiple copies, linked to non-native regulatory sequences, etc). Heterologous genes are distinguished from endogenous genes in that the heterologous gene sequences are typically joined to DNA sequences that are not found naturally associated with the gene sequences in the chromosome or are associated with portions of the chromosome not found in nature (*e.g*., genes expressed in loci where the gene is not normally expressed).

As used herein, the term "gene expression" refers to the process of converting genetic information encoded in a gene into RNA (*e.g*., mRNA, rRNA, tRNA, or snRNA) through "transcription" of the gene (*e.g*., via the enzymatic action of an RNA polymerase), and for protein encoding genes, into protein through "translation" of mRNA. Gene expression can be regulated at many stages in the process. "Up-regulation" or "activation" refers to regulation that increases the production of gene expression products (*e.g*., RNA or protein), while "down-regulation" or "repression" refers to regulation that decrease production. Molecules (*e.g*., transcription factors) that are involved in up-regulation or down-regulation are often called "activators" and "repressors," respectively.

In addition to containing introns, genomic forms of a gene may also include sequences located on both the 5' and 3' end of the sequences that are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers that control or influence the transcription of the gene. The 3' flanking region may contain sequences that direct the termination of transcription, post-transcriptional cleavage and polyadenylation.

The term "siRNAs" refers to short interfering RNAs. In some embodiments, siRNAs comprise a duplex, or double-stranded region, of about 18-25 nucleotides long; often siRNAs contain from about two to four unpaired nucleotides at the 3' end of each strand. At least one strand of the duplex or double-stranded region of a siRNA is substantially homologous to or substantially complementary to a target RNA molecule. The strand complementary to a target RNA molecule is the "antisense strand;" the strand homologous to the target RNA molecule is the "sense strand," and is also complementary to the siRNA antisense strand. siRNAs may also contain additional sequences; non-limiting examples of such sequences include linking sequences, or loops, as well as stem and other folded structures. siRNAs appear to function as key intermediaries in triggering RNA interference in invertebrates and in vertebrates, and in triggering sequence-specific RNA degradation during posttranscriptional gene silencing in plants.

The term "RNA interference" or "RNAi" refers to the silencing or decreasing of gene expression by siRNAs. It is the process of sequence-specific, post-transcriptional gene silencing in animals and plants, initiated by siRNA that is homologous in its duplex region to the sequence of the silenced gene. The gene may be endogenous or exogenous to the organism, present integrated into a chromosome or present in a transfection vector that is not integrated into the genome. The expression of the gene is either completely or partially inhibited. RNAi may also be considered to inhibit the function of a target RNA; the function of the target RNA may be complete or partial.

As used herein, the terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence.

As used herein, the terms "an oligonucleotide having a nucleotide sequence encoding a gene" and "polynucleotide having a nucleotide sequence encoding a gene," means a nucleic acid sequence comprising the coding region of a gene or in other words the nucleic acid sequence that encodes a gene product. The coding region may be present in a cDNA, genomic DNA or RNA form. When present in a DNA form, the oligonucleotide or polynucleotide may be single-stranded (i.e., the sense strand) or double-stranded. Suitable control elements such as enhancers/promoters, splice junctions, polyadenylation signals, etc. may be placed in close proximity to the coding region of the gene if needed to permit proper initiation of transcription and/or correct processing of the primary RNA transcript. Alternatively, the coding region utilized in the expression vectors of the present invention may contain endogenous enhancers/promoters, splice junctions, intervening sequences, polyadenylation signals, etc. or a combination of both endogenous and exogenous control elements.

As used herein the term "portion" when in reference to a nucleotide sequence (as in "a portion of a given nucleotide sequence") refers to fragments of that sequence. The fragments may range in size from four nucleotides to the entire nucleotide sequence minus one nucleotide (10 nucleotides, 20, 30, 40, 50, 100, 200, etc.).

The terms "in operable combination," "in operable order," and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

The term "isolated" when used in relation to a nucleic acid, as in "an isolated oligonucleotide" or "isolated polynucleotide" refers to a nucleic acid sequence that is identified and separated from at least one component or contaminant with which it is ordinarily associated in its natural source. Isolated nucleic acid is such present in a form or setting that is different from that in which it is found in nature. In contrast, non-isolated nucleic acids as nucleic acids such as DNA and RNA found in the state they exist in nature. For example, a given DNA sequence (*e.g*., a gene) is found on the host cell chromosome in proximity to neighboring genes; RNA sequences, such as a specific mRNA sequence encoding a specific protein, are found in the cell as a mixture with numerous other mRNAs that encode a multitude of proteins. However, isolated nucleic acid encoding a given protein includes, by way of example, such nucleic acid in cells ordinarily expressing the given protein where the nucleic acid is in a chromosomal location different from that of natural cells, or is otherwise flanked by a different nucleic acid sequence than that found in nature. The isolated nucleic acid, oligonucleotide, or polynucleotide may be present in single-stranded or double-stranded form. When an isolated nucleic acid, oligonucleotide or polynucleotide is to be utilized to express a protein, the oligonucleotide or polynucleotide will contain at a minimum the sense or coding strand (*i.e*., the oligonucleotide or polynucleotide may be single-stranded), but may contain both the sense and anti-sense strands (*i.e*., the oligonucleotide or polynucleotide may be double-stranded).

"Amino acid sequence" and terms such as "polypeptide" or "protein" are not meant to limit the amino acid sequence to the complete, native amino acid sequence associated with the recited protein molecule.

The term "native protein" as used herein to indicate that a protein does not contain amino acid residues encoded by vector sequences; that is, the native protein contains only those amino acids found in the protein as it occurs in nature. A native protein may be produced by recombinant means or may be isolated from a naturally occurring source.

As used herein the term "portion" when in reference to a protein (as in "a portion of a given protein") refers to fragments of that protein. The fragments may range in size from four amino acid residues to the entire amino acid sequence minus one amino acid.

The term "Southern blot," refers to the analysis of DNA on agarose or acrylamide gels to fractionate the DNA according to size followed by transfer of the DNA from the gel to a solid support, such as nitrocellulose or a nylon membrane. The immobilized DNA is then probed with a labeled probe to detect DNA species complementary to the probe used. The DNA may be cleaved with restriction enzymes prior to electrophoresis. Following electrophoresis, the DNA may be partially depurinated and denatured prior to or during transfer to the solid support. Southern blots are a standard tool of molecular biologists (J. Sambrook et al., Molecular Cloning: A Laboratory Manual, Cold Spring Harbor Press, NY, pp 9.31-9.58 [1989]).

The term "Northern blot," as used herein refers to the analysis of RNA by electrophoresis of RNA on agarose gels to fractionate the RNA according to size followed by transfer of the RNA from the gel to a solid support, such as nitrocellulose or a nylon membrane. The immobilized RNA is then probed with a labeled probe to detect RNA species complementary to the probe used. Northern blots are a standard tool of molecular biologists (J. Sambrook, *et al., supra,* pp 7.39-7.52 [1989]).

The term "Western blot" refers to the analysis of protein(s) (or polypeptides) immobilized onto a support such as nitrocellulose or a membrane. The proteins are run on acrylamide gels to separate the proteins, followed by transfer of the protein from the gel to a solid support, such as nitrocellulose or a nylon membrane. The immobilized proteins are then exposed to antibodies with reactivity against an antigen of interest. The binding of the antibodies may be detected by various methods, including the use of radiolabeled antibodies.

The term "transgene" as used herein refers to a foreign gene that is placed into an organism by, for example, introducing the foreign gene into newly fertilized eggs or early embryos. The term "foreign gene" refers to any nucleic acid (*e.g*., gene sequence) that is introduced into the genome of an animal by experimental manipulations and may include gene sequences found in that animal so long as the introduced gene does not reside in the same location as does the naturally occurring gene.

As used herein, the term "vector" is used in reference to nucleic acid molecules that transfer DNA segment(s) from one cell to another. The term "vehicle" is sometimes used interchangeably with "vector." Vectors are often derived from plasmids, bacteriophages, or plant or animal viruses.

The term "expression vector" as used herein refers to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals.

The terms "overexpression" and "overexpressing" and grammatical equivalents, are used in reference to levels of mRNA to indicate a level of expression approximately 1.5-fold higher (or greater) than that observed in a given tissue in a control or non-transgenic animal. Levels of mRNA are measured using any of a number of techniques known to those skilled in the art including, but not limited to Northern blot analysis. Appropriate controls are included on the Northern blot to control for differences in the amount of RNA loaded from each tissue analyzed (*e.g*., the amount of 28S rRNA, an abundant RNA transcript present at essentially the same amount in all tissues, present in each sample can be used as a means of normalizing or standardizing the mRNA-specific signal observed on Northern blots). The amount of mRNA present in the band corresponding in size to the correctly spliced transgene RNA is quantified; other minor species of RNA which hybridize to the transgene probe are not considered in the quantification of the expression of the transgenic mRNA.

As used herein, the term *"in vitro"* refers to an artificial environment and to processes or reactions that occur within an artificial environment. In vitro environments can consist of, but are not limited to, test tubes and cell culture. The term *"in vivo"* refers to the natural environment (*e.g.*, an animal or a cell) and to processes or reaction that occur within a natural environment.

The terms "test compound" and "candidate compound" refer to any chemical entity, pharmaceutical, drug, and the like that is a candidate for use to treat or prevent a disease, illness, sickness, or disorder of bodily function (*e.g*., cancer). Test compounds comprise both known and potential therapeutic compounds. A test compound can be determined to be therapeutic by screening using the screening methods of the present invention. In some embodiments of the present invention, test compounds include antisense compounds.

As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Biological samples include blood products, such as plasma, serum and the like. Environmental samples include environmental material such as surface matter, soil, water, crystals and industrial samples. Such examples are not however to be construed as limiting the sample types applicable to the present invention.

### DETAILED DESCRIPTION OF THE INVENTION

The present invention provides compositions and methods for treating, characterizing, and diagnosing cancer. In particular, the present invention provides gene expression profiles associated with solid tumor stem cells, as well as novel markers useful for the diagnosis, characterization, and treatment of solid tumor stem cells.

### I. Stem Cells and Solid Tumor Stem Cells

Common cancers arise in tissues that contain a large sub-population of proliferating cells that are responsible for replenishing the short-lived mature cells. In such organs, cell maturation is arranged in a hierarchy in which a rare population of stem cells give rise to the mature cells and perpetuate themselves through a process called self renewal¹⁻¹¹. Due to their rarity, stem cells should be isolated in order to study their biological, molecular, and biochemical properties. Although it is likely that they give rise to most tissues, stem cells have been rigorously identified and purified in only a few tissues. The stem cells that give rise to the lymphohematopoietic system, called hematopoietic stem cells (HSCs), have been isolated from mice and humans and are the best characterized stem cells. The utility of tissue containing HSCs has been demonstrated in cancer therapy with their extensive use for bone marrow transplantation to regenerate the hematolymphoid system following myeloablative protocols¹². The prospective isolation of HSCs from patients can result in a population that is cancer free for autologous transplantation¹³⁻¹⁷.

Understanding the cellular biology of the tissues in which cancers arise, and specifically of the stem cells residing in those tissues, provides new insights into cancer biology. Several aspects of stem cell biology are relevant to cancer. First, both normal stem cells and cancer stem cells undergo self-renewal, and emerging evidence suggests that similar molecular mechanisms regulate self-renewal in normal stem cells and their malignant counterparts. Next, it is quite likely that mutations that lead to cancer accumulate in normal stem cells. Finally, it is likely that tumors contain a "cancer stem cell" population with indefinite proliferative potential that drives the growth and metastasis of tumors¹⁸⁻²⁶.

HSCs are the most studied and best understood somatic stem cell population'. Hematopoiesis is a tightly regulated process in which a pool of hematopoietic stem cells eventually gives rise to the lymphohematopoietic system consisting of the formed blood elements, e.g., red blood cells, platelets, granulocytes, macrophages, and B-and T-lymphocytes. These cells are important for oxygenation, prevention of bleeding, immunity, and infections, respectively. In the adult, HSCs have two fundamental properties. First, HSCs need to self-renew in order to maintain the stem cell pool; the total number of HSCs is under strict genetic regulation²⁷. Second, they must undergo differentiation to maintain a constant pool of mature cells in normal conditions, and to produce increased numbers of a particular lineage in response to stresses such as bleeding or infection.

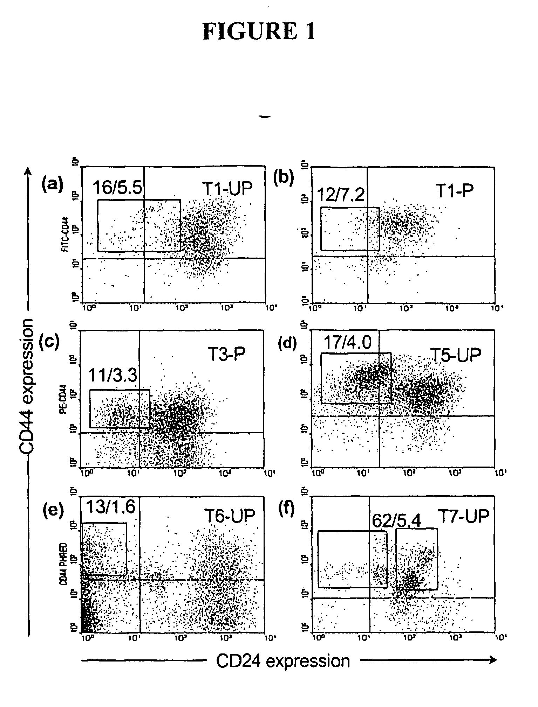

In the hematopoietic system, multipotent cells constitute 0.05% of mouse bone marrow cells and are heterogeneous with respect to their ability to self-renew. There are three different populations of multipotent cells: long-term self-renewing HSCs, short-term self-renewing HSCs, and multipotent progenitors without detectable self-renewal potential^{7,28}. These populations form a hierarchy in which the long-term HSCs give rise to short-term HSCs, which in turn give rise to multipotent progenitors [Fig. 1 in⁷]. As HSCs mature from the long-term self-renewing pool to multipotent progenitors they become more mitotically active but lose the ability to self-renew. Only long-term HSCs can give rise to mature hematopoietic cells for the lifetime of the animal, while short-term HSCs and multipotent progenitors reconstitute lethally irradiated mice for less than eight weeks⁷.

Despite the fact that the phenotypic and functional properties of mouse and human HSCs have been extensively characterized², our understanding of the fundamental stem cell property, self renewal, is minimal^{25,29,30}. In most cases, HSCs differentiate when exposed to combinations of growth factors that can induce extensive proliferation in long-term cultures³¹. Although recent progress has been made in identifying culture conditions that maintain HSC activity in culture for a limited period of time [for example see Miller and Graves ³²], it has proven to be exceedingly difficult to identify tissue culture conditions that promote a significant and prolonged expansion of progenitors with transplantable HSC activity.

Maintenance of a tissue or a tumor is determined by a balance of proliferation and cell death³³. In a normal tissue, stem cell numbers are under tight genetic regulation resulting in maintenance a constant number of stem cells in the organ^{27,34,35}. By contrast, cancer cells have escaped this homeostatic regulation and the number of cells within a tumor that have the ability to self renew is constantly expanding, resulting in the inevitable growth of the tumor. As would be expected, many of the mutations that drive tumor expansion regulate either cell proliferation or survival. For example, the prevention of apoptosis by enforced expression of the oncogene Bcl-2 promotes the development of lymphoma and also results in increased numbers of HSCs *in vivo,* suggesting that cell death plays a role in regulating the homeostasis of HSCs^{36,37}. In fact, the progression to experimental acute myelogenous leukemia in mice requires at least 3, and likely 4 independent events to block the several intrinsically triggered and extrinsically induce programmed cell death pathways of myeloid cells³⁸. Proto-oncogenes such as c-myb and c-myc that drive proliferation of tumor cells are also essential for HSCs development³⁹⁻⁴².

Since cancer cells and normal stem cells share the ability to self-renew, it is not surprising that a number of genes classically associated with cancer may also regulate normal stem cell development [reviewed in ^{25,43}]. In combination with other growth factors, Shh signaling has also been implicated in the regulation of self-renewal by the finding that cells highly enriched for human hematopoietic stem cells (CD34⁺Lin⁻CD38⁻) exhibited increased self-renewal in response to Shh stimulation *in vitro⁴⁴.* Several other genes related to oncogenesis have been shown to be important for stem cell function. For example, mice deficient for *tal-1*/*SCL,* which is involved in some cases of human acute leukemia, lack embryonic hematopoiesis⁴⁵, suggesting that it is required for intrinsic or extrinsic events necessary to initiate hematopoiesis, for maintenance of the earliest definitive blood cells, or for the decision to form blood cells downstream of embryonic HSCs^{45,46}. Members of the Hox family have also been implicated in human leukemia. Enforced expression of *HoxB4* can affect stem cell functions^{47,48}. One of the major targets of the p53 tumor suppressor gene is p21^{cip1}. Bone marrow from p21^{cip1} deficient mice has a reduced ability to serially reconstitute lethally irradiated recipients. Failure at serial transfer could result from exhaustion of the stem cell pool, loss of telomeres, or loss of transplantability ⁴⁹. In mice, *bmi-1,* a gene that cooperates with *c-myc* to induce lymphoma^{50,51}, is required for the maintenance of adult HSCs and leukemia cells. Thus, many genes involved in stem cell fate decisions are also involved in malignant transformation.

Two other signaling pathways implicated in oncogenesis in both mice and humans, the Wnt/β-catenin and Notch pathways, may play central roles in the self-renewal of both normal and cancer stem cells. The Notch family of receptors was first identified in Drosophila and has been implicated in development and differentiation⁵². In *C elegans,* Notch plays a role in germ cell self renewal⁵³. In neural development transient Notch activation initiates an irreversible switch from neurogenesis to gliogenesis by embryonic neural crest stem cells¹⁰. Notch activation of HSCs in culture using either of the Notch ligands Jagged-1 or Delta transiently increased primitive progenitor activity that could be observed *in vitro* and *in vivo,* suggesting that Notch activation promotes either the maintenance of progenitor cell multipotenitality or HSC self-renewal^{54,55}. While the Notch pathway plays a central role in development and the mouse int-3 oncogene is a truncated Notch4⁵⁶, the role for Notch in de novo human cancer is complex and less well understood. Various members of the Notch signaling pathway are expressed in cancers of epithelial origin and activation by Notch by chromosomal translocation is involved in some cases of leukemia ⁵⁷⁻⁶¹. Microarray analysis has shown that members of the Notch pathway are often over-expressed by tumor cells^{58,59}. A truncated Notch4 mRNA is expressed by some breast cancer cell lines⁶². Overexpression of Notch1 leads to growth arrest of a small cell lung cancer cell line, while inhibition of Notch1 signals can induce leukemia cell lines to undergo apoptosis^{52,54,63}. Work by Miele and colleagues showed that activation of Notch-1 signaling maintains the neoplastic phenotype in Ras-transformed human cells⁶⁴. They also found that in *de novo* cancers, cells with an activating Ras mutation also demonstrated increased expression of Notch-1 and Notch-4.

Wnt/β-catenin signaling also plays a pivotal role in the self-renewal of normal stem cells and malignant transformation⁶⁵⁻⁶⁷. The Wnt pathway was first implicated in MMTV-induced breast cancer where in deregulated expression of Wnt-1 due to proviral insertion resulted in mammary tumors^{68,69}. Subsequently, it has been shown that Wnt proteins play a central role in pattern formation. Wnt-1 belongs to large family of highly hydrophobic secreted proteins that function by binding to their cognate receptors, members of the Frizzled and low-density lipoprotein receptor-related protein families, resulting in activation of β-catenin ^{43,58,65,70,71}. In the absence of receptor activation, β-catenin is marked for degradation by a complex consisting of the Adenomatous Polyposis Coli (APC), Axin and glycogen synthase kinase-3β proteins^{58,67,72-74}.) ^{66,75}. Wnt proteins are expressed in the bone marrow, and activation of Wnt/β-catenin signaling by Wnt proteins in vitro or by expression of a constitutively active β-catenin expands the pool of early progenitor cells and enriched normal transplantable hematopoietic stem cells in tissue culture and *in vivo* ^{25,67,72}. Inhibition of Wnt/β-catenin by ectopic expression of Axin, an inhibitor of β-catenin signaling, leads to inhibition of stem cell proliferation both *in vitro* and *in vivo.* Other studies suggest that the Wnt/β-catenin pathway mediates stem or progenitor cell self-renewal in other tissues^{73,74,76,77}. Higher levels of β-catenin are seen in keratinocytes with higher proliferative potential than those seen in keratinocytes with lower proliferative capacity^{73,74,77}. Like their normal hematopoietic stem cell counterparts, enforced expression of an activated β-catenin increased the ability of epidermal stem cells to self renew and decreased their ability to differentiate. Mice that fail to express TCF-4, one of the transcription factors that is activated when bound to β-catenin, soon exhaust their undifferentiated crypt epithelial progenitor cells, further suggesting that Wnt signaling is involved in the self renewal of epithelial stem cells^{43,76}.

Activation of β-catenin in colon cancer by inactivation of the protein degradation pathway, most frequently by mutation of APC, is common^{43,58,66,75}. Expression of certain Wnt genes is elevated in some other epithelial cancers suggesting that activation of β-catenin is secondary to ligand activation in such cancers^{65,78-83}. There is evidence that constitutive activation of the Wnt/β-catenin pathway may confer a stem/progenitor cell phenotype to cancer cells. Inhibition of β-catenin/TCF-4 in a colon cancer cell line induced the expression of the cell cycle inhibitor p21^{cip-1} and induced the cells to stop proliferating and to acquire a more differentiated phenotype⁸³. Enforced expression of the proto-oncogene c-myc, which is transcriptionally activated by β-catenin/TCF-4, inhibited the expression of p21^{cip-1} and allowed the colon cancer cells to proliferate when β-catenin/TCF-4 signaling was blocked, linking Wnt signaling to c-myc in the regulation of cell proliferation and differentiation. Although many studies have implicated the Wnt/β-catenin pathway in breast cancer, activating mutations of β-catenin are rare in this disease and no studies have definitively linked this pathway to human breast cancer⁸⁴⁻⁸⁹.

The implication of roles for genes like Notch, Wnt, c-myc and Shh in the regulation of self-renewal of HSCs and perhaps of stem cells from multiple tissues suggests that there may be common self-renewal pathways in many types of normal somatic stem cells and cancer stem cells. It is important to identify the molecular mechanisms by which these pathways work and to determine whether the pathways interact to regulate the self-renewal of normal stem cells and cancer cells.

The Wnt pathway is involved in the self-renewal of normal stem cells and activating mutations of Wnt induce breast cancer in mice. This pathway plays a role in tumor formation by human breast cancer stem cells isolated from some patients. Furthermore, evidence suggests that the ability of different populations of breast cancer cells to form tumors differs. Interestingly, the expression of members of the Wnt/Frizzled/β-catenin pathway are heterogeneously expressed by different populations of cancer cells and expression of particular members of the pathway may correlate with the capacity to form tumors.

The different populations of cancer cells and tumor cells drive the proliferation of breast cancer cells. Activated β-catenin is seen in the cancer cells in a significant number of patients. The tumors that contain cancer cells with this pathway constitutively active behave differently than those without constitutively activated β-catenin.

### II. Xenograft model of human breast cancer

Although cell lines have led to remarkable advances in our understanding of the molecular and biochemical changes in cancer cells, their use in the identification of effective cancer therapies is somewhat limited^{90,91}. Cell lines are imperfect predictors of drug efficacy in de novo tumors^{90,91}. Several factors likely account for this deficiency. Cancer cell lines are selected from a sub-population of cancer cells that are specifically adapted to growth in tissue culture and the biological and functional properties of these cell lines can change dramatically⁹²⁻⁹⁵ Furthermore, cancer cells from only a minority of breast cancer tumors establish cell lines or xenograft tumors^{96,97}. The phenotypic and functional characteristics of these cell lines can change drastically relative to their properties in vivo⁹⁴. For example, the marker expression of both normal hematopoietic and leukemic tissue culture cells can change rapidly in tissue culture and often does not reflect that of the original stem cells from which they were derived^{92,94,95,98}. Even when conditions are devised to permit the proliferation of normal stem cells in culture, the conditions often promote self-renewal or differentiation in a way that prevents the stem cells in culture from recapitulating the hierarchy of cell populations that exist in vivo. Taken together, these observations suggest that the biological properties of cancer cell lines can differ markedly from the cancer cells from which they were derived. This likely explains at least in part why the cell lines often are poor predictors of a drug's efficacy in the clinic.

Thus, the lack of an effective method to consistently grow primary human breast cancer cells *in vitro* or *in vivo* for long periods of time has severely limited our ability to understand the biology of this disease. The most efficient xenograft models report the engraftment of pieces of breast cancer tumors in the ovarian, but not mammary, fat pad of SCID mice approximately 60-75% of the time⁹⁹. Engraftment of dissociated cells is not possible in this model, and cancer cells isolated from pleural effusions only form tumors in immunodeficient mice approximately 10% of the time⁹⁰. The present invention (see Example 1 below) provides a xenograft model in which one is able to establish tumors from primary breast tumors via injection of tumors in the mammary gland of severely immunodeficient mice. These Xenograft of the present invention allows one to do biological and molecular tests to characterize the clonogenic breast cancer cell as well as other cell types. Importantly, the xenograft tumors developed in accordance with the present invention contain the phenotypically diverse cancer cell types found in the human tumors from which they were derived and the different populations of cancer cells differ markedly in their ability to form tumors¹⁰⁰.

The development of an efficient xenograft model in accordance with the present invention (see e.g., Example 1), has for the first time reliably allows dissociated solid tumor cells obtained from a patient to form tumors. Importantly, this enables one to routinely analyze biochemical pathways in an individual patient's cancer cells and to do molecular manipulations that allow one to understand the cellular consequences of specific genetic pathways on tumor formation by de novo human solid tumor cancer cells.

### III. Solid Tumor Stem Cells Cancer Markers

The present invention provides markers whose expression is specifically altered in solid tumor stem cells (e.g. up regulated or down regulated). Such markers find use in the diagnosis and characterization and alteration (e.g., therapeutic targeting) of various cancers (e.g. breast cancer).

Example 4, provided below, describes methods used to identify solid tumor cancer markers. Preferred cancer markers are provided below in Tables 4-8, as well as Notch 4. While these tables provide gene names, it is noted that the present invention contemplates the use of both the nucleic acid sequences as well as the peptides encoded thereby, as well as fragments of the nucleic acid and peptides, in the therapeutic and diagnostic methods and compositions of the present invention.

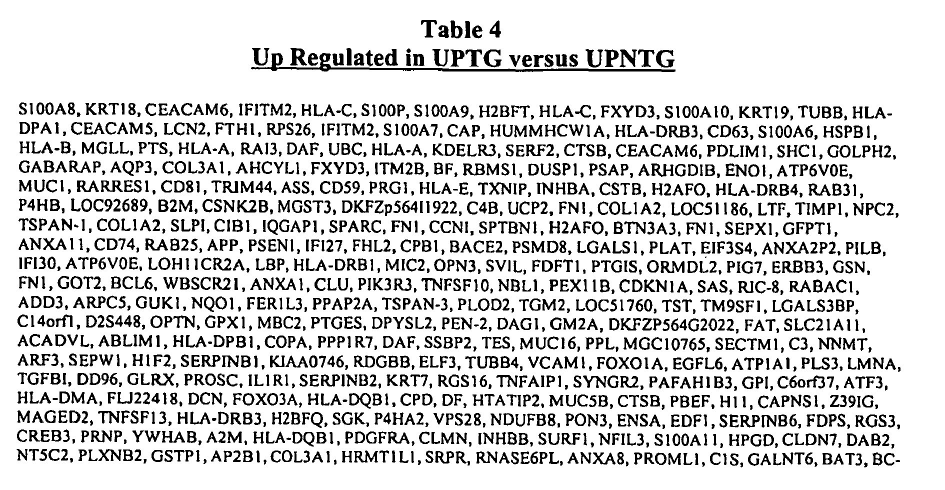

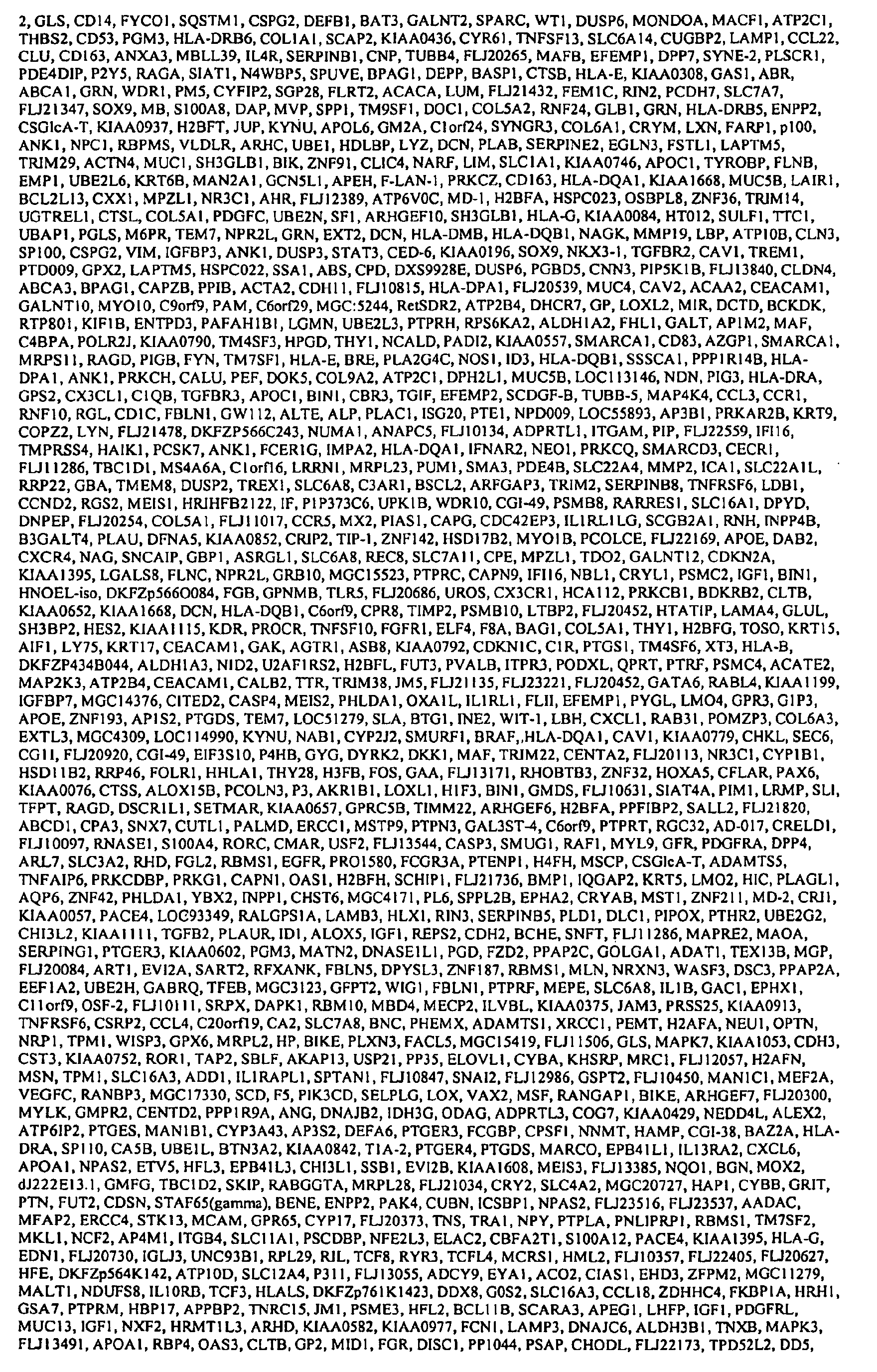

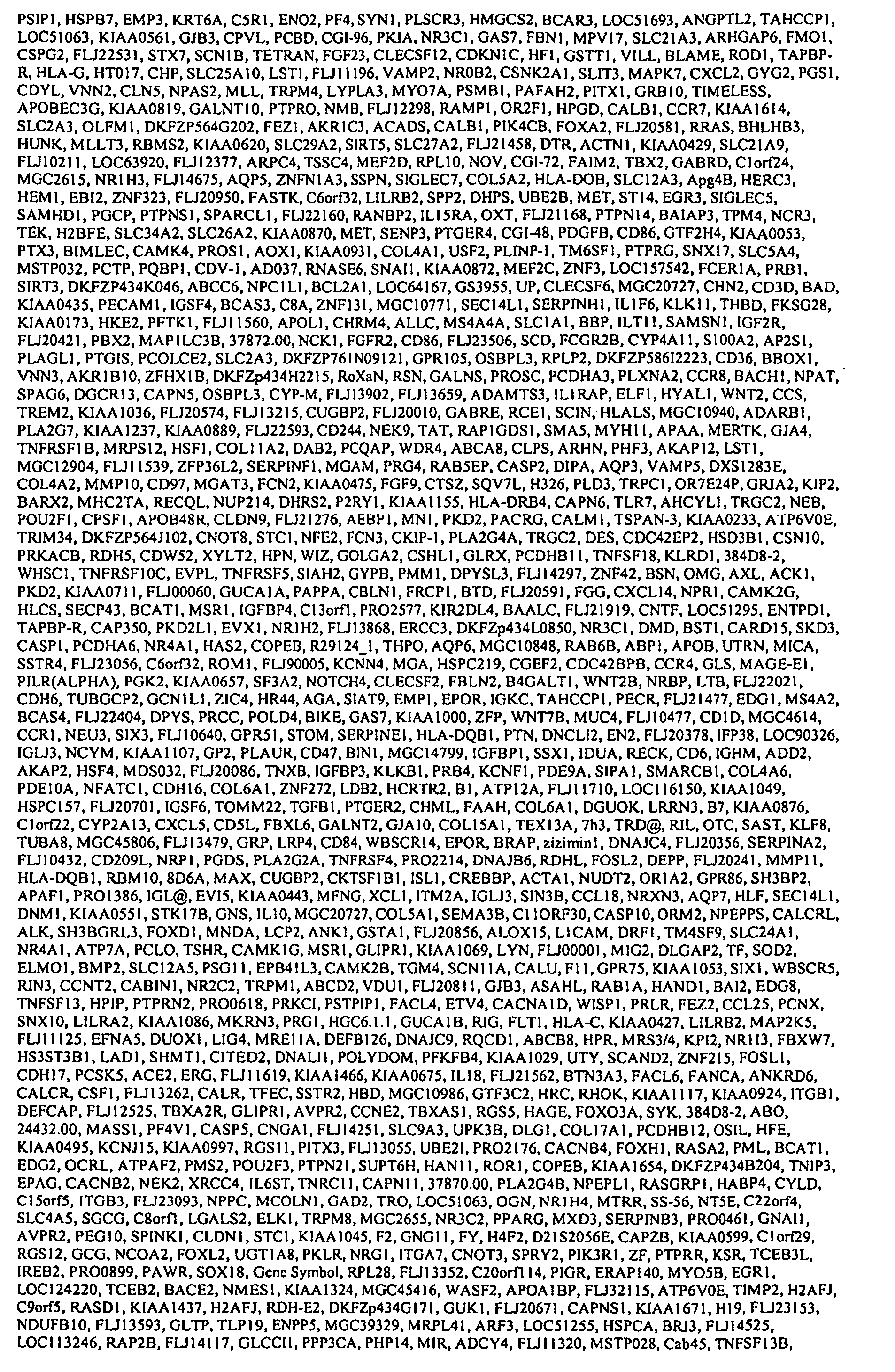

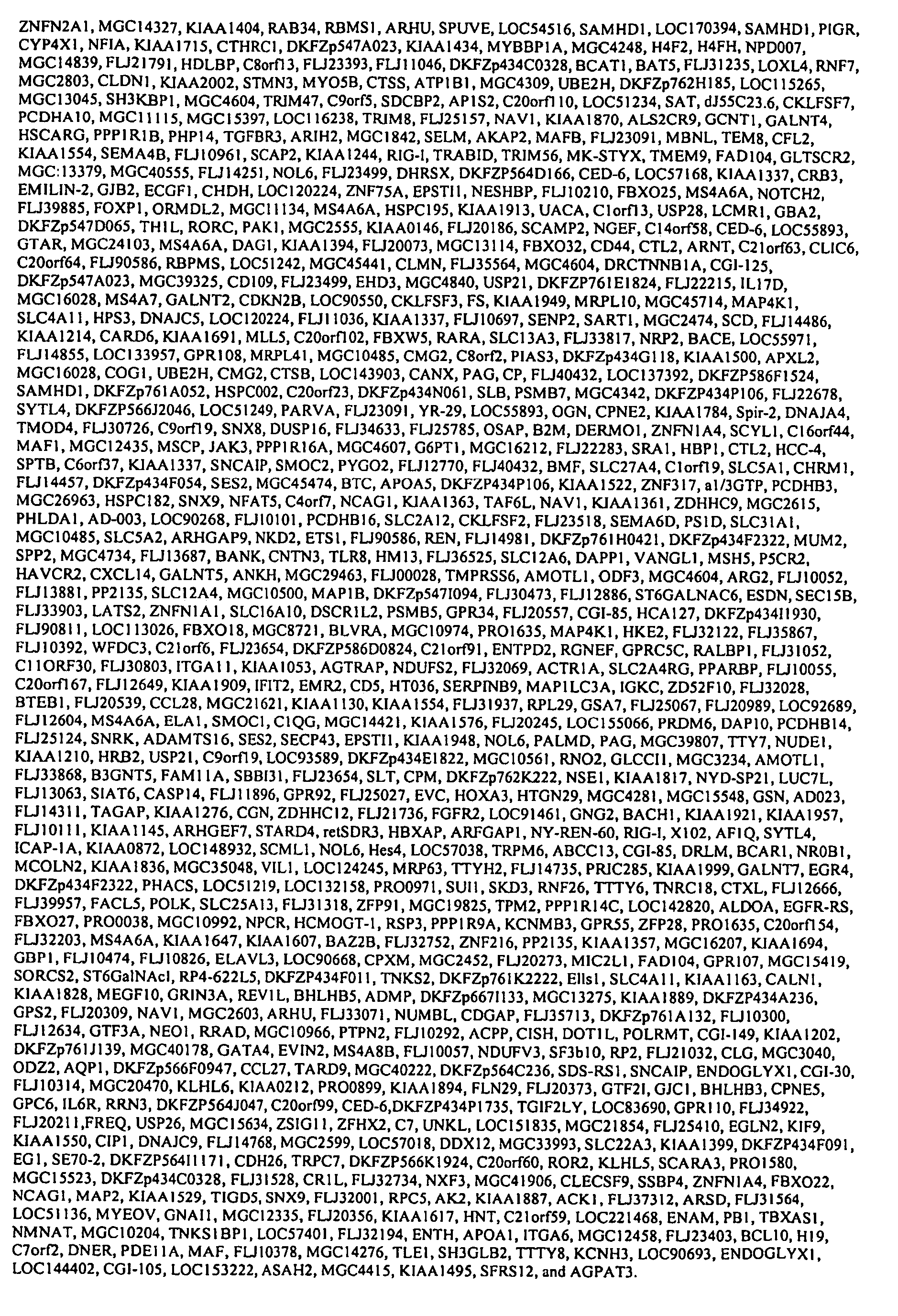

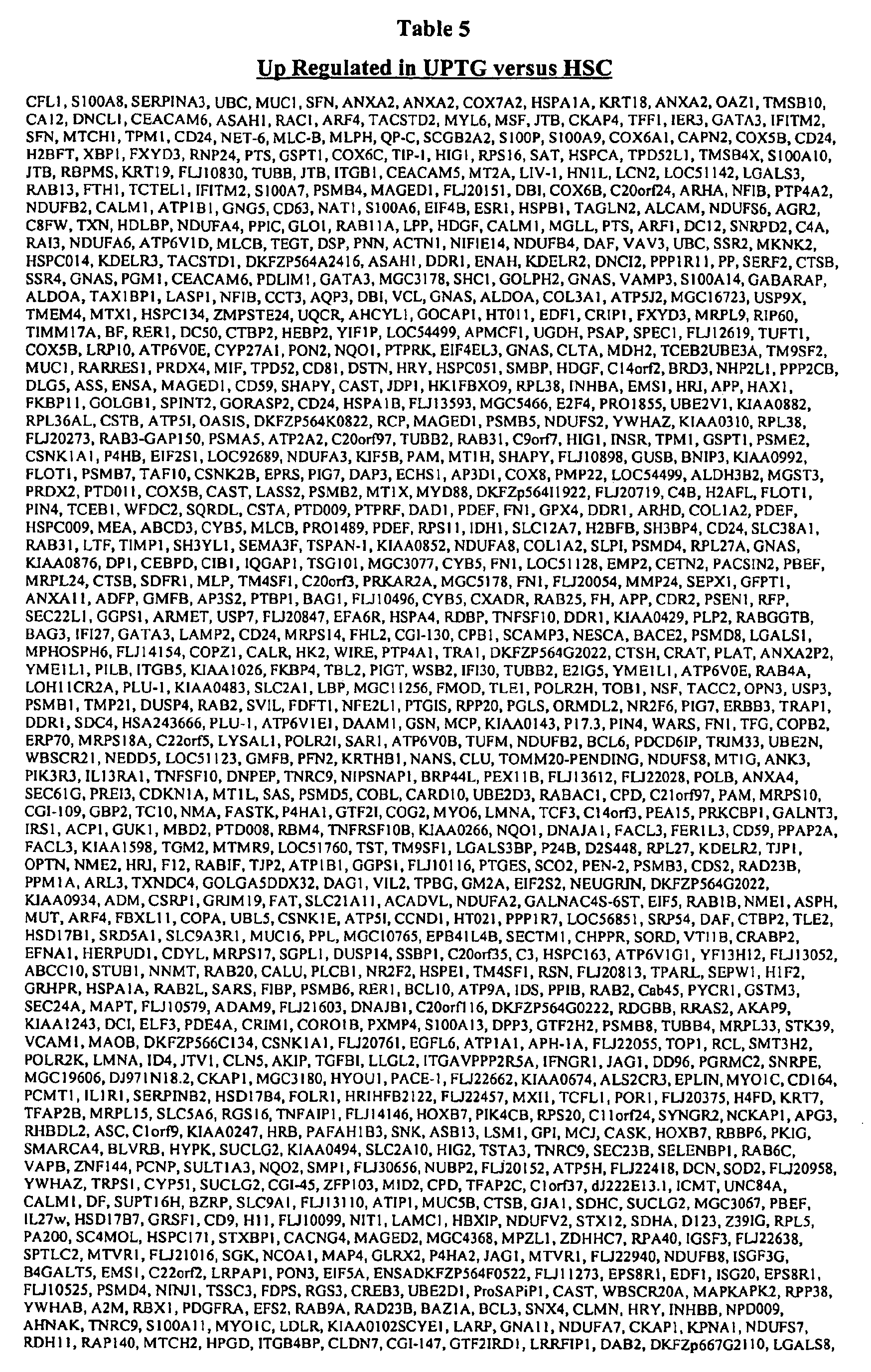

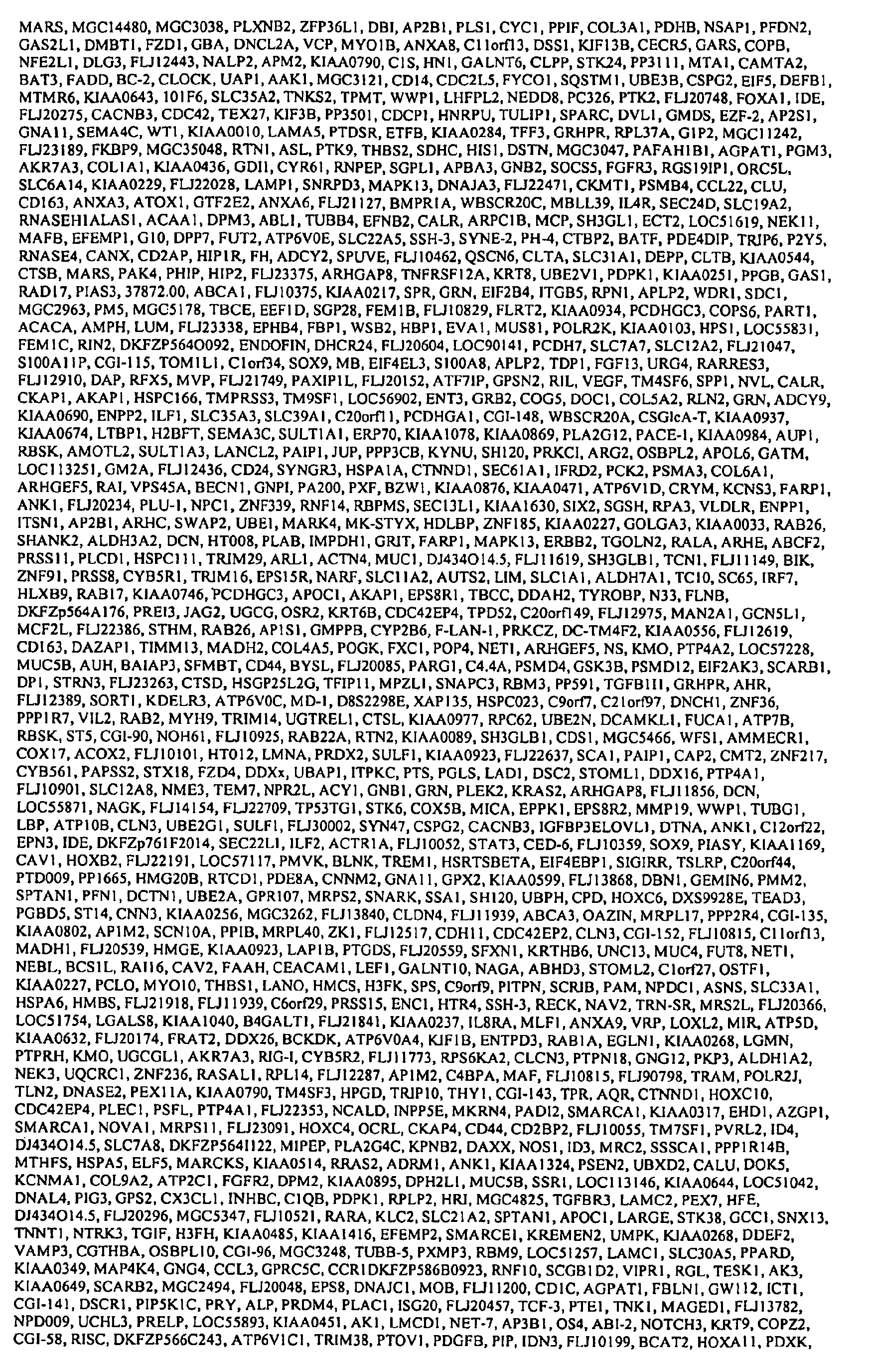

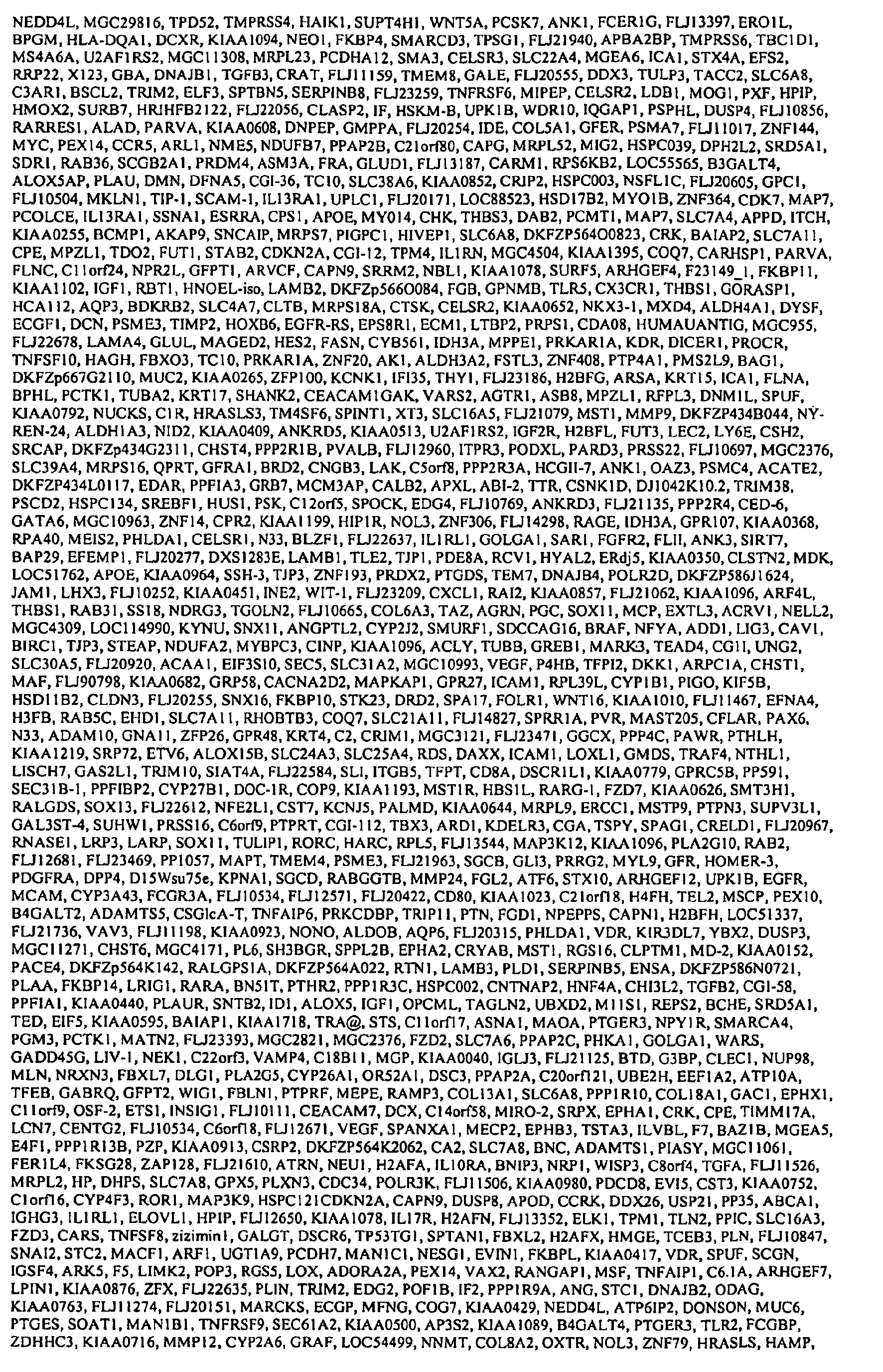

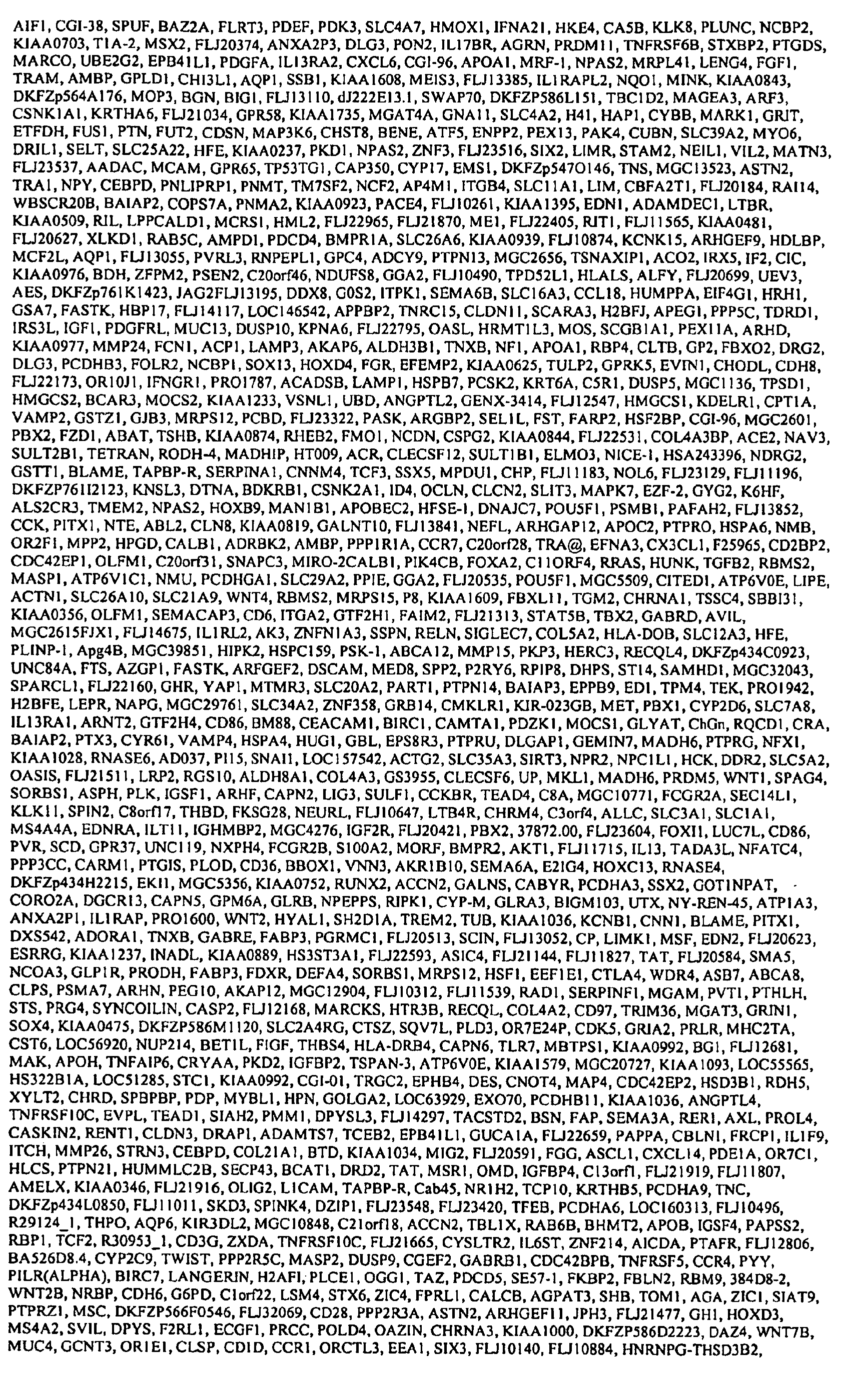

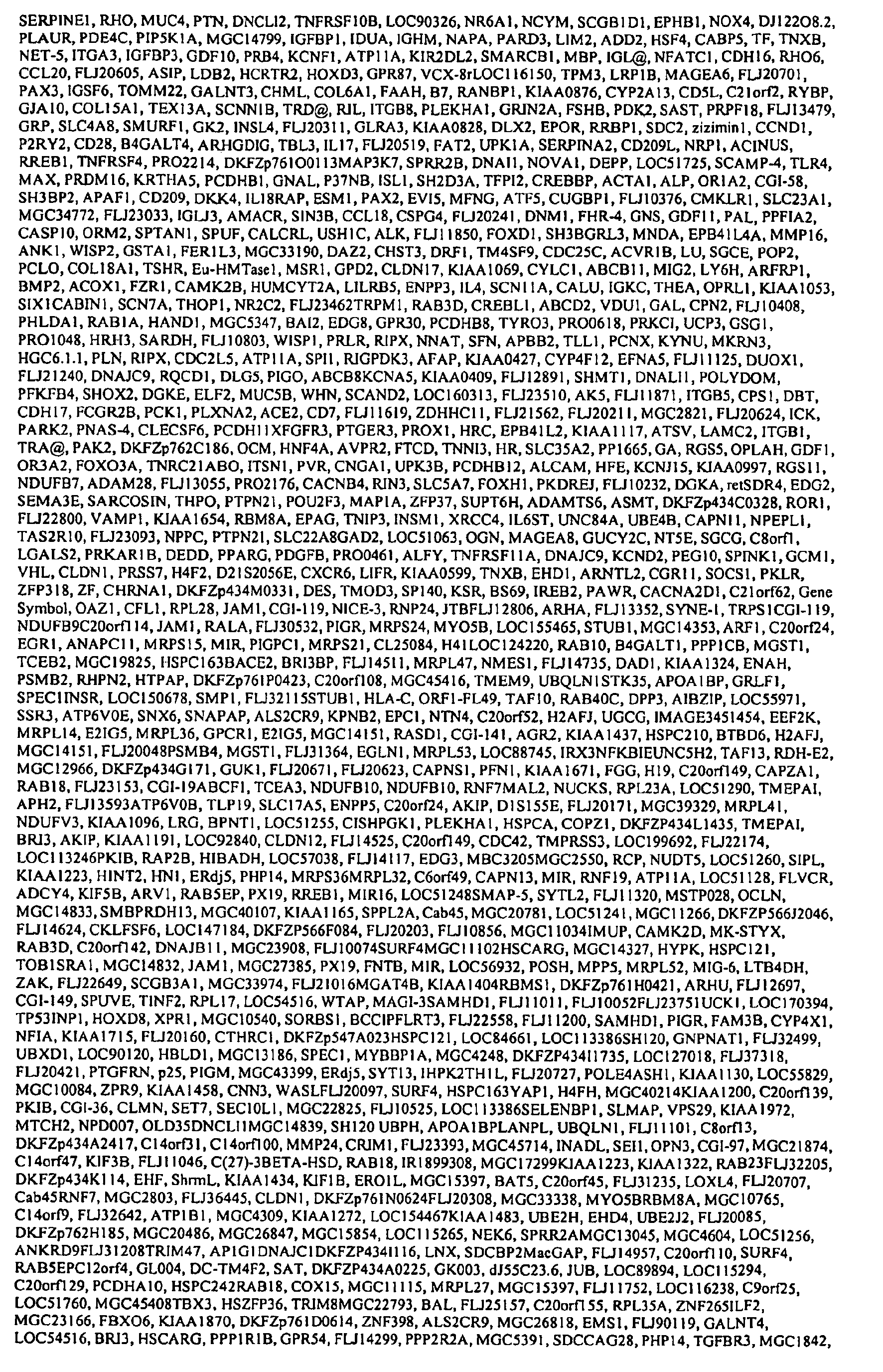

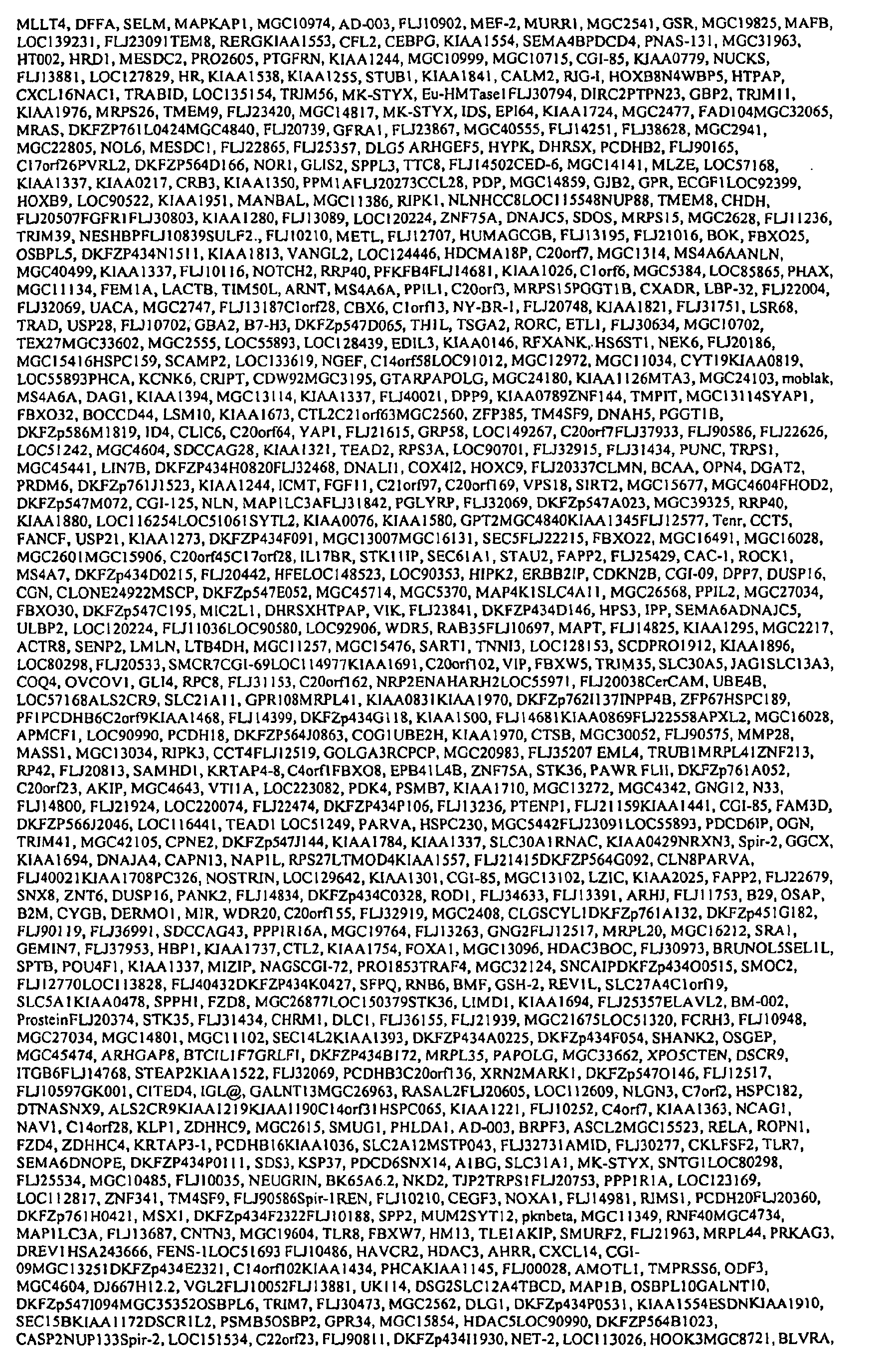

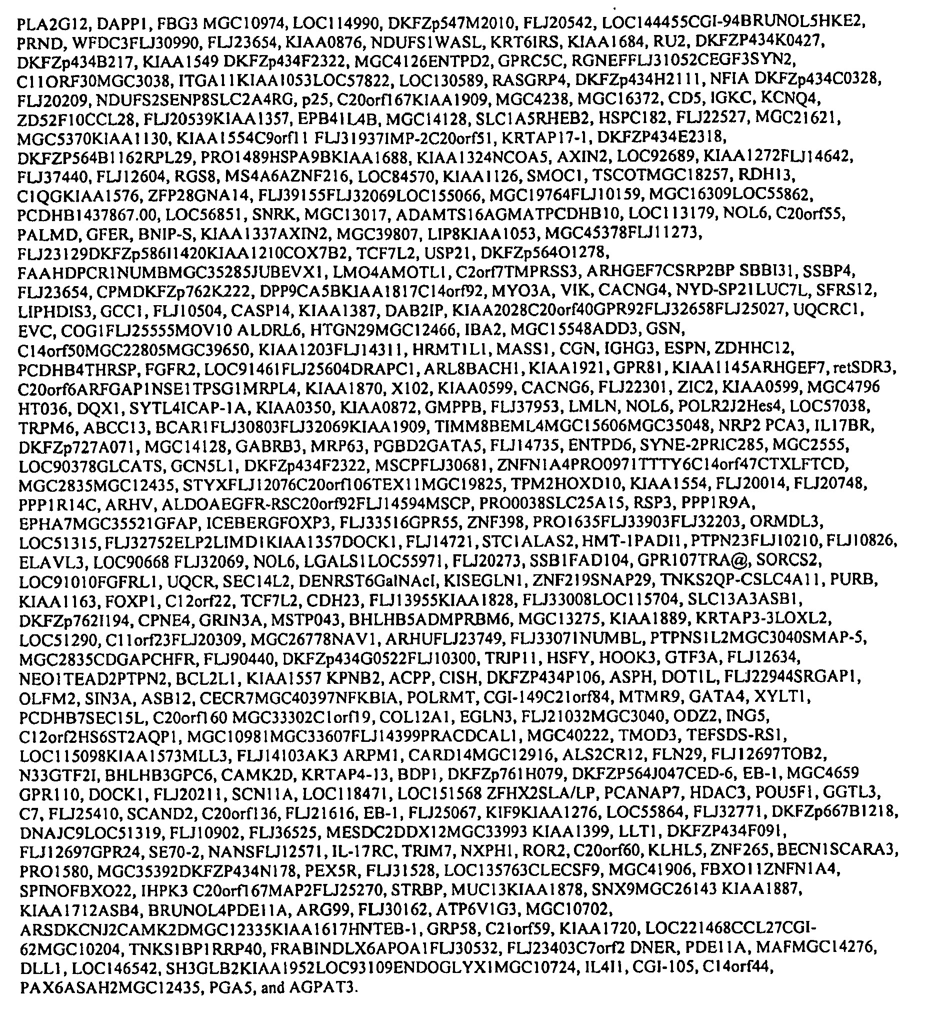

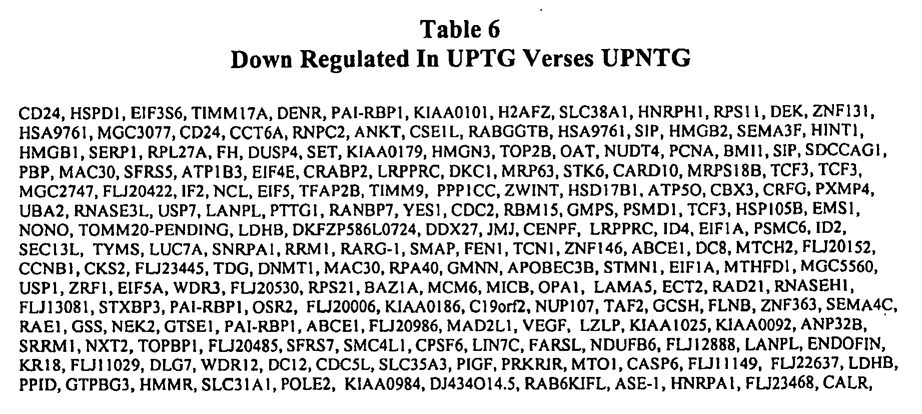

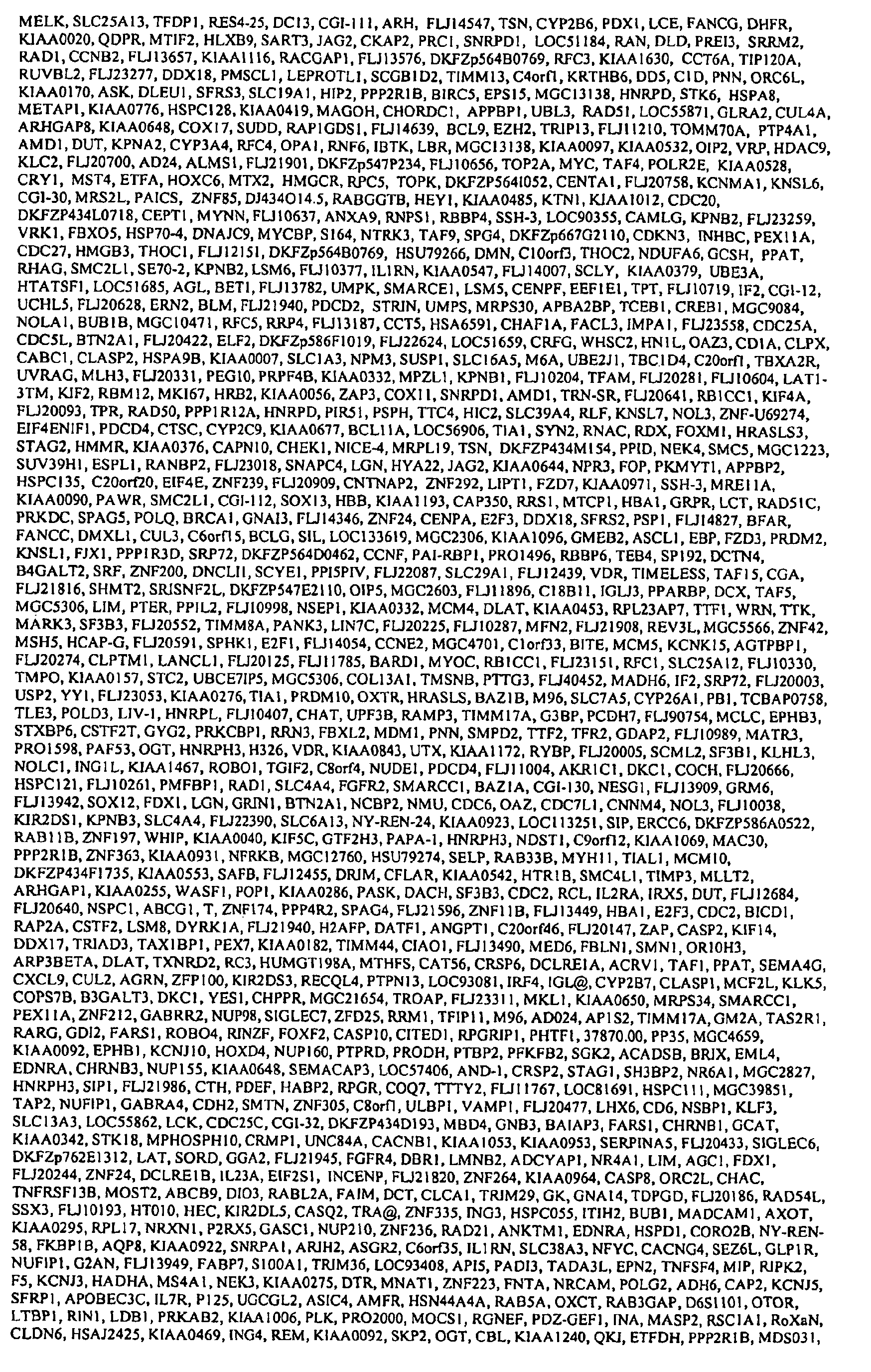

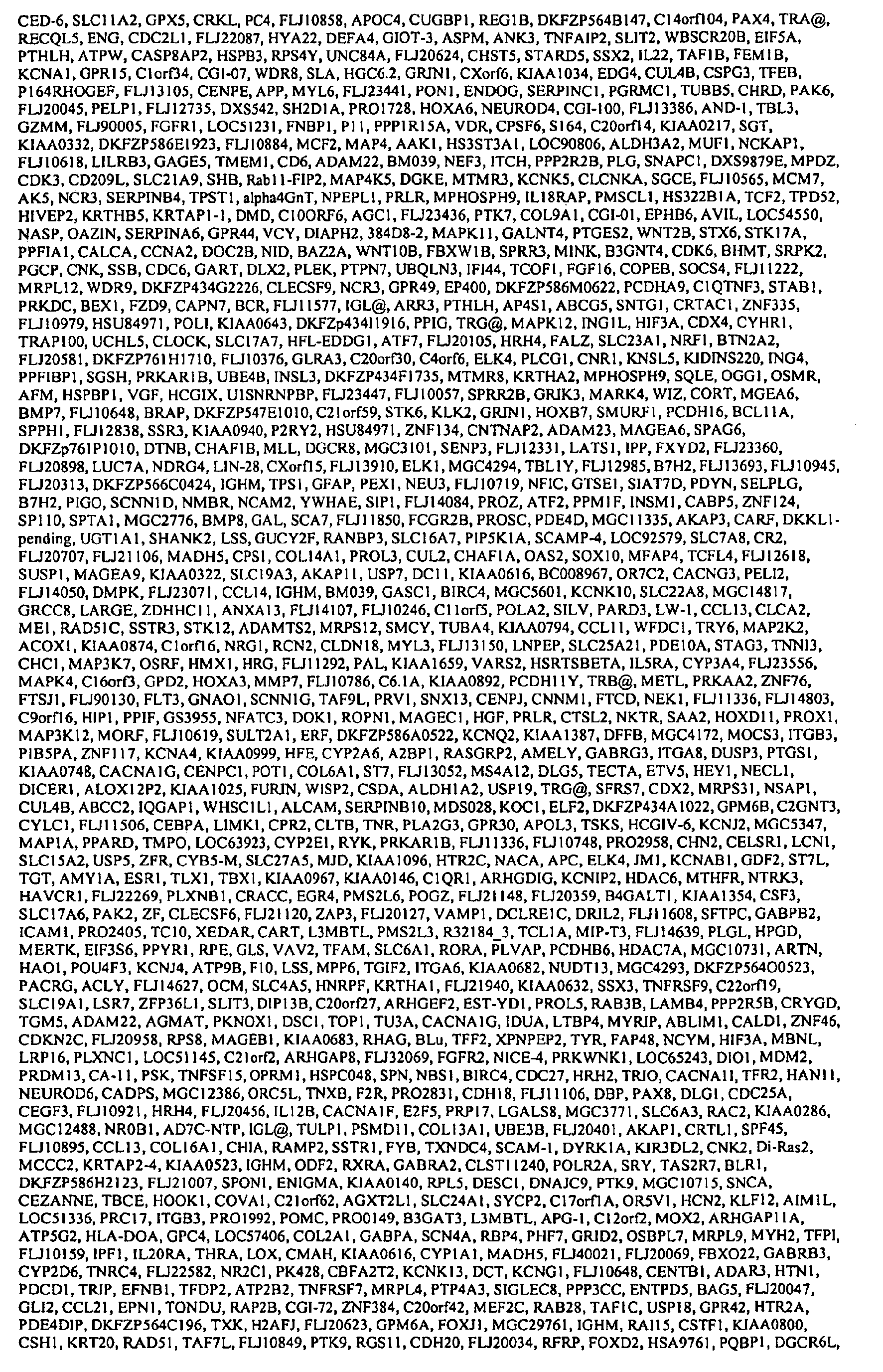

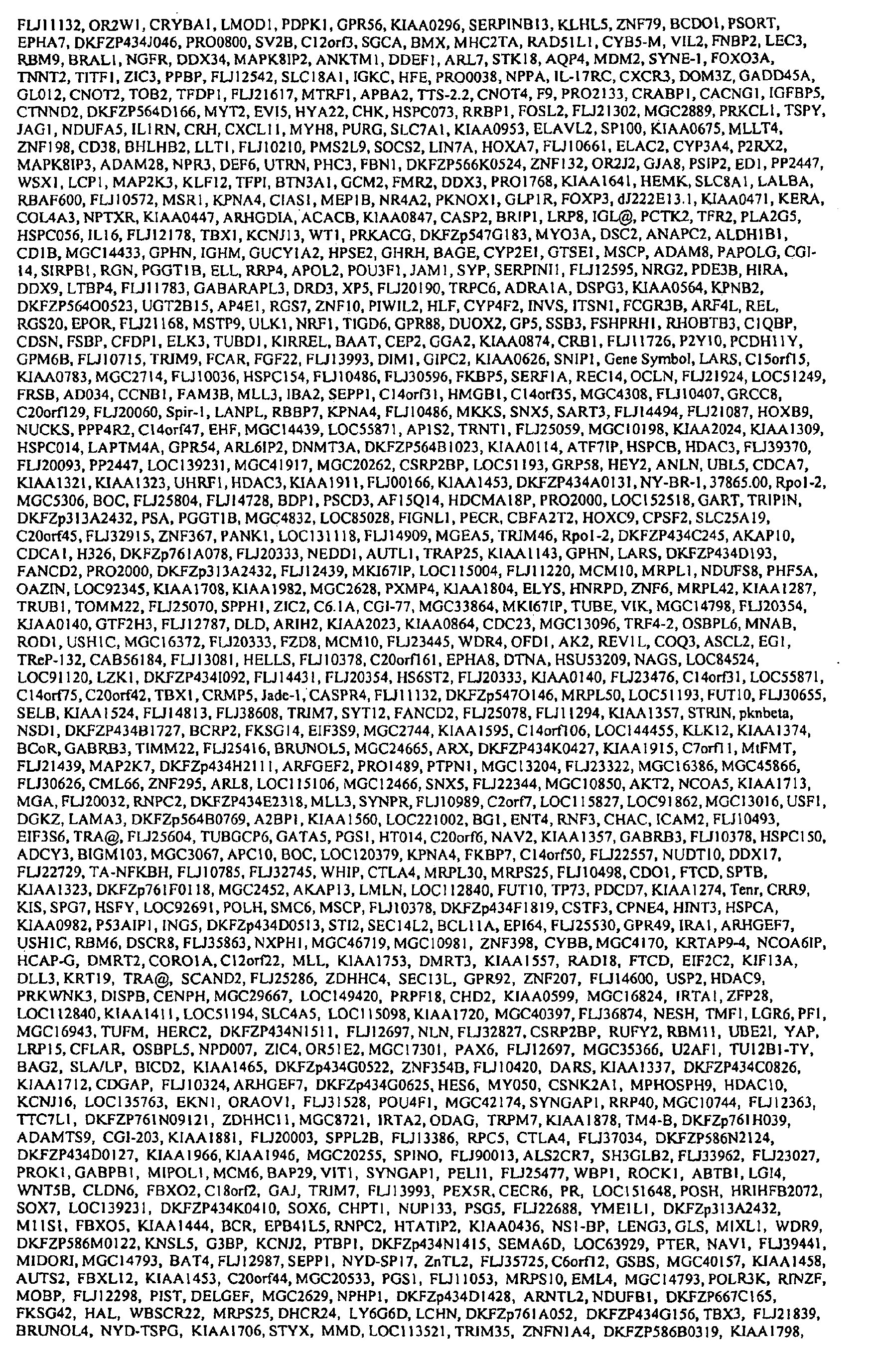

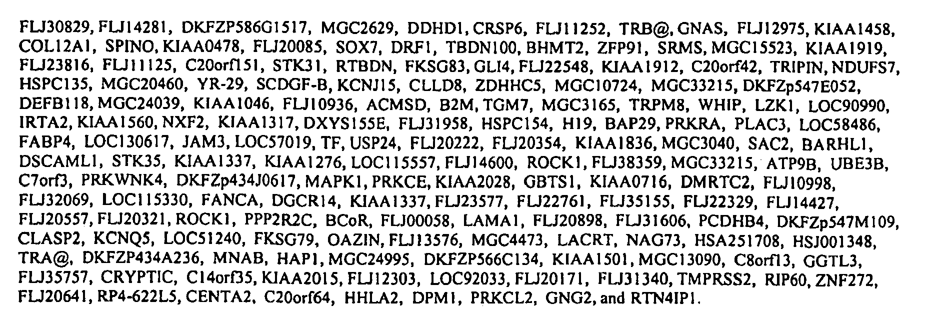

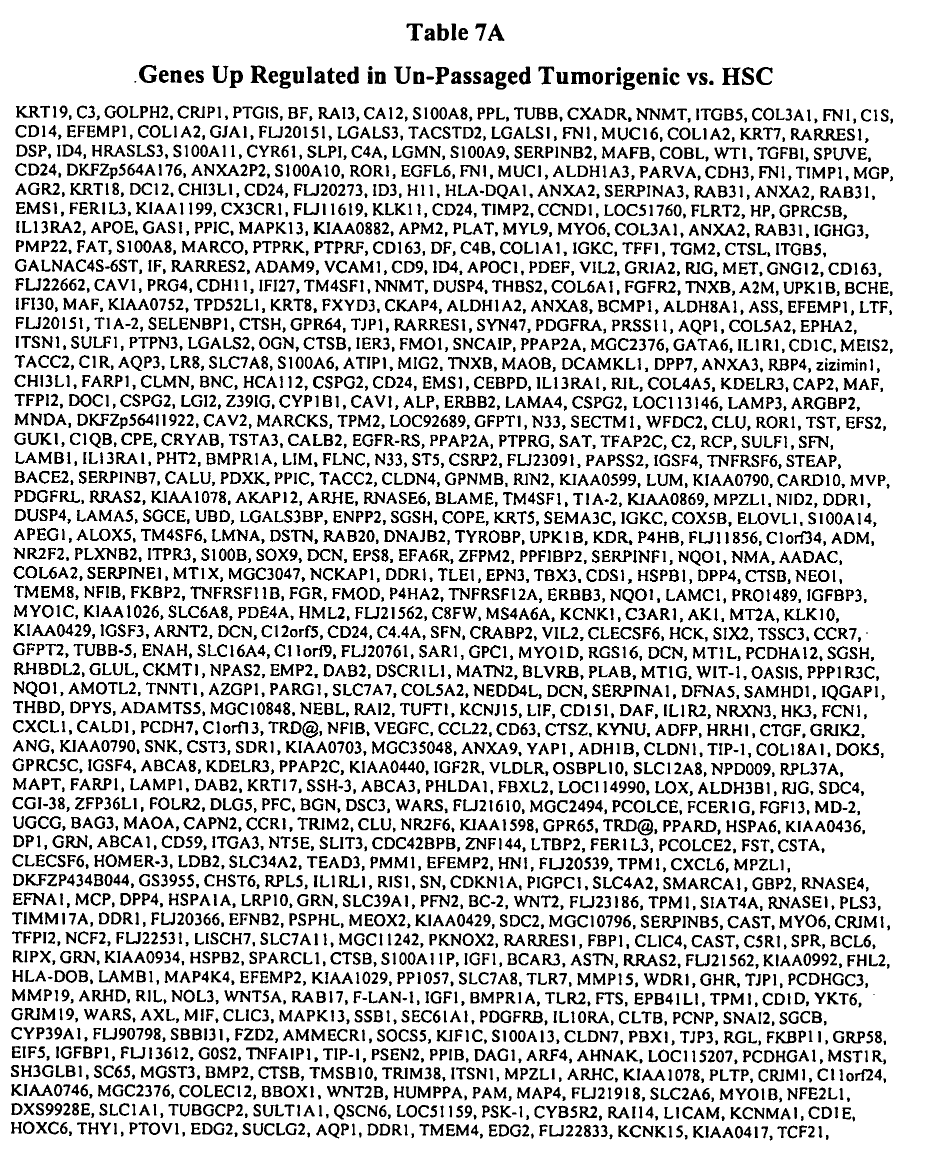

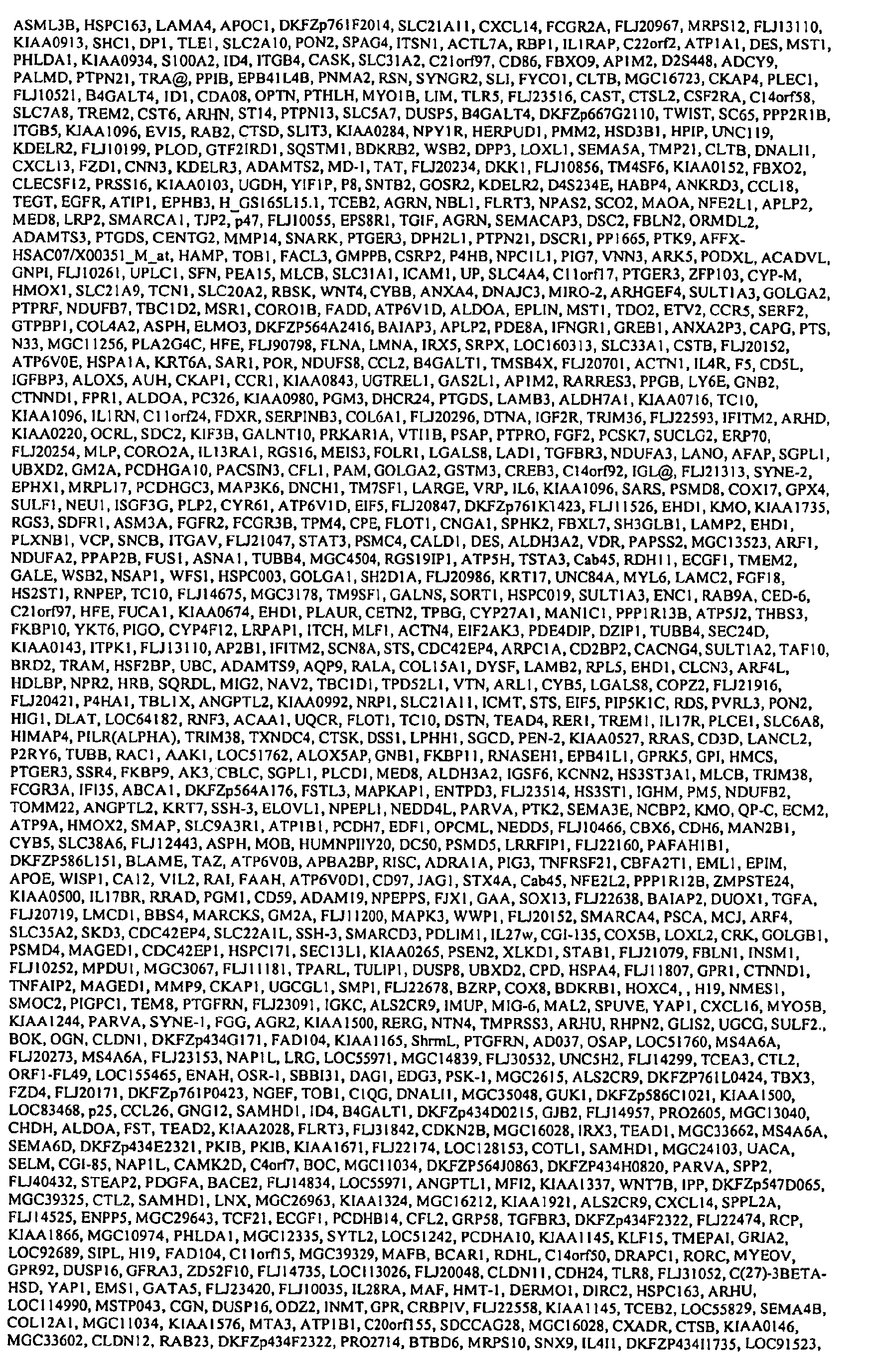

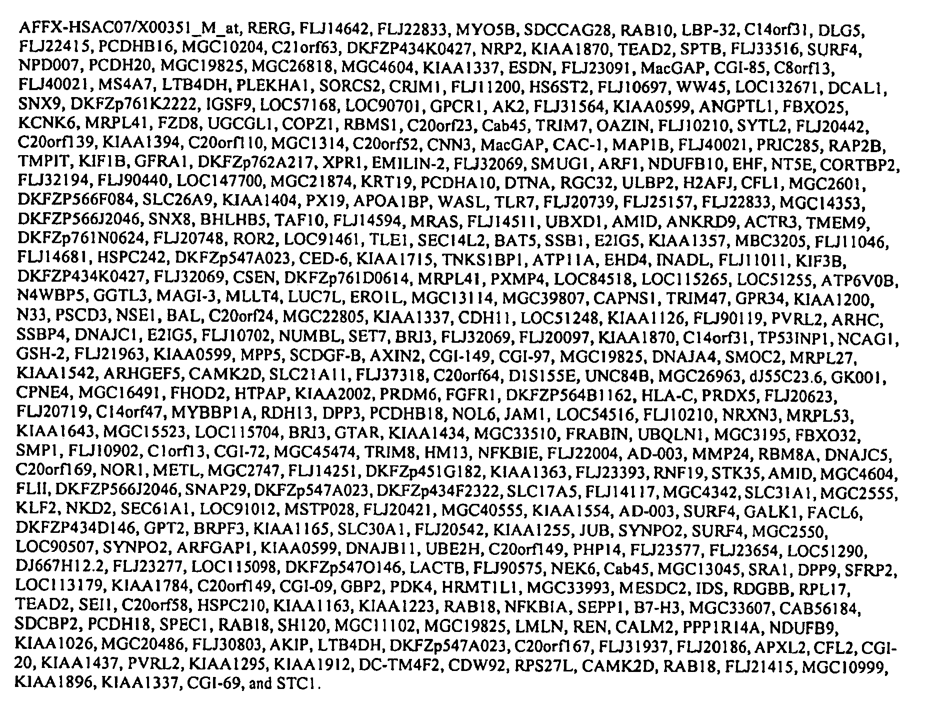

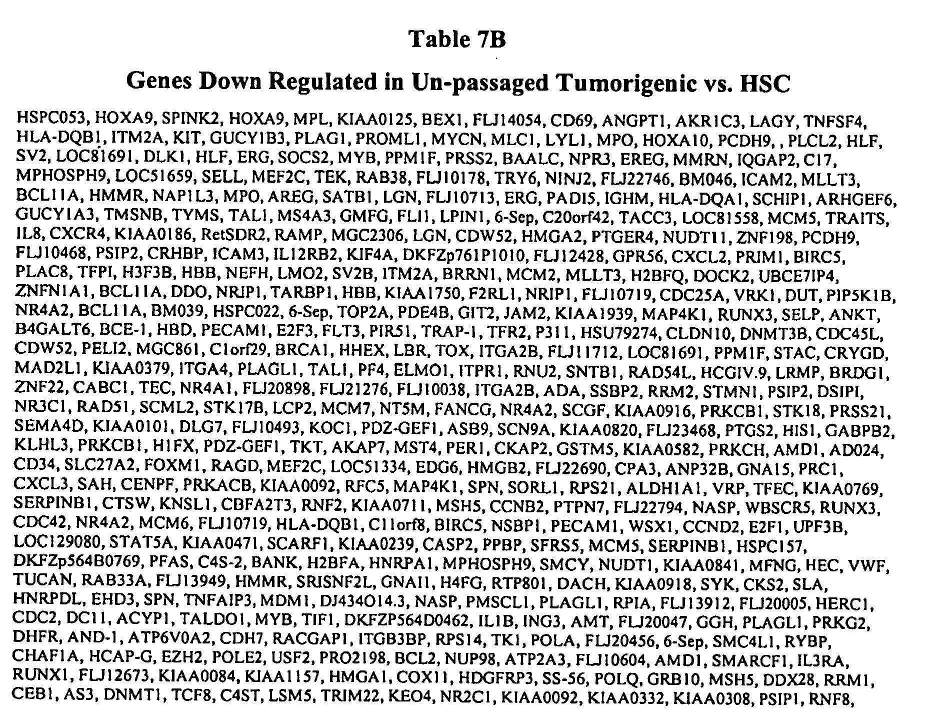

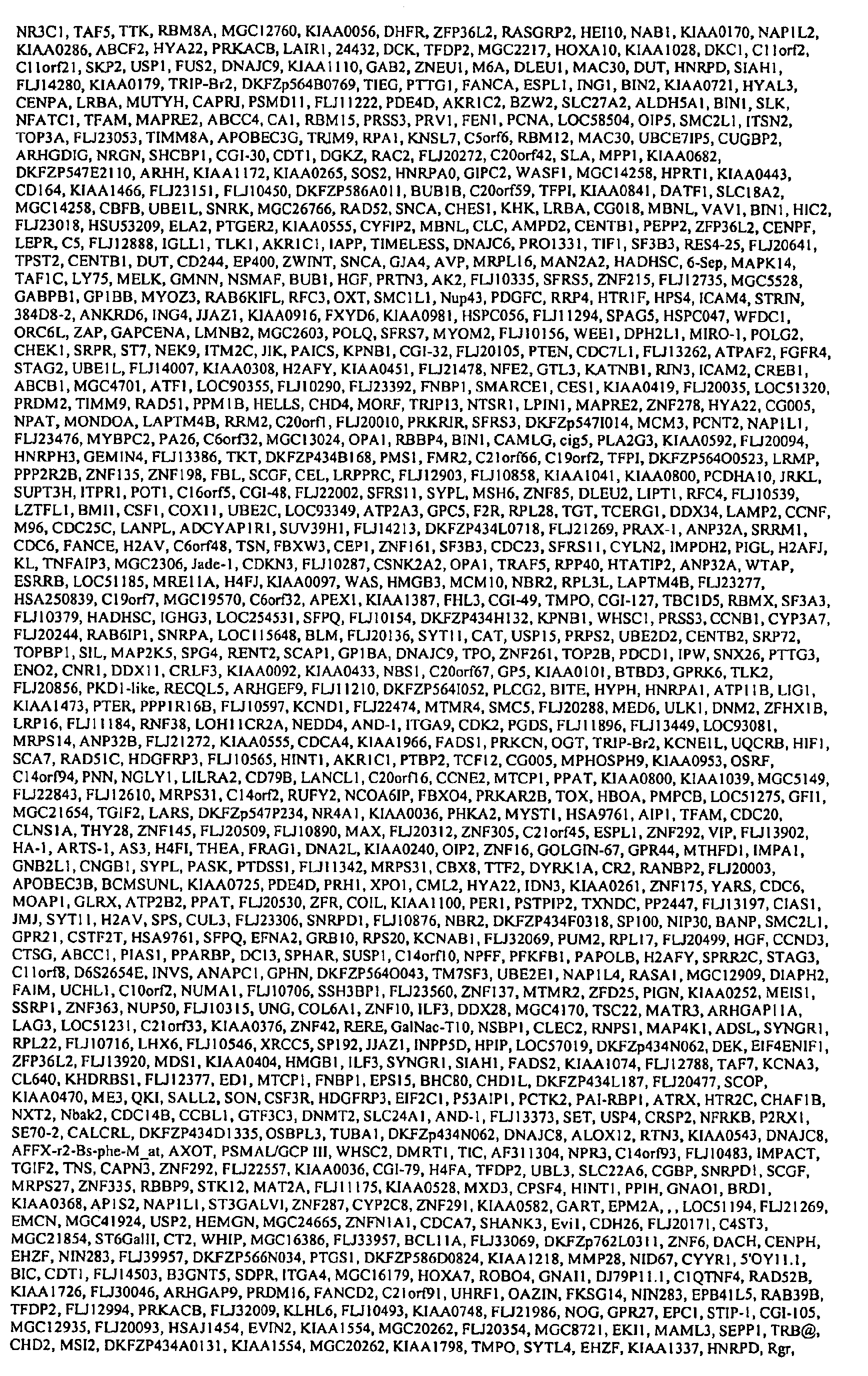

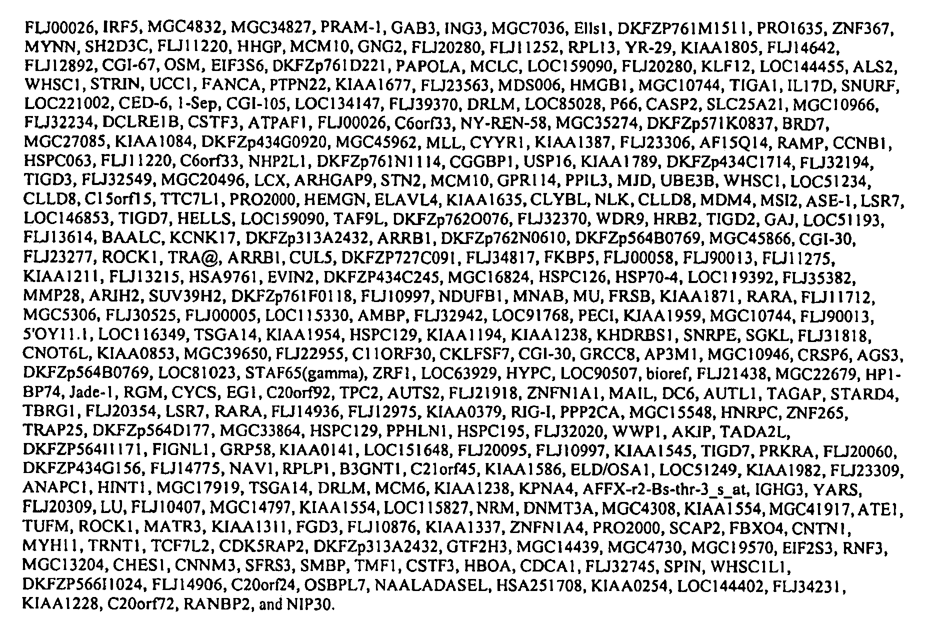

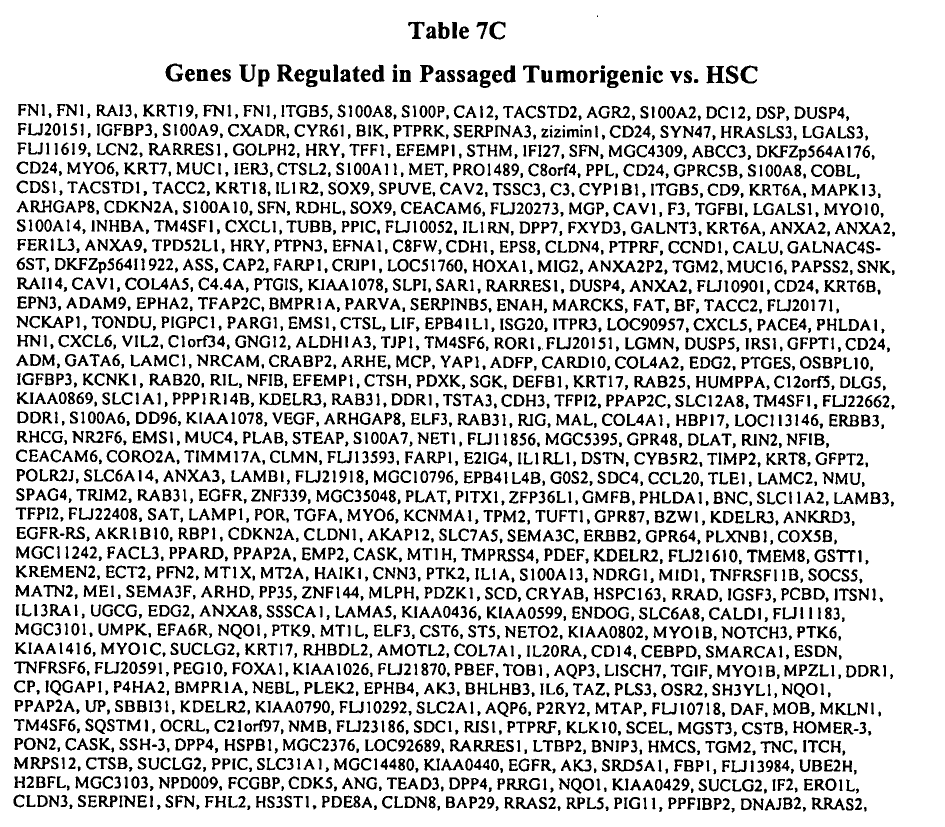

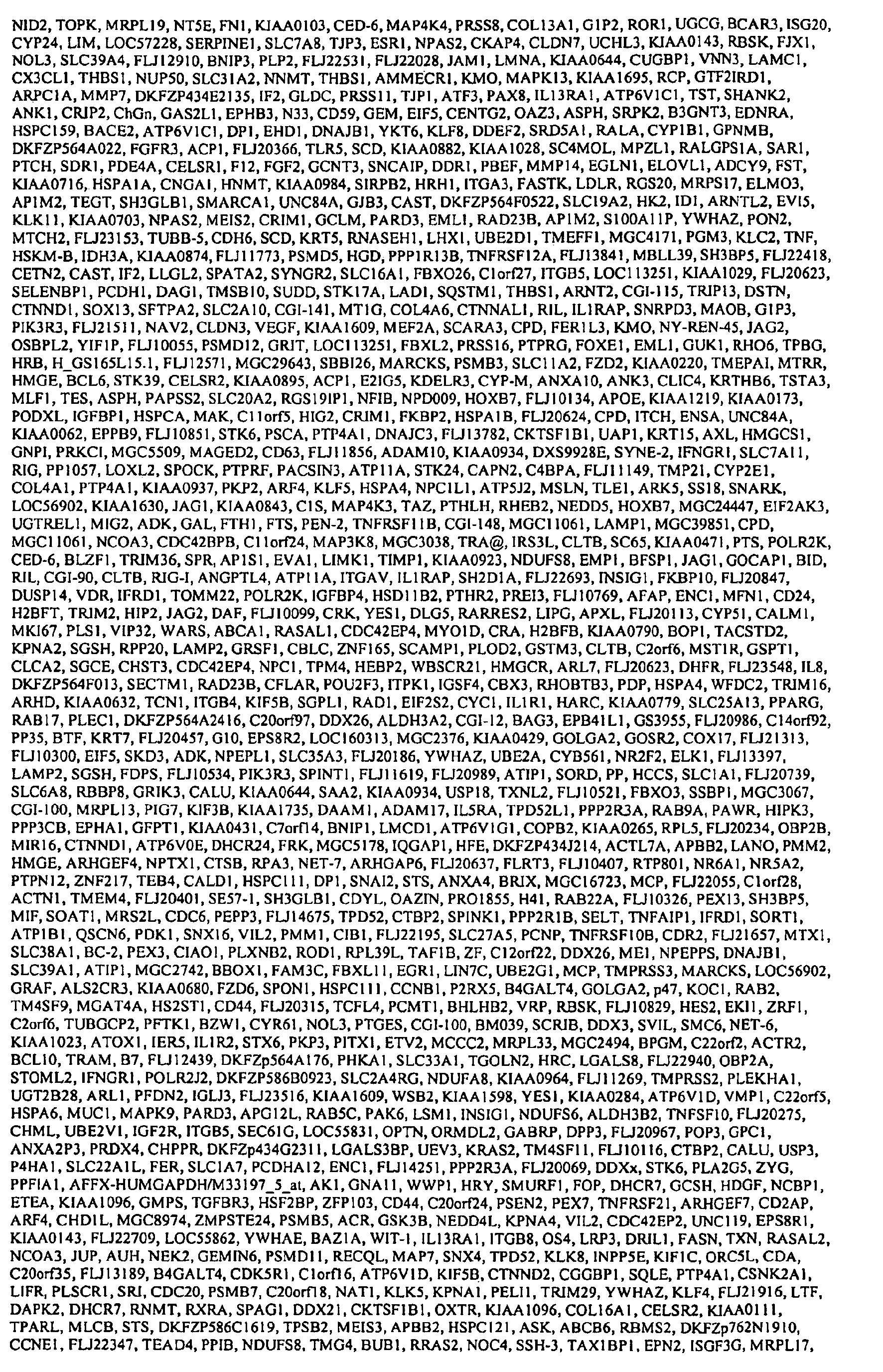

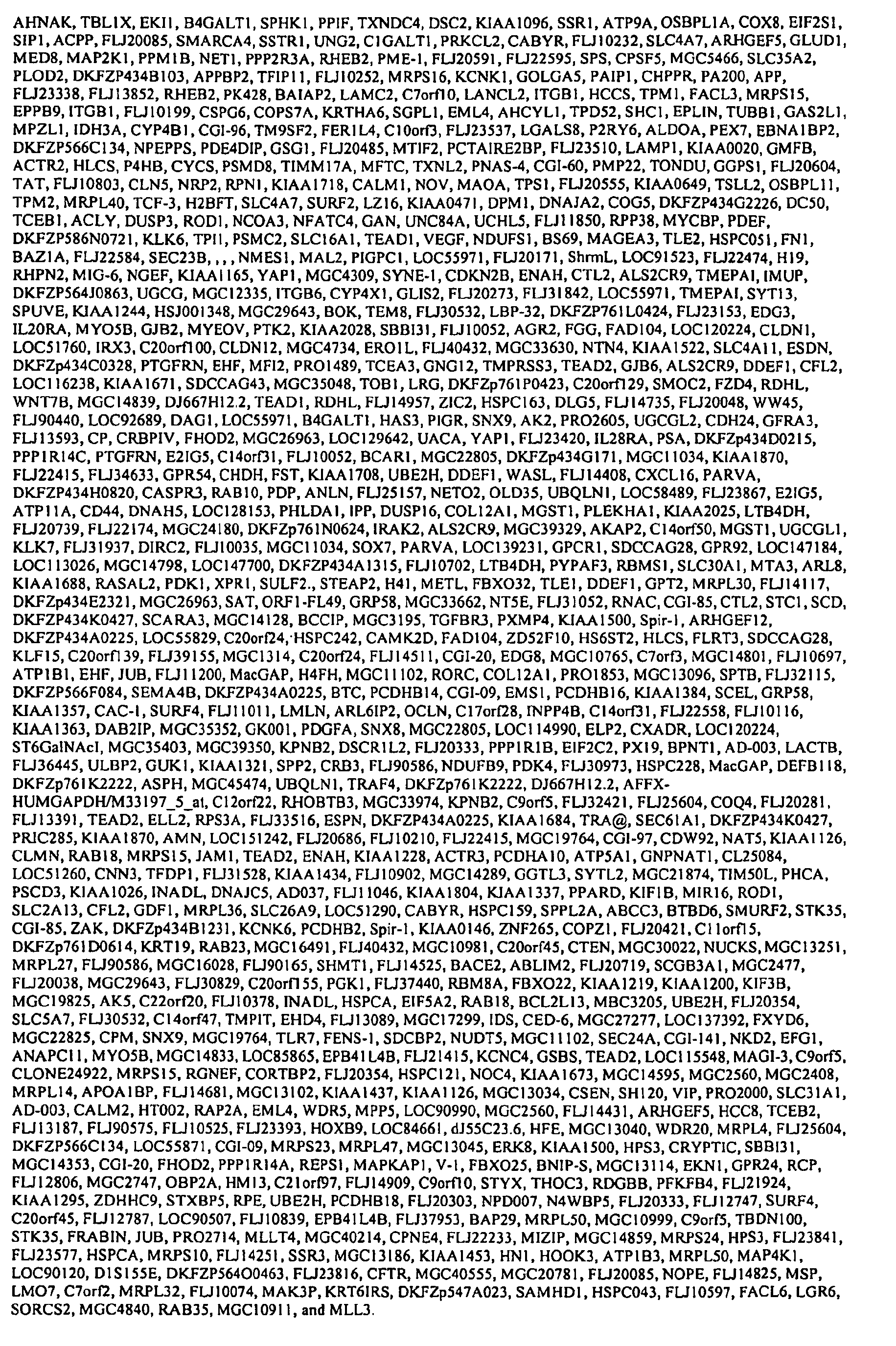

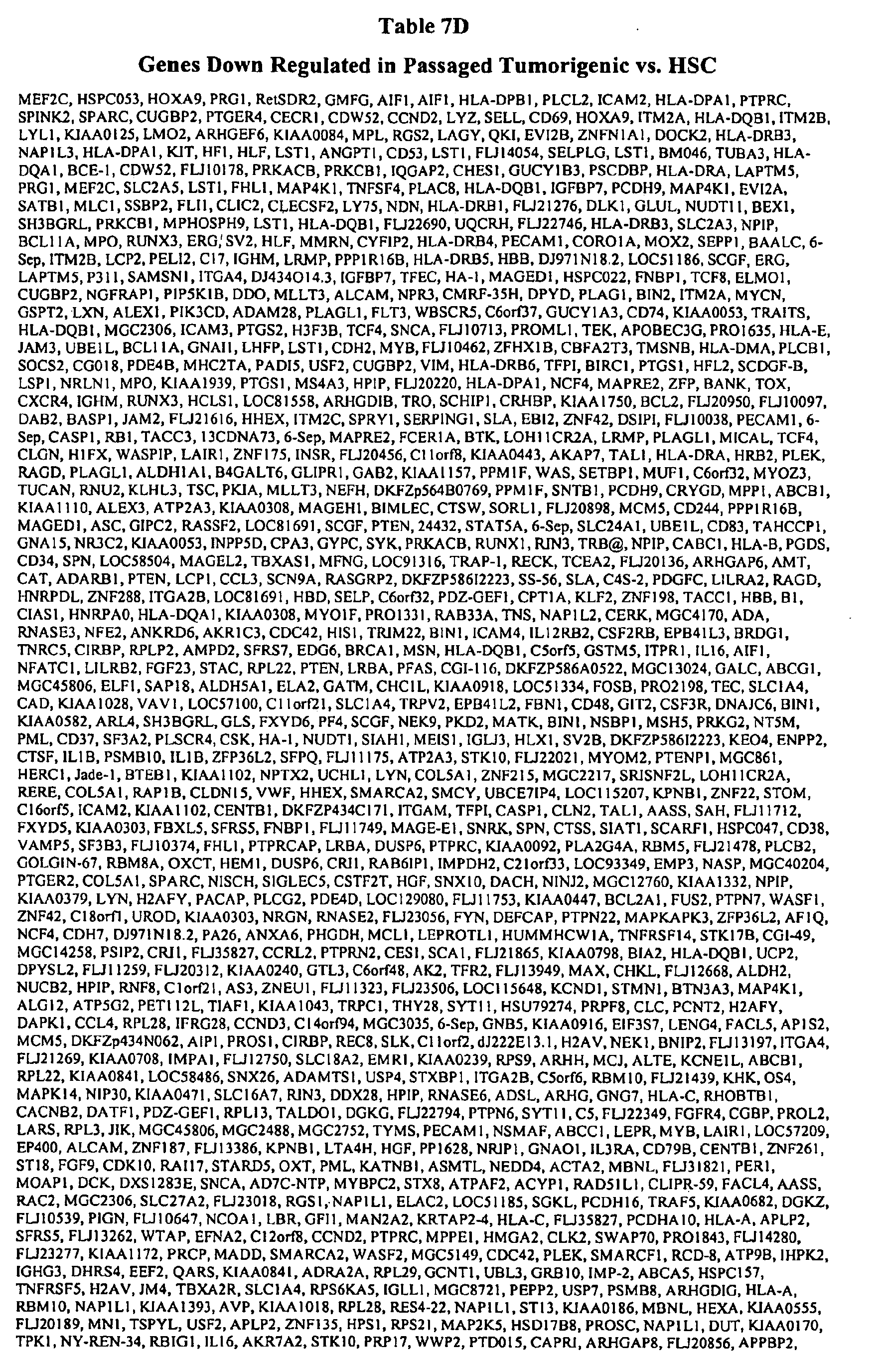

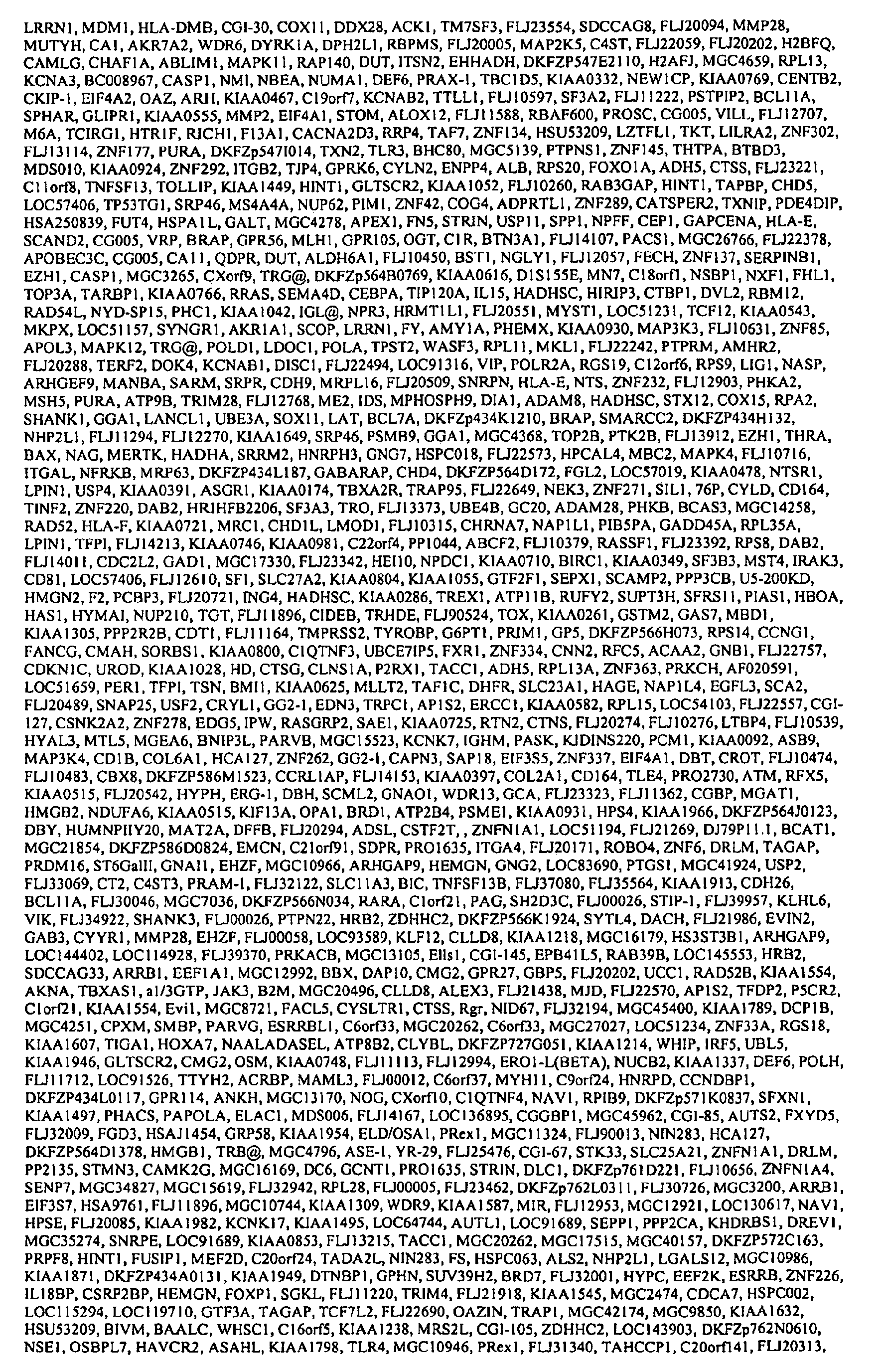

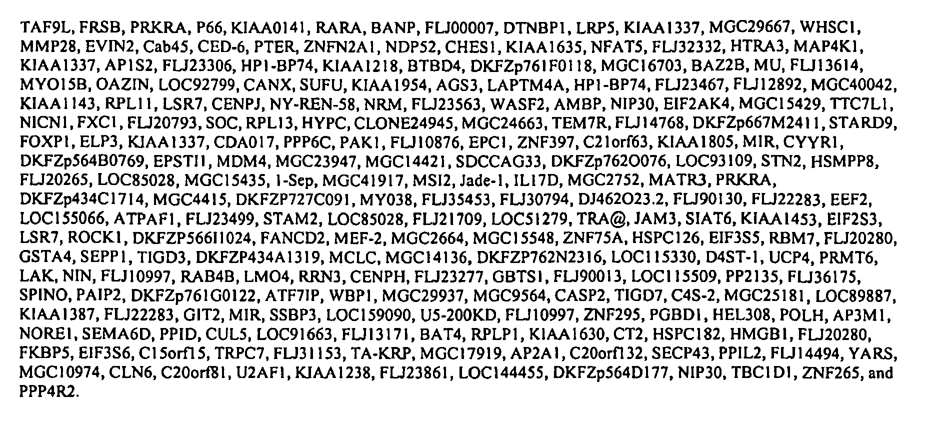

**Table 8**

| **Preferred Solid Tumor Stem Cell Cancer Markers** |
|---|
| Bmi-1, eed, easyhl, easyh2, rnf2, yyl, smarcA3, smarckA5, smarcD3, smarcE1, mllt3, frizzled 2, frizzled 6, frizzled 7, mf2, Frizzled 1, Frizzled2, Frizzled4, Frizzled10, Frizzled6, FZD1, FZD2, FZD3, FZD4, FZD6, FZD7, FZD8, FZD9, FZD10, WNT2, WNT2B, WNT3, WNT5A, WNT10B, WNT16, AXIN1, BCL9, MYC, (TCF4),, SLC7A8, IL1RAP, TEM8, TMPRSS4, MUC16, GPRC5B, SLC6A14, SLC4A11, PPAP2C, CAV1, CAV2, PTPN3, EPHA1, SLC1A1, CX3CL1, ADORA2A, MPZL1, FLJ10052, C4.4A, EDG3, RARRES1, TMEPAI, PTS, CEACAM6, , NID2, STEAP, ABCA3, CRIM1, IL1R1, OPN3, DAF, MUC1, MCP, CPD, NMA, ADAM9, GJA1, CD14, SLC19A2, ABCA1, PCDH7, ADCY9, SLC39A1, NPC1, ENPP1, N33, GPNMB, LY6E, CELSR1, LRP3, C20orf52, TMEPAI, FLVCR, PCDHA10, GPR54, TGFBR3, SEMA4B, and PCDHB2. |

Additional solid tumor stem cells cancer markers can be identified, for example, using the methods described in Example 4 below.

### IV. Detection of Solid Tumor Stem Cell Cancer Markers

In some embodiments, the present invention provides methods for detection of expression of stem cell cancer markers (*e.g*., breast cancer stem cell cancer markers). In preferred embodiments, expression is measured directly (*e.g*., at the RNA or protein level). In some embodiments, expression is detected in tissue samples (*e.g*., biopsy tissue). In other embodiments, expression is detected in bodily fluids (*e.g.*, including but not limited to, plasma, serum, whole blood, mucus, and urine). The present invention further provides panels and kits for the detection of markers. In preferred embodiments, the presence of a stem cell cancer marker is used to provide a prognosis to a subject. The information provided is also used to direct the course of treatment. For example, if a subject is found to have a marker indicative of a solid tumor stem cell (see, e.g. Tables 4-8), additional therapies (*e.g*., hormonal or radiation therapies) can be started at a earlier point when they are more likely to be effective (*e.g*., before metastasis). In addition, if a subject is found to have a tumor that is not responsive to hormonal therapy, the expense and inconvenience of such therapies can be avoided.

The present invention is not limited to the markers described above. Any suitable marker that correlates with cancer or the progression of cancer may be utilized. Additional markers are also contemplated to be within the scope of the present invention. Any suitable method may be utilized to identify and characterize cancer markers suitable for use in the methods of the present invention, including but not limited to, those described in illustrative Example 4 below. For example, in some embodiments, markers identified as being up or down-regulated in solid tumor stem cells using the gene expression microarray methods of the present invention are further characterized using tissue microarray, immunohistochemistry, Northern blot analysis, siRNA or antisense RNA inhibition, mutation analysis, investigation of expression with clinical outcome, as well as other methods disclosed herein.

In some embodiments, the present invention provides a panel for the analysis of a plurality of markers. The panel allows for the simultaneous analysis of multiple markers correlating with carcinogenesis and/or metastasis. Depending on the subject, panels may be analyzed alone or in combination in order to provide the best possible diagnosis and prognosis. Markers for inclusion on a panel are selected by screening for their predictive value using any suitable method, including but not limited to, those described in the illustrative examples below.

### 1. Detection of RNA

In some preferred embodiments, detection of solid tumor stem cell cancer markers (*e.g*., including but not limited to, those disclosed in Tables 4-8) are detected by measuring the expression of corresponding mRNA in a tissue sample (*e.g*., breast cancer tissue). mRNA expression may be measured by any suitable method, including but not limited to, those disclosed below.

In some embodiments, RNA is detection by Northern blot analysis. Northern blot analysis involves the separation of RNA and hybridization of a complementary labeled probe.

In still further embodiments, RNA (or corresponding cDNA) is detected by hybridization to a oligonucleotide probe). A variety of hybridization assays using a variety of technologies for hybridization and detection are available. For example, in some embodiments, TaqMan assay (PE Biosystems, Foster City, CA; *See e.g.,* U.S. Patent Nos. 5,962,233 and 5,538,848, each of which is herein incorporated by reference) is utilized. The assay is performed during a PCR reaction. The TaqMan assay exploits the 5'-3' exonuclease activity of the AMPLITAQ GOLD DNA polymerase. A probe consisting of an oligonucleotide with a 5'-reporter dye (*e.g*., a fluorescent dye) and a 3'-quencher dye is included in the PCR reaction. During PCR, if the probe is bound to its target, the 5'-3' nucleolytic activity of the AMPLITAQ GOLD polymerase cleaves the probe between the reporter and the quencher dye. The separation of the reporter dye from the quencher dye results in an increase of fluorescence. The signal accumulates with each cycle of PCR and can be monitored with a fluorimeter.

In yet other embodiments, reverse-transcriptase PCR (RT-PCR) is used to detect the expression of RNA. In RT-PCR, RNA is enzymatically converted to complementary DNA or "cDNA" using a reverse transcriptase enzyme. The cDNA is then used as a template for a PCR reaction. PCR products can be detected by any suitable method, including but not limited to, gel electrophoresis and staining with a DNA specific stain or hybridization to a labeled probe. In some embodiments, the quantitative reverse transcriptase PCR with standardized mixtures of competitive templates method described in U.S. Patents 5,639,606, 5,643,765, and 5,876,978 (each of which is herein incorporated by reference) is utilized.

### 2. Detection of Protein

In other embodiments, gene expression of stem cell cancer markers is detected by measuring the expression of the corresponding protein or polypeptide. Protein expression may be detected by any suitable method. In some embodiments, proteins are detected by immunohistochemistry. In other embodiments, proteins are detected by their binding to an antibody raised against the protein. The generation of antibodies is described below.

Antibody binding is detected by techniques known in the art (*e.g*., radioimmunoassay, ELISA (enzyme-linked immunosorbant assay), "sandwich" immunoassays, immunoradiometric assays, gel diffusion precipitation reactions, immunodiffusion assays, *in situ* immunoassays (*e.g*., using colloidal gold, enzyme or radioisotope labels, for example), Western blots, precipitation reactions, agglutination assays (*e.g*., gel agglutination assays, hemagglutination assays, etc.), complement fixation assays, immunofluorescence assays, protein A assays, and immunoelectrophoresis assays, etc.

In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many methods are known in the art for detecting binding in an immunoassay and are within the scope of the present invention.

In some embodiments, an automated detection assay is utilized. Methods for the automation of immunoassays include those described in U.S. Patents 5,885,530, 4,981,785, 6,159,750, and 5,358,691, each of which is herein incorporated by reference. In some embodiments, the analysis and presentation of results is also automated. For example, in some embodiments, software that generates a prognosis based on the presence or absence of a series of proteins corresponding to cancer markers is utilized.

In other embodiments, the immunoassay described in U.S. Patents 5,599,677 and 5,672,480; each of which is herein incorporated by reference.

### 3. Data Analysis

In some embodiments, a computer-based analysis program is used to translate the raw data generated by the detection assay (*e.g*., the presence, absence, or amount of a given marker or markers) into data of predictive value for a clinician. The clinician can access the predictive data using any suitable means. Thus, in some preferred embodiments, the present invention provides the further benefit that the clinician, who is not likely to be trained in genetics or molecular biology, need not understand the raw data. The data is presented directly to the clinician in its most useful form. The clinician is then able to immediately utilize the information in order to optimize the care of the subject.

The present invention contemplates any method capable of receiving, processing, and transmitting the information to and from laboratories conducting the assays, information provides, medical personal, and subjects. For example, in some embodiments of the present invention, a sample (*e.g*., a biopsy or a serum or urine sample) is obtained from a subject and submitted to a profiling service (*e.g*., clinical lab at a medical facility, genomic profiling business, etc.), located in any part of the world (*e.g*., in a country different than the country where the subject resides or where the information is ultimately used) to generate raw data. Where the sample comprises a tissue or other biological sample, the subject may visit a medical center to have the sample obtained and sent to the profiling center, or subjects may collect the sample themselves and directly send it to a profiling center. Where the sample comprises previously determined biological information, the information may be directly sent to the profiling service by the subject (*e.g*., an information card containing the information may be scanned by a computer and the data transmitted to a computer of the profiling center using an electronic communication systems). Once received by the profiling service, the sample is processed and a profile is produced (*e.g*., expression data), specific for the diagnostic or prognostic information desired for the subj ect.

The profile data is then prepared in a format suitable for interpretation by a treating clinician. For example, rather than providing raw expression data (e.g. examining a number of the markers described in Tables 4-8), the prepared format may represent a diagnosis or risk assessment for the subject, along with recommendations for particular treatment options. The data may be displayed to the clinician by any suitable method. For example, in some embodiments, the profiling service generates a report that can be printed for the clinician (*e.g*., at the point of care) or displayed to the clinician on a computer monitor.

In some embodiments, the information is first analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.

In some embodiments, the subject is able to directly access the data using the electronic communication system. The subject may chose further intervention or counseling based on the results. In some embodiments, the data is used for research use. For example, the data may be used to further optimize the inclusion or elimination of markers as useful indicators of a particular condition or stage of disease.

### 4. Kits

In yet other embodiments, the present invention provides kits for the detection and characterization of cancer (e.g. for detecting one or more of the markers shown in Tables 4-8, or for modulating the activity of a peptide expressed by one or more of markes shown in Tables 4-8). In some embodiments, the kits contain antibodies specific for a cancer marker, in addition to detection reagents and buffers. In other embodiments, the kits contain reagents specific for the detection of mRNA or cDNA (*e.g*., oligonucleotide probes or primers). In preferred embodiments, the kits contain all of the components necessary to perform a detection assay, including all controls, directions for performing assays, and any necessary software for analysis and presentation of results.

### 5. In vivo Imaging

In some embodiments, in vivo imaging techniques are used to visualize the expression of cancer markers in an animal (*e.g.*, a human or non-human mammal). For example, in some embodiments, cancer marker mRNA or protein is labeled using an labeled antibody specific for the cancer marker. A specifically bound and labeled antibody can be detected in an individual using an *in vivo* imaging method, including, but not limited to, radionuclide imaging, positron emission tomography, computerized axial tomography, X-ray or magnetic resonance imaging method, fluorescence detection, and chemiluminescent detection. Methods for generating antibodies to the cancer markers of the present invention are described below.

The in vivo imaging methods of the present invention are useful in the diagnosis of cancers that express the solid tumor stem cell cancer markers of the present invention (*e.g*., in breast cancer). In vivo imaging is used to visualize the presence of a marker indicative of the cancer. Such techniques allow for diagnosis without the use of an unpleasant biopsy. The in vivo imaging methods of the present invention are also useful for providing prognoses to cancer patients. For example, the presence of a marker indicative of cancer stem cells can be detected. The in vivo imaging methods of the present invention can further be used to detect metastatic cancers in other parts of the body.

In some embodiments, reagents (*e.g*., antibodies) specific for the cancer markers of the present invention are fluorescently labeled. The labeled antibodies are introduced into a subject (*e.g*., orally or parenterally). Fluorescently labeled antibodies are detected using any suitable method (*e.g*., using the apparatus described in U.S. Patent 6,198,107, herein incorporated by reference).

In other embodiments, antibodies are radioactively labeled. The use of antibodies for in vivo diagnosis is well known in the art. Sumerdon et al., (Nucl. Med. Biol 17:247-254 [1990] have described an optimized antibody-chelator for the radioimmunoscintographic imaging of tumors using Indium-111 as the label. Griffin et al., (J Clin One 9:631-640 [1991]) have described the use of this agent in detecting tumors in patients suspected of having recurrent colorectal cancer. The use of similar agents with paramagnetic ions as labels for magnetic resonance imaging is known in the art (Lauffer, Magnetic Resonance in Medicine 22:339-342 [1991]). The label used will depend on the imaging modality chosen. Radioactive labels such as Indium-111, Technetium-99m, or Iodine-131 can be used for planar scans or single photon emission computed tomography (SPECT). Positron emitting labels such as Fluorine-19 can also be used for positron emission tomography (PET). For MRI, paramagnetic ions such as Gadolinium (III) or Manganese (II) can be used.

Radioactive metals with half-lives ranging from 1 hour to 3.5 days are available for conjugation to antibodies, such as scandium-47 (3.5 days) gallium-67 (2.8 days), gallium-68 (68 minutes), technetiium-99m (6 hours), and indium-111 (3.2 days), of which gallium-67, technetium-99m, and indium-111 are preferable for gamma camera imaging, gallium-68 is preferable for positron emission tomography.

A useful method of labeling antibodies with such radiometals is by means of a bifunctional chelating agent, such as diethylenetriaminepentaacetic acid (DTPA), as described, for example, by Khaw et al. (Science 209:295 [1980]) for In-111 and Tc-99m, and by Scheinberg et al. (Science 215:1511 [1982]). Other chelating agents may also be used, but the 1-(p-carboxymethoxybenzyl)EDTA and the carboxycarbonic anhydride of DTPA are advantageous because their use permits conjugation without affecting the antibody's immunoreactivity substantially.

Another method for coupling DPTA to proteins is by use of the cyclic anhydride of DTPA, as described by Hnatowich et al. (Int. J. Appl. Radiat. Isot. 33:327 [1982]) for labeling of albumin with In-111, but which can be adapted for labeling of antibodies. A suitable method of labeling antibodies with Tc-99m which does not use chelation with DPTA is the pretinning method of Crockford *et al.,* (U.S. Pat. No. 4,323,546, herein incorporated by reference).

A preferred method of labeling immunoglobulins with Tc-99m is that described by Wong et al. (Int. J. Appl. Radiat. Isot., 29:251 [1978]) for plasma protein, and recently applied successfully by Wong et al. (J. Nucl. Med., 23:229 [1981]) for labeling antibodies.

In the case of the radiometals conjugated to the specific antibody, it is likewise desirable to introduce as high a proportion of the radiolabel as possible into the antibody molecule without destroying its immunospecificity. A further improvement may be achieved by effecting radiolabeling in the presence of the specific stem cell cancer marker of the present invention, to insure that the antigen binding site on the antibody will be protected.

In still further embodiments, *in vivo* biophotonic imaging (Xenogen, Almeda, CA) is utilized for in vivo imaging. This real-time *in vivo* imaging utilizes luciferase. The luciferase gene is incorporated into cells, microorganisms, and animals (*e.g*., as a fusion protein with a cancer marker of the present invention). When active, it leads to a reaction that emits light. A CCD camera and software is used to capture the image and analyze it.

### V. Antibodies and Antibody Fragments

The present invention provides isolated antibodies and antibody fragments (e.g, Fabs). In preferred embodiments, the present invention provides monoclonal antibodies or antibody fragments that specifically bind to an isolated polypeptide comprised of at least five, or at least 15 amino acid residues of the stem cell cancer markers described herein (*e.g*., as shown in Tables 4-8). These antibodies or antibody fragments find use in the diagnostic, drug screening, and therapetuic methods described herein (e.g. to detect or modulate the activity of a stem cell cancer marker peptide).

An antibody, or antibody fragment, against a protein of the present invention may be any monoclonal or polyclonal antibody, as long as it can recognize the protein. Antibodies can be produced by using a protein of the present invention as the antigen according to a conventional antibody or antiserum preparation process.

The present invention contemplates the use of both monoclonal and polyclonal antibodies. Any suitable method may be used to generate the antibodies used in the methods and compositions of the present invention, including but not limited to, those disclosed herein. For example, for preparation of a monoclonal antibody, protein, as such, or together with a suitable carrier or diluent is administered to an animal (*e.g*., a mammal) under conditions that permit the production of antibodies. For enhancing the antibody production capability, complete or incomplete Freund's adjuvant may be administered. Normally, the protein is administered once every 2 weeks to 6 weeks, in total, about 2 times to about 10 times. Animals suitable for use in such methods include, but are not limited to, primates, rabbits, dogs, guinea pigs, mice, rats, sheep, goats, etc.

For preparing monoclonal antibody-producing cells, an individual animal whose antibody titer has been confirmed (*e.g*., a mouse) is selected, and 2 days to 5 days after the final immunization, its spleen or lymph node is harvested and antibody-producing cells contained therein are fused with myeloma cells to prepare the desired monoclonal antibody producer hybridoma. Measurement of the antibody titer in antiserum can be carried out, for example, by reacting the labeled protein, as described hereinafter and antiserum and then measuring the activity of the labeling agent bound to the antibody. The cell fusion can be carried out according to known methods, for example, the method described by Koehler and Milstein (Nature 256:495 [1975]). As a fusion promoter, for example, polyethylene glycol (PEG) or Sendai virus (HVJ), preferably PEG is used.

Examples of myeloma cells include NS-1, P3U1, SP2/0, AP-1 and the like. The proportion of the number of antibody producer cells (spleen cells) and the number of myeloma cells to be used is preferably about 1:1 to about 20:1. PEG (preferably PEG 1000-PEG 6000) is preferably added in concentration of about 10% to about 80%. Cell fusion can be carried out efficiently by incubating a mixture of both cells at about 20°C to about 40°C, preferably about 30°C to about 37°C for about 1 minute to 10 minutes.

Various methods may be used for screening for a hybridoma producing the antibody (*e.g*., against a tumor antigen or autoantibody of the present invention). For example, where a supernatant of the hybridoma is added to a solid phase (*e.g*., microplate) to which antibody is adsorbed directly or together with a carrier and then an anti-immunoglobulin antibody (if mouse cells are used in cell fusion, anti-mouse immunoglobulin antibody is used) or Protein A labeled with a radioactive substance or an enzyme is added to detect the monoclonal antibody against the protein bound to the solid phase. Alternately, a supernatant of the hybridoma is added to a solid phase to which an anti-immunoglobulin antibody or Protein A is adsorbed and then the protein labeled with a radioactive substance or an enzyme is added to detect the monoclonal antibody against the protein bound to the solid phase.

Selection of the monoclonal antibody can be carried out according to any known method or its modification. Normally, a medium for animal cells to which HAT (hypoxanthine, aminopterin, thymidine) are added is employed. Any selection and growth medium can be employed as long as the hybridoma can grow. For example, RPMI 1640 medium containing 1% to 20%, preferably 10% to 20% fetal bovine serum, GIT medium containing 1% to 10% fetal bovine serum, a serum free medium for cultivation of a hybridoma (SFM-101, Nissui Seiyaku) and the like can be used. Normally, the cultivation is carried out at 20°C to 40°C, preferably 37°C for about 5 days to 3 weeks, preferably 1 week to 2 weeks under about 5% CO₂ gas. The antibody titer of the supernatant of a hybridoma culture can be measured according to the same manner as described above with respect to the antibody titer of the anti-protein in the antiserum.

Separation and purification of a monoclonal antibody (*e.g*., against a cancer marker of the present invention) can be carried out according to the same manner as those of conventional polyclonal antibodies such as separation and purification of immunoglobulins, for example, salting-out, alcoholic precipitation, isoelectric point precipitation, electrophoresis, adsorption and desorption with ion exchangers (*e.g*., DEAE), ultracentrifugation, gel filtration, or a specific purification method wherein only an antibody is collected with an active adsorbent such as an antigen-binding solid phase, Protein A or Protein G and dissociating the binding to obtain the antibody.

Polyclonal antibodies may be prepared by any known method or modifications of these methods including obtaining antibodies from patients. For example, a complex of an immunogen (an antigen against the protein) and a carrier protein is prepared and an animal is immunized by the complex according to the same manner as that described with respect to the above monoclonal antibody preparation. A material containing the antibody against is recovered from the immunized animal and the antibody is separated and purified.

As to the complex of the immunogen and the carrier protein to be used for immunization of an animal, any carrier protein and any mixing proportion of the carrier and a hapten can be employed as long as an antibody against the hapten, which is crosslinked on the carrier and used for immunization, is produced efficiently. For example, bovine serum albumin, bovine cycloglobulin, keyhole limpet hemocyanin, etc. may be coupled to an hapten in a weight ratio of about 0.1 part to about 20 parts, preferably, about 1 part to about 5 parts per 1 part of the hapten.

In addition, various condensing agents can be used for coupling of a hapten and a carrier. For example, glutaraldehyde, carbodiimide, maleimide activated ester, activated ester reagents containing thiol group or dithiopyridyl group, and the like find use with the present invention. The condensation product as such or together with a suitable carrier or diluent is administered to a site of an animal that permits the antibody production. For enhancing the antibody production capability, complete or incomplete Freund's adjuvant may be administered. Normally, the protein is administered once every 2 weeks to 6 weeks, in total, about 3 times to about 10 times.

The polyclonal antibody is recovered from blood, ascites and the like, of an animal immunized by the above method. The antibody titer in the antiserum can be measured according to the same manner as that described above with respect to the supernatant of the hybridoma culture. Separation and purification of the antibody can be carried out according to the same separation and purification method of immunoglobulin as that described with respect to the above monoclonal antibody.

The protein used herein as the immunogen is not limited to any particular type of immunogen. For example, a stem cell cancer marker of the present invention (further including a gene having a nucleotide sequence partly altered) can be used as the immunogen. Further, fragments of the protein may be used. Fragments may be obtained by any methods including, but not limited to expressing a fragment of the gene, enzymatic processing of the protein, chemical synthesis, and the like. The antibodies and antibody fragments may also be conjugated to therapeutic (e.g. cancer cell killing compounds). In this regard, the antibody directed toward one of the stem cell cancer markers is used to specifically deliver a therapeutic agent to a solid tumor cancer cell (e.g. to inhibit the proliferation of such sell or kill such a cell).

### VI. Drug Screening

In some embodiments, the present invention provides drug screening assays (*e.g*., to screen for anticancer drugs). The screening methods of the present invention utilize stem cell cancer markers identified using the methods of the present invention (*e.g*., including but not limited to, the stem cell cancer markers shown in Tables 4-8). For example, in some embodiments, the present invention provides methods of screening for compound that alter (*e.g*., increase or decrease) the expression of stem cell cancer marker genes. In some embodiments, candidate compounds are antisense agents or siRNA agents (*e.g*., oligonucleotides) directed against cancer markers. In other embodiments, candidate compounds are antibodies that specifically bind to a stem cell cancer marker of the present invention. In certain embodiments, libraries of compounds of small molecules are screened using the methods described herein.

In one screening method, candidate compounds are evaluated for their ability to alter stem cell cancer marker expression by contacting a compound with a cell expressing a stem cell cancer marker and then assaying for the effect of the candidate compounds on expression. In some embodiments, the effect of candidate compounds on expression of a cancer marker gene is assayed by detecting the level of cancer marker mRNA expressed by the cell. mRNA expression can be detected by any suitable method. In other embodiments, the effect of candidate compounds on expression of cancer marker genes is assayed by measuring the level of polypeptide encoded by the cancer markers. The level of polypeptide expressed can be measured using any suitable method, including but not limited to, those disclosed herein. In some embodiments, other changes in cel biology (e.g., apoptosis) are detected.

Specifically, the present invention provides screening methods for identifying modulators, *i.e*., candidate or test compounds or agents (*e.g*., proteins, peptides, peptidomimetics, peptoids, small molecules or other drugs) which bind to, or alter the signalling or function associated with the cancer markers of the present invention, have an inhibitory (or stimulatory) effect on, for example, stem cell cancer marker expression or cancer markers activity, or have a stimulatory or inhibitory effect on, for example, the expression or activity of a cancer marker substrate. Compounds thus identified can be used to modulate the activity of target gene products (*e.g*., stem cell cancer marker genes) either directly or indirectly in a therapeutic protocol, to elaborate the biological function of the target gene product, or to identify compounds that disrupt normal target gene interactions. Compounds which inhibit the activity or expression of cancer markers are useful in the treatment of proliferative disorders, *e.g*., cancer, particularly metastatic cancer or eliminating or controlling tumor stem cells to prevent or reduce the risk of cancer.

In one embodiment, the invention provides assays for screening candidate or test compounds that are substrates of a cancer markers protein or polypeptide or a biologically active portion thereof. In another embodiment, the invention provides assays for screening candidate or test compounds that bind to or modulate the activity of a cancer marker protein or polypeptide or a biologically active portion thereof.

The test compounds of the present invention can be obtained using any of the numerous approaches in combinatorial library methods known in the art, including biological libraries; peptoid libraries (libraries of molecules having the functionalities of peptides, but with a novel, non-peptide backbone, which are resistant to enzymatic degradation but which nevertheless remain bioactive; see, *e.g*., Zuckennann et al., J. Med. Chem. 37: 2678-85 [1994]); spatially addressable parallel solid phase or solution phase libraries; synthetic library methods requiring deconvolution; the 'one-bead one-compound' library method; and synthetic library methods using affinity chromatography selection. The biological library and peptoid library approaches are preferred for use with peptide libraries, while the other four approaches are applicable to peptide, non-peptide oligomer or small molecule libraries of compounds (Lam (1997) Anticancer Drug Des. 12:145).

Examples of methods for the synthesis of molecular libraries can be found in the art, for example in: DeWitt et al., Proc. Natl. Acad. Sci. U.S.A. 90:6909 [1993]; Erb et al., Proc. Nad. Acad. Sci. USA 91:11422 [1994]; Zuckermann et al., J. Med. Chem. 37:2678 [1994]; Cho et al., Science 261:1303 [1993]; Carrell et al., Angew. Chem. Int. Ed. Engl. 33.2059 [1994]; Carell et al., Angew. Chem. Int. Ed. Engl. 33:2061 [1994]; and Gallop et al., J. Med. Chem. 37:1233 [1994].

Libraries of compounds may be presented in solution (*e.g*., Houghten, Biotechniques 13:412-421 [1992]), or on beads (Lam, Nature 354:82-84 [1991]), chips (Fodor, Nature 364:555-556 [1993]), bacteria or spores (U.S. Patent No. 5,223,409; herein incorporated by reference), plasmids (Cull et al., Proc. Nad. Acad. Sci. USA 89:18651869 [1992]) or on phage (Scott and Smith, Science 249:386-390 [1990]; Devlin Science 249:404-406 [1990]; Cwirla et al., Proc. Natl. Acad. Sci. 87:6378-6382 [1990]; Felici, J. Mol. Biol. 222:301 [1991]).

In one embodiment, an assay is a cell-based assay in which a cell that expresses a stem cell cancer marker protein or biologically active portion thereof is contacted with a test compound, and the ability of the test compound to the modulate cancer marker's activity is determined. Determining the ability of the test compound to modulate stem cell cancer marker activity can be accomplished by monitoring, for example, changes in enzymatic activity. The cell, for example, can be of mammalian origin.

The ability of the test compound to modulate cancer marker binding to a compound, *e.g*., a stem cell cancer marker substrate, can also be evaluated. This can be accomplished, for example, by coupling the compound, *e.g*., the substrate, with a radioisotope or enzymatic label such that binding of the compound, *e.g*., the substrate, to a cancer marker can be determined by detecting the labeled compound, *e.g*., substrate, in a complex.

Alternatively, the stem cell cancer marker is coupled with a radioisotope or enzymatic label to monitor the ability of a test compound to modulate cancer marker binding to a cancer markers substrate in a complex. For example, compounds (*e.g*., substrates) can be labeled with ¹²⁵I, ³⁵S ¹⁴C or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (*e.g*., a stem cell cancer marker substrate) to interact with a stem cell cancer marker with or without the labeling of any of the interactants can be evaluated. For example, a microphysiorneter can be used to detect the interaction of a compound with a cancer marker without the labeling of either the compound or the cancer marker (McConnell et al. Science 257:1906-1912 [1992]). As used herein, a "microphysiometer" (*e.g*., Cytosensor) is an analytical instrument that measures the rate at which a cell acidifies its environment using a light-addressable potentiometric sensor (LAPS). Changes in this acidification rate can be used as an indicator of the interaction between a compound and cancer markers.

In yet another embodiment, a cell-free assay is provided in which a cancer marker protein or biologically active portion thereof is contacted with a test compound and the ability of the test compound to bind to the stem cell cancer marker protein or biologically active portion thereof is evaluated. Preferred biologically active portions of the cancer markers proteins to be used in assays of the present invention include fragments that participate in interactions with substrates or other proteins, *e.g.*, fragments with high surface probability scores.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, *e.g*., using fluorescence energy transfer (FRET) (see, for example, Lakowicz et al., U.S. Patent No. 5,631,169; Stavrianopoulos et al., U.S. Patent No. 4,968,103; each of which is herein incorporated by reference). A fluorophore label is selected such that a first donor molecule's emitted fluorescent energy will be absorbed by a fluorescent label on a second, 'acceptor' molecule, which in turn is able to fluoresce due to the absorbed energy.

Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in 1 5 the assay should be maximal. An FRET binding event can be conveniently measured through standard fluorometric detection means well known in the art (*e.g*., using a fluorimeter).

In another embodiment, determining the ability of the stem cell cancer markers protein to bind to a target molecule can be accomplished using real-time Biomolecular Interaction Analysis (BIA) (*see, e.g.,* Sjolander and Urbaniczky, Anal. Chem. 63:2338-2345 [1991] and Szabo et al. Curr. Opin. Struct. Biol. 5:699-705 [1995]). "Surface plasmon resonance" or "BIA" detects biospecific interactions in real time, without labeling any of the interactants (*e.g*., BlAcore). Changes in the mass at the binding surface (indicative of a binding event) result in alterations of the refractive index of light near the surface (the optical phenomenon of surface plasmon resonance (SPR)), resulting in a detectable signal that can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize stem cell cancer markers, an anti-cancer marker antibody or its target molecule to facilitate separation of complexed from non-complexed forms of one or both of the proteins, as well as to accommodate automation of the assay. Binding of a test compound to a stem cell cancer marker protein, or interaction of a cancer marker protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase-cancer marker fusion proteins or glutathione-S-transferase/target fusion proteins can be adsorbed onto glutathione Sepharose beads (Sigma Chemical, St. Louis, MO) or glutathione-derivatized microtiter plates, which are then combined with the test compound or the test compound and either the non-adsorbed target protein or cancer marker protein, and the mixture incubated under conditions conducive for complex formation (*e.g*., at physiological conditions for salt and pH). Following incubation, the beads or microtiter plate wells are washed to remove any unbound components, the matrix immobilized in the case of beads, complex determined either directly or indirectly, for example, as described above.

Alternatively, the complexes can be dissociated from the matrix, and the level of cancer markers binding or activity determined using standard techniques. Other techniques for immobilizing either cancer markers protein or a target molecule on matrices include using conjugation of biotin and streptavidin. Biotinylated cancer marker protein or target molecules can be prepared from biotin-NHS (N-hydroxysuccinimide) using techniques known in the art (*e.g*., biotinylation kit, Pierce Chemicals, Rockford, EL), and immobilized in the wells of streptavidin-coated 96 well plates (Pierce Chemical).

In order to conduct the assay, the non-immobilized component is added to the coated surface containing the anchored component. After the reaction is complete, unreacted components are removed (*e.g*., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; *e.g*., using a labeled antibody specific for the immobilized component (the antibody, in turn, can be directly labeled or indirectly labeled with, *e.g*., a labeled anti-IgG antibody).

This assay is performed utilizing antibodies reactive with stem cell cancer marker protein or target molecules but which do not interfere with binding of the stem cell cancer markers protein to its target molecule. Such antibodies can be derivatized to the wells of the plate, and unbound target or cancer markers protein trapped in the wells by antibody conjugation. Methods for detecting such complexes, in addition to those described above for the GST-immobilized complexes, include immunodetection of complexes using antibodies reactive with the cancer marker protein or target molecule, as well as enzyme-linked assays which rely on detecting an enzymatic activity associated with the cancer marker protein or target molecule.

Alternatively, cell free assays can be conducted in a liquid phase. In such an assay, the reaction products are separated from unreacted components, by any of a number of standard techniques, including, but not limited to: differential centrifugation (see, for example, Rivas and Minton, Trends Biochem Sci 18:284-7 [1993]); chromatography (gel filtration chromatography, ion-exchange chromatography); electrophoresis (*see, e.g.,* Ausubel et al., eds. Current Protocols in Molecular Biology 1999, J. Wiley: New York.); and immunoprecipitation (*see*, for example, Ausubel et al., eds. Current Protocols in Molecular Biology 1999, J. Wiley: New York). Such resins and chromatographic techniques are known to one skilled in the art (*See e.g.,* Heegaard J. Mol. Recognit 11:141-8 [1998]; Hageand Tweed J. Chromatogr. Biomed. Sci. Appl 699:499-525 [1997]). Further, fluorescence energy transfer may also be conveniently utilized, as described herein, to detect binding without further purification of the complex from solution.

The assay can include contacting the stem cell cancer markers protein or biologically active portion thereof with a known compound that binds the cancer marker to form an assay mixture, contacting the assay mixture with a test compound, and determining the ability of the test compound to interact with a cancer marker protein, wherein determining the ability of the test compound to interact with a cancer marker protein includes determining the ability of the test compound to preferentially bind to cancer markers or biologically active portion thereof, or to modulate the activity of a target molecule, as compared to the known compound.

To the extent that stem cell cancer markers can, *in vivo,* interact with one or more cellular or extracellular macromolecules, such as proteins, inhibitors of such an interaction are useful. A homogeneous assay can be used can be used to identify inhibitors

For example, a preformed complex of the target gene product and the interactive cellular or extracellular binding partner product is prepared such that either the target gene products or their binding partners are labeled, but the signal generated by the label is quenched due to complex formation *(*see*, e.g.,* U.S. Patent No. 4,109,496, herein incorporated by reference, that utilizes this approach for immunoassays). The addition of a test substance that competes with and displaces one of the species from the preformed complex will result in the generation of a signal above background. In this way, test substances that disrupt target gene product-binding partner interaction can be identified. Alternatively, cancer markers protein can be used as a "bait protein" in a two-hybrid assay or three-hybrid assay (*see, e.g.,* U.S. Patent No. 5,283,317; Zervos et al., Cell 72:223-232 [1993]; Madura et al., J. Biol. Chem. 268.12046-12054 [1993]; Bartel et al., Biotechniques 14:920-924 [1993]; Iwabuchi et al., Oncogene 8:1693-1696 [1993]; and Brent W0 94/10300; each of which is herein incorporated by reference), to identify other proteins, that bind to or interact with cancer markers ("cancer marker-binding proteins" or "cancer marker-bp") and are involved in cancer marker activity. Such cancer marker-bps can be activators or inhibitors of signals by the cancer marker proteins or targets as, for example, downstream elements of a cancer markers-mediated signaling pathway.

Modulators of cancer markers expression can also be identified. For example, a cell or cell free mixture is contacted with a candidate compound and the expression of cancer marker mRNA or protein evaluated relative to the level of expression of stem cell cancer marker mRNA or protein in the absence of the candidate compound. When expression of cancer marker mRNA or protein is greater in the presence of the candidate compound than in its absence, the candidate compound is identified as a stimulator of cancer marker mRNA or protein expression. Alternatively, when expression of cancer marker mRNA or protein is less (i.e., statistically significantly less) in the presence of the candidate compound than in its absence, the candidate compound is identified as an inhibitor of cancer marker mRNA or protein expression. The level of cancer markers mRNA or protein expression can be determined by methods described herein for detecting cancer markers mRNA or protein.

A modulating agent can be identified using a cell-based or a cell free assay, and the ability of the agent to modulate the activity of a cancer markers protein can be confirmed *in vivo, e.g.,* in an animal such as an animal model for a disease (*e.g*., an animal with prostate cancer or metastatic prostate cancer; or an animal harboring a xenograft of a prostate cancer from an animal (*e.g*., human) or cells from a cancer resulting from metastasis of a prostate cancer (*e.g*., to a lymph node, bone, or liver), or cells from a prostate cancer cell line.

This invention further pertains to novel agents identified by the above-described screening assays (*See e.g.,* below description of cancer therapies). Accordingly, it is within the scope of this invention to further use an agent identified as described herein (*e.g*., a cancer marker modulating agent, an antisense cancer marker nucleic acid molecule, a siRNA molecule, a cancer marker specific antibody, or a cancer marker-binding partner) in an appropriate animal model (such as those described herein) to determine the efficacy, toxicity, side effects, or mechanism of action, of treatment with such an agent. Furthermore, novel agents identified by the above-described screening assays can be, *e.g*., used for treatments as described herein (e.g. to treat a human patient who has cancer).

### VII. Cancer Therapies

In some embodiments, the present invention provides therapies for cancer (*e.g*., breast cancer). In some embodiments, therapies target cancer markers (*e.g*., including but not limited to, those shown in Tables 4-8).

### A. Antisense Therapies

Candidate therapeutic agents also find use in drug screening and research applications. In some embodiments, the present invention targets the expression of stem cell cancer markers. For example, in some embodiments, the present invention employs compositions comprising oligomeric antisense compounds, particularly oligonucleotides (*e.g*., those identified in the drug screening methods described above), for use in modulating the function of nucleic acid molecules encoding stem cell cancer markers of the present invention, ultimately modulating the amount of cancer marker expressed. This is accomplished by providing antisense compounds that specifically hybridize with one or more nucleic acids encoding cancer markers of the present invention. The specific hybridization of an oligomeric compound with its target nucleic acid interferes with the normal function of the nucleic acid. This modulation of function of a target nucleic acid by compounds that specifically hybridize to it is generally referred to as "antisense." The functions of DNA to be interfered with include replication and transcription. The functions of RNA to be interfered with include all vital functions such as, for example, translocation of the RNA to the site of protein translation, translation of protein from the RNA, splicing of the RNA to yield one or more mRNA species, and catalytic activity that may be engaged in or facilitated by the RNA. The overall effect of such interference with target nucleic acid function is modulation of the expression of cancer markers of the present invention. In the context of the present invention, "modulation" means either an increase (stimulation) or a decrease (inhibition) in the expression of a gene. For example, expression may be inhibited to potentially prevent tumor proliferation.

It is preferred to target specific nucleic acids for antisense. "Targeting" an antisense compound to a particular nucleic acid, in the context of the present invention, is a multistep process. The process usually begins with the identification of a nucleic acid sequence whose function is to be modulated. This may be, for example, a cellular gene (or mRNA transcribed from the gene) whose expression is associated with a particular disorder or disease state, or a nucleic acid molecule from an infectious agent. In the present invention, the target is a nucleic acid molecule encoding a stem cell cancer marker of the present invention. The targeting process also includes determination of a site or sites within this gene for the antisense interaction to occur such that the desired effect, *e.g*., detection or modulation of expression of the protein, will result. Within the context of the present invention, a preferred intragenic site is the region encompassing the translation initiation or termination codon of the open reading frame (ORF) of the gene. Since the translation initiation codon is typically 5'-AUG (in transcribed mRNA molecules; 5'-ATG in the corresponding DNA molecule), the translation initiation codon is also referred to as the "AUG codon," the "start codon" or the "AUG start codon". A minority of genes have a translation initiation codon having the RNA sequence 5'-GUG, 5'-UUG or 5'-CUG, and 5'-AUA, 5'-ACG and 5'-CUG have been shown to function in vivo. Thus, the terms "translation initiation codon" and "start codon" can encompass many codon sequences, even though the initiator amino acid in each instance is typically methionine (in eukaryotes) or formylmethionine (in prokaryotes). Eukaryotic and prokaryotic genes may have two or more alternative start codons, any one of which may be preferentially utilized for translation initiation in a particular cell type or tissue, or under a particular set of conditions. In the context of the present invention, "start codon" and "translation initiation codon" refer to the codon or codons that are used *in vivo* to initiate translation of an mRNA molecule transcribed from a gene encoding a tumor antigen of the present invention, regardless of the sequence(s) of such codons.

Translation termination codon (or "stop codon") of a gene may have one of three sequences (*i.e.*, 5'-UAA, 5'-UAG and 5'-UGA; the corresponding DNA sequences are 5'-TAA, 5'-TAG and 5'-TGA, respectively). The terms "start codon region" and "translation initiation codon region" refer to a portion of such an mRNA or gene that encompasses from about 25 to about 50 contiguous nucleotides in either direction (*i.e*., 5' or 3') from a translation initiation codon. Similarly, the terms "stop codon region" and "translation termination codon region" refer to a portion of such an mRNA or gene that encompasses from about 25 to about 50 contiguous nucleotides in either direction (*i.e*., 5' or 3') from a translation termination codon.

The open reading frame (ORF) or "coding region," which refers to the region between the translation initiation codon and the translation termination codon, is also a region that may be targeted effectively. Other target regions include the 5' untranslated region (5' UTR), referring to the portion of an mRNA in the 5' direction from the translation initiation codon, and thus including nucleotides between the 5' cap site and the translation initiation codon of an mRNA or corresponding nucleotides on the gene, and the 3' untranslated region (3' UTR), referring to the portion of an mRNA in the 3' direction from the translation termination codon, and thus including nucleotides between the translation termination codon and 3' end of an mRNA or corresponding nucleotides on the gene. The 5' cap of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap. The cap region may also be a preferred target region.

Although some eukaryotic mRNA transcripts are directly translated, many contain one or more regions, known as "introns," that are excised from a transcript before it is translated. The remaining (and therefore translated) regions are known as "exons" and are spliced together to form a continuous mRNA sequence. mRNA splice sites (*i.e*., intron-exon junctions) may also be preferred target regions, and are particularly useful in situations where aberrant splicing is implicated in disease, or where an overproduction of a particular mRNA splice product is implicated in disease. Aberrant fusion junctions due to rearrangements or deletions are also preferred targets. It has also been found that introns can also be effective, and therefore preferred, target regions for antisense compounds targeted, for example, to DNA or pre-mRNA.

In some embodiments, target sites for antisense inhibition are identified using commercially available software programs (e.g., Biognostik, Gottingen, Germany; SysArris Software, Bangalore, India; Antisense Research Group, University of Liverpool, Liverpool, England; GeneTrove, Carlsbad, CA). In other embodiments, target sites for antisense inhibition are identified using the accessible site method described in U.S. Patent W00198537A2, herein incorporated by reference.

Once one or more target sites have been identified, oligonucleotides are chosen that are sufficiently complementary to the target (*i.e.*, hybridize sufficiently well and with sufficient specificity) to give the desired effect. For example, in preferred embodiments of the present invention, antisense oligonucleotides are targeted to or near the start codon.

In the context of this invention, "hybridization," with respect to antisense compositions and methods, means hydrogen bonding, which may be Watson-Crick, Hoogsteen or reversed Hoogsteen hydrogen bonding, between complementary nucleoside or nucleotide bases. For example, adenine and thymine are complementary nucleobases that pair through the formation of hydrogen bonds. It is understood that the sequence of an antisense compound need not be 100% complementary to that of its target nucleic acid to be specifically hybridizable. An antisense compound is specifically hybridizable when binding of the compound to the target DNA or RNA molecule interferes with the normal function of the target DNA or RNA to cause a loss of utility, and there is a sufficient degree of complementarity to avoid non-specific binding of the antisense compound to non-target sequences under conditions in which specific binding is desired (*i.e*., under physiological conditions in the case of *in vivo* assays or therapeutic treatment, and in the case of *in vitro* assays, under conditions in which the assays are performed).

Antisense compounds are commonly used as research reagents and diagnostics. For example, antisense oligonucleotides, which are able to inhibit gene expression with specificity, can be used to elucidate the function of particular genes. Antisense compounds are also used, for example, to distinguish between functions of various members of a biological pathway.

The specificity and sensitivity of antisense is also applied for therapeutic uses. For example, antisense oligonucleotides have been employed as therapeutic moieties in the treatment of disease states in animals and man. Antisense oligonucleotides have been safely and effectively administered to humans and numerous clinical trials are presently underway. It is thus established that oligonucleotides are useful therapeutic modalities that can be configured to be useful in treatment regimes for treatment of cells, tissues, and animals, especially humans.

While antisense oligonucleotides are a preferred form of antisense compound, the present invention comprehends other oligomeric antisense compounds, including but not limited to oligonucleotide mimetics such as are described below. The antisense compounds in accordance with this invention preferably comprise from about 8 to about 30 nucleobases (*i.e.*, from about 8 to about 30 linked bases), although both longer and shorter sequences may find use with the present invention. Particularly preferred antisense compounds are antisense oligonucleotides, even more preferably those comprising from about 12 to about 25 nucleobases.

Specific examples of preferred antisense compounds useful with the present invention include oligonucleotides containing modified backbones or non-natural internucleoside linkages. As defined in this specification, oligonucleotides having modified backbones include those that retain a phosphorus atom in the backbone and those that do not have a phosphorus atom in the backbone. For the purposes of this specification, modified oligonucleotides that do not have a phosphorus atom in their internucleoside backbone can also be considered to be oligonucleosides.

Preferred modified oligonucleotide backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms are also included.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts.

In other preferred oligonucleotide mimetics, both the sugar and the internucleoside linkage (*i.e.*, the backbone) of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S. Pat. Nos.: 5,539,082; 5,714,331; and 5,719,262, each of which is herein incorporated by reference. Further teaching of PNA compounds can be found in Nielsen et al., Science 254:1497 (1991).

Most preferred embodiments of the invention are oligonucleotides with phosphorothioate backbones and oligonucleosides with heteroatom backbones, and in particular --CH₂, --NH--O--CH₂--, --CH₂--N(CH₃)--O--CH₂-- [known as a methylene (methylimino) or MMI backbone], --CH₂--O--N(CH₃)--CH₂--, --CH₂--N(CH₃)--N(CH₃)--CH₂--, and --O--N(CH₃)--CH₂--CH₂-- [wherein the native phosphodiester backbone is represented as --O--P--O--CH₂--] of the above referenced U.S. Pat. No. 5,489,677, and the amide backbones of the above referenced U.S. Pat. No. 5,602,240. Also preferred are oligonucleotides having morpholino backbone structures of the above-referenced U.S. Pat. No. 5,034,506.

Modified oligonucleotides may also contain one or more substituted sugar moieties. Preferred oligonucleotides comprise one of the following at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. Particularly preferred are O[(CH₂)ₙO]ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃)]₂, where n and m are from 1 to about 10. Other preferred oligonucleotides comprise one of the following at the 2' position: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy (2'-O--CH₂CH₂OCH₃, also known as 2'-O-(2-methoxyethyl) or 2'-MOE) (Martin et al., Helv. Chim. Acta 78:486 [1995]) *i.e.,* an alkoxyalkoxy group. A further preferred modification includes 2'-dimethylaminooxyethoxy (*i.e.,* a O(CH₂)₂ON(CH₃)₂ group), also known as 2'-DMAOE, and 2'-dimethylaminoethoxyethoxy (also known in the art as 2'-O-dimethylaminoethoxyethyl or 2'-DMAEOE), *i.e*., 2'-O--CH₂--O--CH₂--N(CH₂)₂.

Other preferred modifications include 2'-methoxy(2'-O--CH₃), 2'-aminopropoxy(2'-OCH₂CH₂CH₂NH₂) and 2'-fluoro (2'-F). Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar.

Oligonucleotides may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Further nucleobases include those disclosed in U.S. Pat. No. 3,687,808. Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2. degree °C and are presently preferred base substitutions, even more particularly when combined with 2'-O-methoxyethyl sugar modifications.

Another modification of the oligonucleotides of the present invention involves chemically linking to the oligonucleotide one or more moieties or conjugates that enhance the activity, cellular distribution or cellular uptake of the oligonucleotide. Such moieties include but are not limited to lipid moieties such as a cholesterol moiety, cholic acid, a thioether, (*e.g*., hexyl-S-tritylthiol), a thiocholesterol, an aliphatic chain, (*e.g.*, dodecandiol or undecyl residues), a phospholipid, (*e.g*., di-hexadecyl-rac-glycerol or triethylammonium 1,2-di-O-hexadecyl-rac-glycero-3-H-phosphonate), a polyamine or a polyethylene glycol chain or adamantane acetic acid, a palmityl moiety, or an octadecylamine or hexylamino-carbonyl-oxycholesterol moiety.

One skilled in the relevant art knows well how to generate oligonucleotides containing the above-described modifications. The present invention is not limited to the antisensce oligonucleotides described above. Any suitable modification or substitution may be utilized.

It is not necessary for all positions in a given compound to be uniformly modified, and in fact more than one of the aforementioned modifications may be incorporated in a single compound or even at a single nucleoside within an oligonucleotide. The present invention also includes antisense compounds that are chimeric compounds. "Chimeric" antisense compounds or "chimeras," in the context of the present invention, are antisense compounds, particularly oligonucleotides, which contain two or more chemically distinct regions, each made up of at least one monomer unit, *i.e.*, a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNaseH is a cellular endonuclease that cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Cleavage of the RNA target can be routinely detected by gel electrophoresis and, if necessary, associated nucleic acid hybridization techniques known in the art.

Chimeric antisense compounds of the present invention may be formed as composite structures of two or more oligonucleotides, modified oligonucleotides, oligonucleosides and/or oligonucleotide mimetics as described above.

The present invention also includes pharmaceutical compositions and formulations that include the antisense compounds of the present invention as described below.

### B. Genetic Therapies

The present invention contemplates the use of any genetic manipulation for use in modulating the expression of stem cell cancer markers of the present invention. Examples of genetic manipulation include, but are not limited to, gene knockout (*e.g*., removing the cancer marker gene from the chromosome using, for example, recombination), expression of antisense constructs with or without inducible promoters, addition of a heterologous gene (e.g. controlled by an inducible promoter), and the like. Delivery of nucleic acid construct to cells *in vitro* or in *vivo* may be conducted using any suitable method. A suitable method is one that introduces the nucleic acid construct into the cell such that the desired event occurs (*e.g*., expression of an antisense construct).

Introduction of molecules carrying genetic information into cells is achieved by any of various methods including, but not limited to, directed injection of naked DNA constructs, bombardment with gold particles loaded with said constructs, and macromolecule mediated gene transfer using, for example, liposomes, biopolymers, and the like. Preferred methods use gene delivery vehicles derived from viruses, including, but not limited to, adenoviruses, retroviruses, vaccinia viruses, and adeno-associated viruses. Because of the higher efficiency as compared to retroviruses, vectors derived from adenoviruses are the preferred gene delivery vehicles for transferring nucleic acid molecules into host cells *in vivo.* Adenoviral vectors have been shown to provide very efficient *in vivo* gene transfer into a variety of solid tumors in animal models and into human solid tumor xenografts in immune-deficient mice. Examples of adenoviral vectors and methods for gene transfer are described in PCT publications WO 00/12738 and WO 00/09675 and U.S. Pat. Appl. Nos. 6,033,908, 6,019,978, 6,001,557, 5,994,132, 5,994,128, 5,994,106, 5,981,225, 5,885,808, 5,872,154, 5,830,730, and 5,824,544, each of which is herein incorporated by reference in its entirety.

Vectors may be administered to a subject in a variety of ways. For example, in some embodiments of the present invention, vectors are administered into tumors or tissue associated with tumors using direct injection. In other embodiments, administration is via the blood or lymphatic circulation (*See e.g.,* PCT publication 99/02685 herein incorporated by reference in its entirety). Exemplary dose levels of adenoviral vector are preferably 10⁸ to 10¹¹ vector particles added to the perfusate.

### C. Antibody Therapy

In some embodiments, the present invention provides antibodies that target tumors that express a stem cell cancer marker of the present invention (*e.g*., those shown in Tables 4-8). Any suitable antibody (*e.g*., monoclonal, polyclonal, or synthetic) may be utilized in the therapeutic methods disclosed herein. In preferred embodiments, the antibodies used for cancer therapy are humanized antibodies. Methods for humanizing antibodies are well known in the art (*See e.g.,* U.S. Patents 6,180,370, 5,585,089, 6,054,297, and 5,565,332; each of which is herein incorporated by reference).

In some embodiments, the therapeutic antibodies comprise an antibody generated against a stem cell cancer marker of the present invention, wherein the antibody is conjugated to a cytotoxic agent. In such embodiments, a tumor specific therapeutic agent is generated that does not target normal cells, thus reducing many of the detrimental side effects of traditional chemotherapy. For certain applications, it is envisioned that the therapeutic agents will be pharmacologic agents that will serve as useful agents for attachment to antibodies, particularly cytotoxic or otherwise anticellular agents having the ability to kill or suppress the growth or cell division of endothelial cells. The present invention contemplates the use of any pharmacologic agent that can be conjugated to an antibody, and delivered in active form. Exemplary anticellular agents include chemotherapeutic agents, radioisotopes, and cytotoxins. The therapeutic antibodies of the present invention may include a variety of cytotoxic moieties, including but not limited to, radioactive isotopes (*e.g*., iodine-131, iodine-123, technicium-99m, indium-111, rhenium-188, rhenium-186, gallium-67, copper-67, yttrium-90, iodine-125 or astatine-211), hormones such as a steroid, antimetabolites such as cytosines (*e.g*., arabinoside, fluorouracil, methotrexate or aminopterin; an anthracycline; mitomycin C), vinca alkaloids (*e.g*., demecolcine; etoposide; mithramycin), and antitumor alkylating agent such as chlorambucil or melphalan. Other embodiments may include agents such as a coagulant, a cytokine, growth factor, bacterial endotoxin or the lipid A moiety of bacterial endotoxin. For example, in some embodiments, therapeutic agents will include plant-, fungus- or bacteria-derived toxin, such as an A chain toxins, a ribosome inactivating protein, α - sarcin, aspergillin, restrictocin, a ribonuclease, diphtheria toxin or pseudomonas exotoxin, to mention just a few examples. In some preferred embodiments, deglycosylated ricin A chain is utilized.

In any event, it is proposed that agents such as these may, if desired, be successfully conjugated to an antibody, in a manner that will allow their targeting, internalization, release or presentation to blood components at the site of the targeted tumor cells as required using known conjugation technology (*See, e.g.,* Ghose et al., Methods Enzymol., 93:280 [1983]).

For example, in some embodiments the present invention provides immunotoxins targeted a stem cell cancer marker of the present invention. Immunotoxins are conjugates of a specific targeting agent typically a tumor-directed antibody or fragment, with a cytotoxic agent, such as a toxin moiety. The targeting agent directs the toxin to, and thereby selectively kills, cells carrying the targeted antigen. In some embodiments, therapeutic antibodies employ crosslinkers that provide high *in vivo* stability (Thorpe et al., Cancer Res., 48:6396 [1988]).

In other embodiments, particularly those involving treatment of solid tumors, antibodies are designed to have a cytotoxic or otherwise anticellular effect against the tumor vasculature, by suppressing the growth or cell division of the vascular endothelial cells. This attack is intended to lead to a tumor-localized vascular collapse, depriving the tumor cells, particularly those tumor cells distal of the vasculature, of oxygen and nutrients, ultimately leading to cell death and tumor necrosis.

In preferred embodiments, antibody based therapeutics are formulated as pharmaceutical compositions as described below. In preferred embodiments, administration of an antibody composition of the present invention results in a measurable decrease in cancer (*e.g*., decrease or elimination of tumor).

### D. RNAi Therapies

In other embodiments, RNAi is used to regulate expression of the stem cell cancer markers of the present invention (e.g. those shown in Tables 4-8). RNAi represents an evolutionary conserved cellular defense for controlling the expression of foreign genes in most eukaryotes, including humans. RNAi is triggered by double-stranded RNA (dsRNA) and causes sequence-specific mRNA degradation of single-stranded target RNAs homologous in response to dsRNA. The mediators of mRNA degradation are small interfering RNA duplexes (siRNAs), which are normally produced from long dsRNA by enzymatic cleavage in the cell. siRNAs are generally approximately twenty-one nucleotides in length (e.g. 21-23 nucleotides in length), and have a base-paired structure characterized by two nucleotide 3'-overhangs. Following the introduction of a small RNA, or RNAi, into the cell, it is believed the sequence is delivered to an enzyme complex called RISC (RNA-induced silencing complex). RISC recognizes the target and cleaves it with an endonuclease. It is noted that if larger RNA sequences are delivered to a cell, RNase III enzyme (Dicer) converts longer dsRNA into 21-23 nt ds siRNA fragments.

Chemically synthesized siRNAs have become powerful reagents for genome-wide analysis of mammalian gene function in cultured somatic cells. Beyond their value for validation of gene function, siRNAs also hold great potential as genespecific therapeutic agents (Tuschl and Borkhardt, Molecular Intervent. 2002; 2(3):158-67, herein incorporated by reference).

The transfection of siRNAs into animal cells results in the potent, long-lasting post-transcriptional silencing of specific genes (Caplen et al, Proc Natl Acad Sci U.S.A. 2001; 98: 9742-7; Elbashir et al., Nature. 2001; 411:494-8; Elbashir et al., Genes Dev. 2001;15: 188-200; and Elbashir et al., EMBO J. 2001; 20: 6877-88, all of which are herein incorporated by reference). Methods and compositions for performing RNAi with siRNAs are described, for example, in U.S. Patent 6,506,559, herein incorporated by reference.

siRNAs are extraordinarily effective at lowering the amounts of targeted RNA, and by extension proteins, frequently to undetectable levels. The silencing effect can last several months, and is extraordinarily specific, because one nucleotide mismatch between the target RNA and the central region of the siRNA is frequently sufficient to prevent silencing Brummelkamp et al, Science 2002; 296:550-3; and Holen et al, Nucleic Acids Res. 2002; 30:1757-66, both of which are herein incorporated by reference.

### E. Pharmaceutical Compositions

The present invention further provides pharmaceutical compositions (*e.g*., comprising a small molecule, antisent, antibody, or siRNA that targets the stem cell cancer markers of the present invention). The pharmaceutical compositions of the present invention may be administered in a number of ways depending upon whether local or systemic treatment is desired and upon the area to be treated. Administration may be topical (including ophthalmic and to mucous membranes including vaginal and rectal delivery), pulmonary (*e.g*., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, *e.g*., intrathecal or intraventricular, administration.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable.

Compositions and formulations for oral administration include powders or granules, suspensions or solutions in water or non-aqueous media, capsules, sachets or tablets. Thickeners, flavoring agents, diluents, emulsifiers, dispersing aids or binders may be desirable.

Compositions and formulations for parenteral, intrathecal or intraventricular administration may include sterile aqueous solutions that may also contain buffers, diluents and other suitable additives such as, but not limited to, penetration enhancers, carrier compounds and other pharmaceutically acceptable carriers or excipients.

Pharmaceutical compositions of the present invention include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by uniformly and intimately bringing into association the active ingredients with liquid carriers or finely divided solid carriers or both, and then, if necessary, shaping the product.

The compositions of the present invention may be formulated into any of many possible dosage forms such as, but not limited to, tablets, capsules, liquid syrups, soft gels, suppositories, and enemas. The compositions of the present invention may also be formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers.

In one embodiment of the present invention the pharmaceutical compositions may be formulated and used as foams. Pharmaceutical foams include formulations such as, but not limited to, emulsions, microemulsions, creams, jellies and liposomes. While basically similar in nature these formulations vary in the components and the consistency of the final product.

Agents that enhance uptake of oligonucleotides at the cellular level may also be added to the pharmaceutical and other compositions of the present invention. For example, cationic lipids, such as lipofectin (U.S. Pat. No. 5,705,188), cationic glycerol derivatives, and polycationic molecules, such as polylysine (WO 97/30731), also enhance the cellular uptake of oligonucleotides.

The compositions of the present invention may additionally contain other adjunct components conventionally found in pharmaceutical compositions. Thus, for example, the compositions may contain additional, compatible, pharmaceutically-active materials such as, for example, antipruritics, astringents, local anesthetics or anti-inflammatory agents, or may contain additional materials useful in physically formulating various dosage forms of the compositions of the present invention, such as dyes, flavoring agents, preservatives, antioxidants, opacifiers, thickening agents and stabilizers. However, such materials, when added, should not unduly interfere with the biological activities of the components of the compositions of the present invention. The formulations can be sterilized and, if desired, mixed with auxiliary agents, *e.g*., lubricants, preservatives, stabilizers, wetting agents, emulsifiers, salts for influencing osmotic pressure, buffers, colorings, flavorings and/or aromatic substances and the like which do not deleteriously interact with the nucleic acid(s) of the formulation.

Certain embodiments of the invention provide pharmaceutical compositions containing (a) one or more compounds that modulate the activity of a stem cell caner marker (e.g. antibody, small molecule, siRNA, anti-sense, etc.) and (b) one or more other chemotherapeutic agents. Examples of such chemotherapeutic agents include, but are not limited to, anticancer drugs such as daunorubicin, dactinomycin, doxorubicin, bleomycin, mitomycin, nitrogen mustard, chlorambucil, melphalan, cyclophosphamide, 6-mercaptopurine, 6-thioguanine, cytarabine (CA), 5-fluorouracil (5-FU), floxuridine (5-FUdR), methotrexate (MTX), colchicine, vincristine, vinblastine, etoposide, teniposide, cisplatin and diethylstilbestrol (DES). Anti-inflammatory drugs, including but not limited to nonsteroidal anti-inflammatory drugs and corticosteroids, and antiviral drugs, including but not limited to ribivirin, vidarabine, acyclovir and ganciclovir, may also be combined in compositions of the invention. Other chemotherapeutic agents are also within the scope of this invention. Two or more combined compounds may be used together or sequentially.

Dosing is dependent on severity and responsiveness of the disease state to be treated, with the course of treatment lasting from several days to several months, or until a cure is effected or a diminution of the disease state is achieved (e.g. reduction in tumor size). Optimal dosing schedules can be calculated from measurements of drug accumulation in the body of the patient. The administering physician can easily determine optimum dosages, dosing methodologies and repetition rates. Optimum dosages may vary depending on the relative potency of individual oligonucleotides, and can generally be estimated based on EC₅₀s found to be effective in *in vitro* and *in vivo* animal models or based on the examples described herein. In general, dosage is from 0.01 µg to 100 g per kg of body weight, and may be given once or more daily, weekly, monthly or yearly. The treating physician can estimate repetition rates for dosing based on measured residence times and concentrations of the drug in bodily fluids or tissues. Following successful treatment, it may be desirable to have the subject undergo maintenance therapy to prevent the recurrence of the disease state, wherein the oligonucleotide is administered in maintenance doses, ranging from 0.01 µg to 100 g per kg of body weight, once or more daily, to once every 20 years.

### VIII. Transgenic Animals Expressing Cancer Marker Genes

The present invention contemplates the generation of transgenic animals comprising an exogenous cancer marker gene of the present invention or mutants and variants thereof (*e.g*., truncations or single nucleotide polymorphisms) or knock-outs thereof. In preferred embodiments, the transgenic animal displays an altered phenotype (*e.g*., increased or decreased presence of markers) as compared to wild-type animals. Methods for analyzing the presence or absence of such phenotypes include but are not limited to, those disclosed herein. In some preferred embodiments, the transgenic animals further display an increased or decreased growth of tumors or evidence of cancer.

The transgenic animals of the present invention find use in drug (*e.g*., cancer therapy) screens. In some embodiments, test compounds (*e.g*., a drug that is suspected of being useful to treat cancer) and control compounds (*e.g*., a placebo) are administered to the transgenic animals and the control animals and the effects evaluated.

The transgenic animals can be generated via a variety of methods. In some embodiments, embryonal cells at various developmental stages are used to introduce transgenes for the production of transgenic animals. Different methods are used depending on the stage of development of the embryonal cell. The zygote is the best target for micro-injection. In the mouse, the male pronucleus reaches the size of approximately 20 micrometers in diameter that allows reproducible injection of 1-2 picoliters (pl) of DNA solution. The use of zygotes as a target for gene transfer has a major advantage in that in most cases the injected DNA will be incorporated into the host genome before the first cleavage (Brinster et al., Proc. Natl. Acad. Sci. USA 82:4438-4442 [1985]). As a consequence, all cells of the transgenic non-human animal will carry the incorporated transgene. This will in general also be reflected in the efficient transmission of the transgene to offspring of the founder since 50% of the germ cells will harbor the transgene. U.S. Patent No. 4,873,191 describes a method for the micro-injection of zygotes; the disclosure of this patent is incorporated herein in its entirety.

In other embodiments, retroviral infection is used to introduce transgenes into a non-human animal. In some embodiments, the retroviral vector is utilized to transfect oocytes by injecting the retroviral vector into the perivitelline space of the oocyte (U.S. Pat. No. 6,080,912, incorporated herein by reference). In other embodiments, the developing non-human embryo can be cultured *in vitro* to the blastocyst stage. During this time, the blastomeres can be targets for retroviral infection (Janenich, Proc. Natl. Acad. Sci. USA 73:1260 [1976]). Efficient infection of the blastomeres is obtained by enzymatic treatment to remove the zona pellucida (Hogan et al., in Manipulating the Mouse Embryo, Cold Spring Harbor Laboratory Press, Cold Spring Harbor, N.Y. [1986]). The viral vector system used to introduce the transgene is typically a replication-defective retrovirus carrying the transgene (Jahner et al., Proc. Natl. Acad Sci. USA 82:6927 [1985]). Transfection is easily and efficiently obtained by culturing the blastomeres on a monolayer of virus-producing cells (Stewart, et al., EMBO J., 6:383 [1987]).

Alternatively, infection can be performed at a later stage. Virus or virus-producing cells can be injected into the blastocoele (Jahner et al., Nature 298:623 [1982]). Most of the founders will be mosaic for the transgene since incorporation occurs only in a subset of cells that form the transgenic animal. Further, the founder may contain various retroviral insertions of the transgene at different positions in the genome that generally will segregate in the offspring. In addition, it is also possible to introduce transgenes into the germline, albeit with low efficiency, by intrauterine retroviral infection of the midgestation embryo (Jahner *et al., supra* [1982]). Additional means of using retroviruses or retroviral vectors to create transgenic animals known to the art involve the micro-injection of retroviral particles or mitomycin C-treated cells producing retrovirus into the perivitelline space of fertilized eggs or early embryos (PCT International Application WO 90/08832 [1990], and Haskell and Bowen, Mol. Reprod. Dev., 40:386 [1995]).

In other embodiments, the transgene is introduced into embryonic stem cells and the transfected stem cells are utilized to form an embryo. ES cells are obtained by culturing pre-implantation embryos *in vitro* under appropriate conditions (Evans et al., Nature 292:154 [1981]; Bradley et al., Nature 309:255 [1984]; Gossler et al., Proc. Acad. Sci. USA 83:9065 [1986]; and Robertson et al., Nature 322:445 [1986]). Transgenes can be efficiently introduced into the ES cells by DNA transfection by a variety of methods known to the art including calcium phosphate co-precipitation, protoplast or spheroplast fusion, lipofection and DEAE-dextran-mediated transfection. Transgenes may also be introduced into ES cells by retrovirus-mediated transduction or by micro-injection. Such transfected ES cells can thereafter colonize an embryo following their introduction into the blastocoel of a blastocyst-stage embryo and contribute to the germ line of the resulting chimeric animal (for review, *See*, Jaenisch, Science 240:1468 [1988]). Prior to the introduction of transfected ES cells into the blastocoel, the transfected ES cells may be subjected to various selection protocols to enrich for ES cells which have integrated the transgene assuming that the transgene provides a means for such selection. Alternatively, the polymerase chain reaction may be used to screen for ES cells that have integrated the transgene. This technique obviates the need for growth of the transfected ES cells under appropriate selective conditions prior to transfer into the blastocoel.

In still other embodiments, homologous recombination is utilized to knock-out gene function or create deletion mutants (*e.g*., truncation mutants). Methods for homologous recombination are described in U.S. Pat. No. 5,614,396, incorporated herein by reference.

### EXPERIMENTAL

The following examples are provided in order to demonstrate and further illustrate certain preferred embodiments and aspects of the present invention and are not to be construed as limiting the scope thereof.

In the experimental disclosure which follows, the following abbreviations apply: N (normal); M (molar); mM (millimolar); µM (micromolar); mol (moles); mmol (millimoles); µmol (micromoles); nmol (nanomoles); pmol (picomoles); g (grams); mg (milligrams); µg (micrograms); ng (nanograms); 1 or L (liters); ml (milliliters); µl (microliters); cm (centimeters); mm (millimeters); µm (micrometers); nm (nanometers); and °C (degrees Centigrade).

### Example 1

### Establishing and Analyzing a Solid Tumor Cell Xenograft Model

This examples describes the generation of tumors in mice using human solid tumor cells from humans and the analysis of these tumors.

### Materails and Methods

**Mouse preparation.** 8-week old female NOD-SCID mice were anesthetized by an intra-peritoneal injection of 0.2ml Ketamine/Xylazine (300mg Ketamine combined with 20mg Xylazine in a 4ml volume. 0.02ml of the solution was used per 20g mouse). Dilution to 200µl was done using HBSS. Mice were then treated with VP-16 (etoposide) via an intra-peritoneal injection (30mg etoposide dose per 1 kg mouse, diluted in serum-free HBSS for a final injection volume of 200µl). At the same time, estrogen pellets were placed subcutaneously on the back of the mouse's neck using a trocar. All tumor injections/implants were done 5 days after this procedure. In the following procedures, mice were anesthetized as described above.

**Primary tumor specimen implantations.** For the implantation of fresh specimens, samples of human breast tumors were received within an hour after surgery. The tumors were cut up with scissors into small pieces, and the pieces were then minced with a blade to yield 2x2 mm-size pieces. Mincing was done in sterile RPMI 1640 medium supplemented with 20% Fetal Bovine Serum (FBS) under sterile conditions on ice. The tumor pieces were washed with serum-free HBSS before implantation. A 2-mm incision was then made in the mid abdomen area, and using a trocar, one to two small tumor pieces were implanted in the region of the upper right and upper left mammary fat pats (right below the second nipple on both sides). A 6-0 suture was wrapped twice around the MFP-Nipple allowing it to hold the implanted pieces in place. Sutures were removed after 5 days. Nexaban was used to seal the incision and mice were monitored weekly for tumor growth.

**Pleural effusions injections.** For the injection of the pleural effusions, cells were received shortly after thorocentesis and washed with serum-free HBSS. Cells were then suspended in serum free-RPMI/Matrigel mixture (1:1 volume) and then injected into the upper right and left mammary pads using an 18G needed. 0.2ml containing 1-2 million cells were typically injected. The site of the needle injection was sealed with Nexaban to prevent any cell leakage.

**Preparation of single cell suspensions of tumor cells.** Prior to digestion with collagenase, Xenograft tumors or primary human tumors were cut up into small pieces and then minced completely using sterile blades. To obtain single cell suspensions, either pleural effusion cells or the resulting tumor pieces were then mixed with ultra-pure Collagenase III in HBSS solution (200-250U Collagenase per ml) and allowed to incubate at 37°C for 3-4 hours. Pipetting with a 10ml pipette was done every 15-20 minutes. At the end of the incubation, cells were filtered through a 45µl nylon mesh and washed with RPMI-20% FBS, then washed twice with HBSS. Cells to be injected were then suspended in HBSS/Matrigel mix (1:1 volume) and injected into the area of the mammary fat pad as described above. Nexaban was used to seal the injection site.

**Cell staining for flow-cytometry.** Cells were counted and then transferred to a 5 ml tube, washed twice with HBSS with 2% Heat-inactivated calf serum (HICS) (5 min @ 1000 rpm), then re-suspended in 100µl (per 10⁶ cells) of HBSS with 2% HICS. 5ml of Sandoglobin solution (1mg/ml) was then added and incubated on ice for 10 minutes, after which the sample was washed twice with HBSS 2% HICS and re-suspended in 100ml (per 10⁶ cells) of HBSS 2% HICS. Antibodies (using appropriate dilution per antibody) were then added and incubated for 20 minutes on ice, and then washed twice with HBSS 2% HICS. When needed, a secondary antibody addition was conducted by re-suspending in 100ul (per 10⁶ cells) of HBSS 2% HICS, and then adding 1-4 ml of secondary antibody (depending on the secondary antibody and its concentration), followed by a 20 minute incubation. When a streptavidin step was used, cells were re-suspended in 100ul (per 10⁶ cells) of HBSS 2% HICS and then 1ul of strepavidin conjugated with the indicated fluorescent dye was added, followed by a 20 minute incubation. The cells were washed twice with HBSS 2% heat-inactivated fetal calf serum (HICS) and re-suspended in 0.5ml (per million cells) of HBSS 2% HICS that contained 7AAD (1 mg/ml final concentration).

**Flow-cytometry.** The antibodies used were anti-CD44 (APC, PE or Biotin), anti-CD24 (PE or FITC), anti-B38.1 (APC), anti-ESA-FITC (Biomeda, CA), anti-H2K^{d}, (Santa Cruz Products, Santa Cruz, CA). Lineage marker antibodies were anti-CD2, -CD3 - CD10, -CD 16, -CD18, -CD31, -CD64 and -CD140b. Unless noted, antibodies were purchased from Pharmingen (San Diego, CA). Antibodies were directly conjugated to various fluorochromes depending on the tests. In all tests, mouse cells and/or Lineage⁺ cells were eliminated by discarding H2K^{d+} (class I MHC) cells or Lineage⁺ cells during flow-cytometry. Dead cells were eliminated using the viability dye 7-AAD. Flow-cytometry was performed on a FACSVantage (Becton Dickinson, San Jose, CA). Side scatter and forward scatter profiles were used to eliminate cell doublets. Cells were routinely sorted twice and the cells were re-analyzed for purity, which typically was greater than 95%.

In solid tumors, it has been demonstrated that only a small proportion of the tumor cells are able to form colonies in an *in vitro* clonogenic assay^{21-24,101-103}. Furthermore, large numbers of cells must typically be transplanted to form tumors in xenograft models. One possible explanation for these observations is that every cell within a tumor has the ability to proliferate and form new tumors but that the probability of an individual cell completing the necessary steps in these assays is small. An alternative explanation is that only a rare, phenotypically distinct subset of cells has the capacity to significantly proliferate and form new tumors, but that cells within this subset do so very efficiently ²⁵. To distinguish between these possibilities it is necessary to identify the clonogenic cells in these tumors with markers that distinguish these cells from other non-tumorigenic cells. This has been accomplished in acute myelogenous leukemia (AML), where it was demonstrated that a specific subpopulation of leukemia cells (that expressed markers similar to normal hematopoietic stem cells) was consistently enriched for clonogenic activity in NOD/SCID immunocompromised mice while other cancer cells were depleted of clonogenic activity¹⁰⁴⁻¹⁰⁶. Such tests have not been reported in solid cancers.

To investigate the mechanisms of solid tumor heterogeneity, a mouse model was developed that was a modification of the NOD/SCID immunodeficient mouse model in which human breast cancers were efficiently propagated in the mouse mammary fat pad⁹⁹. In the present application, it was shown that solid tumors contain a distinct population of cells with the exclusive ability to form tumors in mice. These cells are referred to as tumorigenic cells or cancer initiating cells since they consistently formed tumors while other cancer cell populations were depleted of cells capable of tumor formation. Cell surface markers were identified which can distinguish between these cell populations. These findings provide a new model of breast tumor biology in which a defined subset of cells drives tumorigenesis, as well as generating tumor cell heterogeneity. The prospective identification of this tumorigenic population of cancer cells allows for the identification of molecules expressed in these cells that can then serve as targets to eliminate this critical population of cancer cells.

**Tumor specimens and engraftment rate.** Human breast cancer specimens obtained from primary or metastatic sites in 9 different patients (designated tumors 1-9; T1-T9) all engrafted in the NOD/SCID mice. (Table 1). In one case, the cancer cells were obtained from a primary breast tumor (T2) while in other cases the cells were obtained from metastatic pleural effusions (T1, T3-T9). Some tests were conducted on cells after they had been passaged once or twice in mice (designated Passage 1 & 2) while other tests were conducted on unpassaged fresh or frozen tumor samples obtained directly from patients. When using human cancer cells from tumors passaged in mice, contaminating mouse cells were removed by eliminating H2K⁺ cells [mouse histocompatability class I (MHC)].

**Table 1**

| Tumor | Origin | Formation In mice | Passage In mice | Diagnosis |
|---|---|---|---|---|
| 1 | Metastasis | Yes | Yes | Infiltrating ductal carcinoma |
| T2 | Breast Primary | Yes | Yes | Adenocarcinoma |
| T3 | Metastasis | Yes | Yes | Invasive lobular carcinoma |
| T4 | Metastasis | Yes | No | Invasive lobular carcinoma |
| T5 | Metastasis | Yes | Yes | Invasive lobular carcinoma |
| T6 | Metastasis | Yes | Yes | Inflammatory breast carcinoma |
| T7 | Metastasis | Yes | Yes | Invasive lobular carcinoma |
| T8 | Metastasis | Yes | Yes | Inflammatory breast carcinoma |
| T9 | Metastasis | Yes | Yes | Adenocarcinoma |

Table 1 presented the results of engraftment of human breast cancers into NOD/SCID mice. Mice were injected with unsorted T1 and T3 cells, and a 2 mm piece of T2. Cells from T4-T9 were isolated by flow cytometry as described in Figure 1. All 9 tumors tested engrafted in the NOD/SCID mouse model. Except for T2 which was a primary breast tumor, all other tumors were metastases. All of the tumors were passaged serially in mice except for T4.

**Identification of tumorigenicity markers.** Breast cancer cells were heterogeneous with respect to expression of a variety of cell surface-markers including CD44, CD24, and B38.1. CD24 and CD44 are adhesion molecules, while B38.1 has been described as a breast/ovarian cancer-specific marker ^{9,107,108}. To determine whether these markers could distinguish tumorigenic from non-tumorigenic cells, flow-cytometry was used to isolate cells that were positive or negative for each marker from first passage T1 or T2 cells. When 2x10⁵-8x10⁵ cells of each population were injected, all injections of CD44⁺ cells (8/8), B38.1⁺ cells (8/8), or CD24^{-/low} cells (12/12) gave rise to visible tumors within 12 weeks of injection, but none of the CD44⁻ cell (0/8), or B38.1⁻ cell (0/8) injections formed detectable tumors (Table 2). Although no tumors could be detected by palpation in the locations injected with CD24⁺ cells, 2 of 12 mice injected with CD24⁺ cells did contain small growths at the injection site that were detected upon necropsy. These growths most likely arose from the 1-3% of CD24⁻ cells that invariably contaminate the sorted CD24⁺ cells, or alternatively from CD24⁺ cells with reduced proliferative capacity (Table 2). Because the CD44⁺ cells were exclusively B38.1⁺, we focused on the CD44 and CD24 markers in subsequent tests.

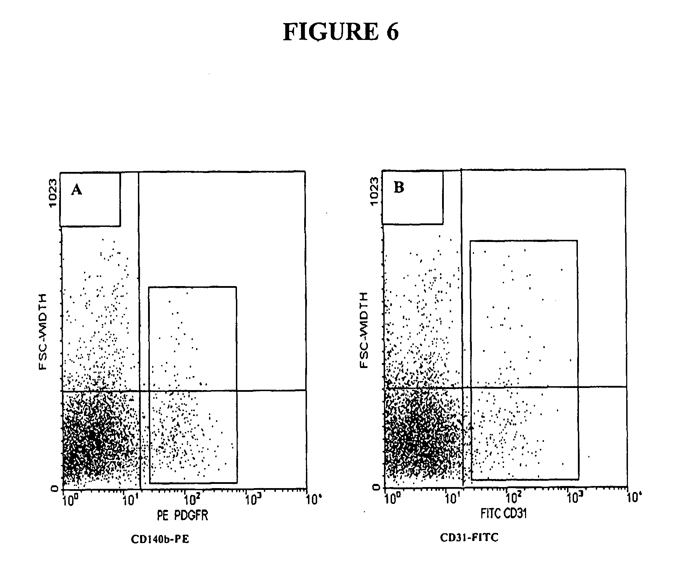

Several antigens associated with normal cell types (Lineage markers; CD2, CD3, CD10, CD16, CD18, CD31, CD64, and CD140b) were found not to be expressed by the cancer cells based on analyses of tumors that had been passaged multiple times in mice. By eliminating Lineage⁺ cells from unpassaged or early passage tumor cells, normal human leukocytes, endothelial cells, mesothelial cells and fibroblasts were eliminated. By microscopic examination, the Lineage tumor cells had the appearance of neoplastic cells (Figure 6).

**Table 2**

| ***Tumors*/*Injections*** | | | |
|---|---|---|---|
| Cells/Injection | 8x10⁵ | 5x10⁵ | 2x10⁵ |
| ***Passsaged T1*** | | | |
| CD44- | 0/2 | 0/2 | - |
| CD44+ | 2/2 | 2/2 | - |
| B38.1- | 0/2 | 0/2 | - |
| B38.1+ | 2/2 | 2/2 | - |
| CD24+ | - | - | 1/6 |
| CD24- | - | - | 6/6 |

| ***Passaged T2*** | | | |
|---|---|---|---|
| CD44- | 0/2 | 0/2 | - |
| CD44+ | 2/2 | 2/2 | - |
| B38.1- | 0/2 | 0/2 | - |
| B38.1+ | 2/2 | 2/2 | - |
| CD24+ | - | - | 1/6 |
| CD24- | - | - | 6/6 |

Table 2 shows the results of cells isolated by flow cytometry as described in figure 1 based upon expression of the indicated marker and assayed for the ability to form tumors after injection into the mammary fat pads of NOD/SCID mice. For 12 weeks, mice were examined weekly for tumors by observation and palpation, then all mice were necropsied to look for growths at injection sites that were too small to palpate. The number of tumors that formed/ the number of injections that were performed is indicated for each population. All tumors were readily apparent by visual inspection and palpation except for tumors from the CD24+ population that were only detected upon necropsy.

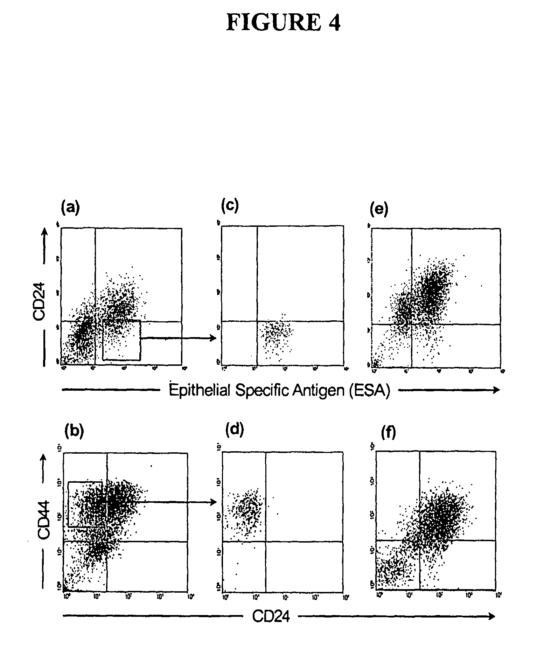

Depending on the tumor, 11% to 35% of the Lineage⁻ cancer cells in tumors or pleural effusions were CD44⁺CD24^{-/low} (Figure 4a-1f). CD44⁺CD24^{-/low} Lineage⁻ cells or other populations of Lineage cancer cells that had been isolated from nine patients were injected into the mammary fat pads of mice (Table 3). When injecting unsorted, passaged T1 or T2 cells, 5x10⁴ cells consistently gave rise to tumors, but 10⁴ cells gave rise to tumors in only a minority of cases. In contrast, as few as 10³ T1 or T2 CD44⁺CD24^{-/low} Lineage cells gave rise to tumors in all cases (Table 3). In T1 and T2, up to 2x10⁴ cells that were CD44⁺Lineage but CD24⁺ failed to form tumors. These data suggest that the CD44⁺CD24^{-/low} Lineage⁻ population is 10-50 fold enriched for the ability to form tumors in NOD/SCID mice relative to unfractionated tumor cells. Whether the CD44⁺CD24^{-/low} Lineage⁻ cells were isolated from passaged tumors (T1, T2, T3) or from unpassaged cancer cells obtained directly from patients (T1, T4-T6, T8, T9), they were enriched for tumorigenic activity. Note that T7 was the only one of 9 cancers studied that did not fit this pattern (Figure 4f). Other than T7, CD24⁺Lineage cancer cells in both unpassaged and passaged tumors were unable to form new tumors (Table 3). Therefore, the xenograft and unpassaged patient tumors were composed of similar populations of phenotypically diverse cancer cell types, and in both cases only the CD44⁺CD24^{-/low} Lineage cells had the capacity to proliferate to form new tumors (p<0.001).

**Table 3**

| | | | | | | | | | | |
|---|---|---|---|---|---|---|---|---|---|---|
| **# of cells per injection** | 5x10⁵ | 10⁵ | 5 x10⁴ | 2 x10⁴ | 10⁴ | 5 x10³ | 10³ 500 200 | | | 100 |

| *Mouse passage 1* | | | | | | | | | | |
|---|---|---|---|---|---|---|---|---|---|---|
| Unsorted | 8/8 | 8/8 | 10/10 | | 3/12 | | 0/12 | | | |
| CD44⁺CD24⁺ | | | | 0/10 | 0/10 | 0/14 | 0/10 | | | |
| CD44⁺CD24^{-/low} | | | | 10/10 | 10/10 | 14/14 | 10/1 0 | | | |
| CD44⁺CD24⁻ | | | | | | | 10/1 0* | 4/4 | 4/4 | 1/6 |
| CD44⁺CD24^{- /low}ESA⁻ | | | | | | | 0/10* | 0/4 | 0/4 | 0/6 |

| *Mouse passage 2* | | | | | | | | | | |
|---|---|---|---|---|---|---|---|---|---|---|
| CD44⁺CD24⁺ | | | | | 0/9 | | | | | |
| CD44⁺CD24^{-/low} | | | | | 9/9 | | | | | |

| *Patients' tumor cells* | | | | | | | | | | |
|---|---|---|---|---|---|---|---|---|---|---|
| CD44⁺CD24⁺ | | 0/3 | 0/4 | 0/8 | 1/13 | 0/2 | | | | |
| CD44⁺CD24^{-/low} | | 3/3 | 4/4 | | 11/13 | 1/1 | | | | |
| CD44⁺CD24^{- /low}ESA⁺ | | | | | | 2/2 | 2/2 | | | |
| CD44⁺CD24^{- /low}ESA⁻ | | | | | | 2/2^{#} | 0/2 | | | |

As shown in Table 3, tumorigenic breast cancer cells were highly enriched in the ESA+CD44+CD24-/low population. Cells were isolated from first passage (designated Mouse Passage 1) Tumor 1, Tumour 2 and Tumor 3, second passage Tumor 3 (designated mouse Passage 2), unpassaged cells obtained from 6 different patients, T1, T4, T5, T6 , T8 and T9, (designated Patients' tumor cells). CD44+CD24+Lineage- populations and CD44+CD24-/lowLineage- cells were isolated by flow-cytometry as described in figure 1. The indicated number of cells of each phenotype was injected into the breast of NOD/SCID mice. The frequency of tumorigenic cells calculated by the modified maximum likelihood analysis method is ~5/10⁵ if single tumorigenic cells were capable of forming tumors, and every transplanted tumorigenic cell gave rise to a tumor¹⁰⁹. Therefore, this calculation may underestimate the frequency of the tumorigenic cells since it does not take into account cell-cell interactions and local environment factors that may influence engraftment. In addition to the markers that are shown, all sorted cells in all tests were Lineage-, and the tumorigenic cells from T1, T2, and T3 were further selected as B38.1+, The mice were observed weekly for 4-6½ months, or until the mice became sick from the tumors. #Tumor formation by T5 ESA-CD44+CD24-/lowLINEAGE-cells was delayed by 2-4 weeks. *2,000 cells were injected in these tests.

Figure 1 shows isolation of tumorigenic cells. Flow cytometry was used to isolate subpopulations of Tumor 1 (a, b), Tumor 3 (c), Tumor 5 (d), Tumor 6 (e) and Tumor 7 cells (f) that were tested for tumorigenicity in NOD/SCID mice. T1 (b) and T3 (c) had been passaged (P) once in NOD/SCID mice while the rest of the cells were frozen or unfrozen samples obtained directly after removal from a patient (UP). Cells were stained with antibodies against CD44, CD24, Lineage markers, and mouse-H2K (for passaged tumors obtained from mice), and 7AAD. Dead cells (7AAD+), mouse cells (H2K+) and Lineage+ normal cells were eliminated from all analyses. Each plot in Figure 1 depicts the CD24 and CD44 staining patterns of live human Lineage-cancer cells, and the frequency of the boxed tumorigenic cancer population as a percentage of cancer cells/all cells in each specimen is shown.

In three of the tumors, further enrichment of tumorigenic activity was possible by isolating the ESA⁺ subset of the CD44⁺CD24^{-/low} population. ESA (Epithelial Specific Antigen, Ep-CAM) has been used in the past to distinguish epithelial cancer cells from benign reactive mesothelial cells¹¹⁰. When ESA⁺CD44⁺CD24^{-/low}Lineage⁻ cells were isolated from passaged T1, as few as 200 cells consistently formed tumors of approximately 1 cm between 5-6 months after injection whereas 2000 ESA⁻ CD44⁺CD24^{-/low}Lineage⁻ cells or 20,000 CD44⁺CD24⁺ cells always failed to form tumors (Table 3). Ten thousand unsorted cells formed tumors in only 3 of 12 mice. This suggests that the ESA⁺CD44⁺CD24^{-/low}Lineage⁻ population was more than 50 fold enriched for the ability to form tumors relative to unfractionated tumor cells (Table 3). The ESA⁺CD44⁺CD24^{-/low}Lineage⁻ population accounted for 2-4% of first passage T1 cells (2.5-5% of cancer cells). The ESA⁺CD44⁺CD24^{-/low}Lineage⁻ population (0.6% of cancer cells) from unpassaged T5 cells was also enriched for tumorigenic activity compared to ESA⁻CD44⁺CD24^{-/low}Lineage⁻ cells, but both the ESA⁺ and ESA⁻ fractions had some tumorigenic activity (Table 3). Among unpassaged T5 cells, as few as 1000 ESA⁺CD44⁺CD24^{-/low}Lineage⁻ cells consistently formed tumors.

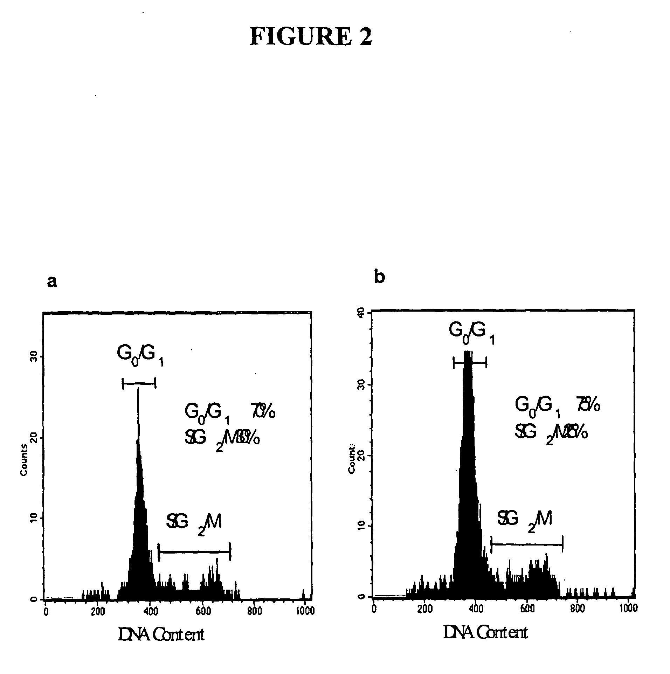

In order to determine whether the difference in tumorigenicity of the cell populations was due to differences in cell cycle, populations were analyzed by flow-cytometry. Comparison of the cell cycle status of tumorigenic and non-tumorigenic cancer cells from T1 revealed that both exhibited a similar cell cycle distribution (Fig. 2a, 2b). Therefore, neither population was enriched for cells at a particular stage of the cell-cycle, and the non-tumorigenic cells were able to undergo at least a limited number of divisions in the xenograft model.

Figure 2 shows the DNA content of tumorigenic and non-tumorigenic breast cancer cells. The cell cycle status of the ESA+CD44+CD24-/lowLineage-tumorigenic cells (a) and the remaining Lineage- non-tumorigenic cancer cells (b) isolated from T1 were determined by hoechst 33342 staining of DNA content (20). The tumorigenic and non-tumorigenic cell populations exhibited similar cell cycle distributions

Six months after inoculation, the injection sites of 20,000 tumorigenic CD44⁺CD24^{-/low}Lineage⁻ cells and 20,000 CD44⁺CD24⁺Lineage⁻ cells were examined by histology. The CD44⁺CD24^{-/low}Lineage⁻ injection sites contained tumors approximately 1 cm in diameter while the CD44⁺CD24⁺Lineage⁻ injection sites contained no detectable tumors (Figure 6c). Only normal mouse mammary tissue was seen by histology at the sites of the CD44⁺CD24⁺Lineage⁻ injections (Figure 3a), whereas the tumors formed the CD44⁺CD24^{-/low}Lineage⁻ cells contained malignant cells as judged by hematoxylin and eosin stained sections (Figure 3b). Even when CD44⁺CD24⁺Lineage⁻ injection sites from 58 mice, each administered 1,000-50,000 cells, were examined after 16-29 weeks, no tumors were detected. Furthermore, the tumorigenic and non-tumorigenic populations were indistinguishable morphologically. Both the tumorigenic and non-tumorigenic subsets of Lineage- cells from passaged and unpassaged tumors contained >95% cancer cells as judged by Wright staining or Papanicolaou staining and microscopic analysis. By histology, the CD44⁺CD24^{-/low}Lineage⁻ cells and the rest of the Lineage- cells had the appearances of epithelial cancer cells (Figure 3d, 3e).

Figure 3 shows histology from the CD24⁺ injection site (a), (20x objective magnification) revealed only normal mouse tissue while the CD24^{-/low} injection site (b), (40x objective magnification) contained malignant cells. (c) A representative tumor in a mouse at the CD44⁺CD24^{-/low}Lineage⁻ injection site, but not at the CD44⁺CD24⁺Lineage⁻ injection site. T3 cells were stained with Papanicolaou stain and examined microscopically (100x objective). Both the non-tumorigenic (c) and tumorigenic (d) populations contained cells with a neoplastic appearance, with large nuclei and prominent nucleoli.

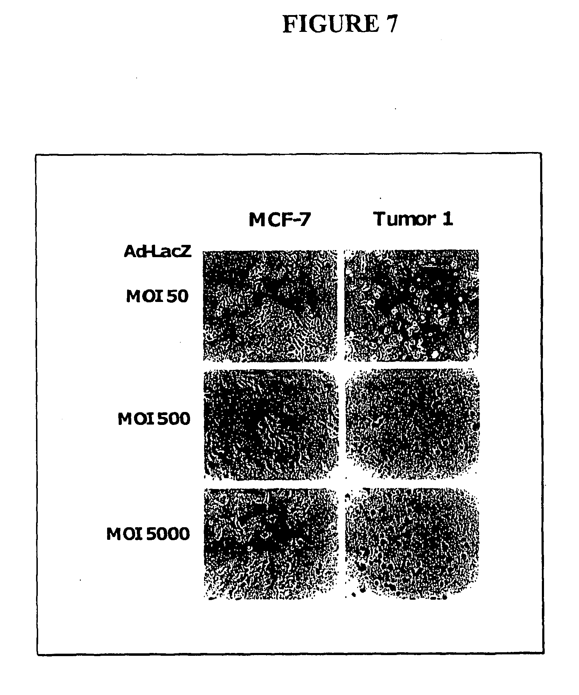

The tumorigenic population is capable of generating the phenotypic heterogeneity found in the initial tumor. The ability of small numbers of CD44⁺CD24^{-/low}Lineage⁻ tumorigenic cells to give rise to new tumors was reminiscent of the organogenic capacity of normal stem cells. Normal stem cells self-renew and give rise to phenotypically diverse cells with reduced proliferative potential. To test whether tumorigenic breast cancer cells also exhibit these properties; tumors arising from 200 ESA⁺CD44⁺CD24^{-/low}Lineage⁻ T1 or 1,000 CD44⁺CD24^{-/low}Lineage⁻ T2 cells were dissociated and analyzed by flow-cytometry. The heterogeneous expression patterns of ESA, CD44 or CD24 in the secondary tumors resembled the phenotypic complexity of the tumors from which they were derived (Figure 7a,7b vs 7e,7f). Within these secondary tumors, the CD44⁺CD24^{-/low}Lineage⁻ cells remained tumorigenic, while other populations of Lineage cancer cells remained non-tumorigenic (Table 3). Thus tumorigenic cells gave rise to both additional CD44⁺CD24^{-/low}Lineage⁻ tumorigenic cells as well as to phenotypically diverse non-tumorigenic cells that recapitulated the complexity of the primary tumors from which the tumorigenic cells had been derived. These CD44⁺CD24^{-/low}Lineage⁻ tumorigenic cells from T1, T2 and T3 have now been serially passaged through four rounds of tumor formation in mice, yielding similar results in each passage with no evidence of decreased tumorigeneity. These observations suggest that CD44⁺CD24^{-/low}Lineage⁻ tumorigenic cancer cells undergo processes analogous to the self-renewal and differentiation of normal stem cells.

Figure 4 shows the phenotypic diversity in tumors arising from CD44+CD24-/lowLineage- cells. The plots depict the CD24 and CD44 or ESA staining patterns of live human Lineage- cancer cells from Tumor 1 (a, c and e) or Tumor 2 (b, d and f). T1 CD44+Lineage- cells (a) or T2 Lineage- cells (b) were obtained from tumors that had been passaged once in NOD/SCID mice. ESA+CD44+CD24-/lowLineage-tumorigenic cells from T1 (c) or CD44+CD24-/lowLineage- tumorigenic cells from T2 (d) were isolated and injected into the breasts of NOD/SCID mice. Panels (e) and (f) depict analyses of the tumors that arose from these cells. In both cases, the tumorigenic cells formed tumors that contained phenotypically diverse cells similar to those observed in the original tumor.

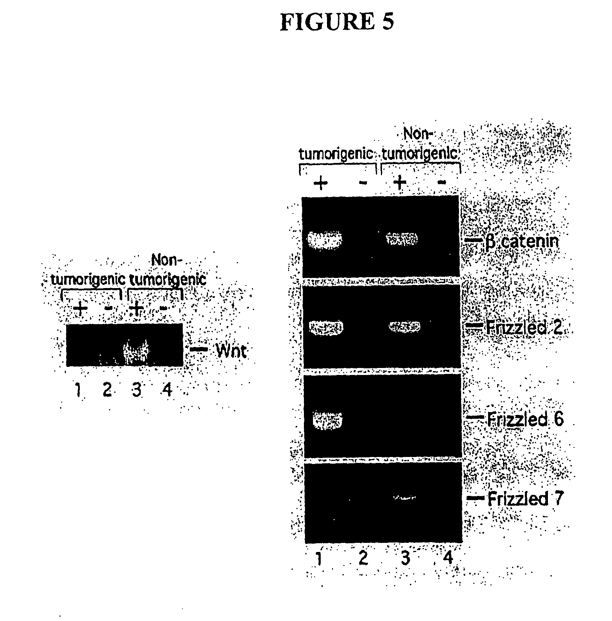

**Expression of Wnt pathway genes in subpopulations of breast cancer tumor cells.** The Frizzled proteins are receptors for the growth/survival factors of the Wnt family. In some normal stem cells, Wnt is known to play a role in proliferation, survival and differentiation. In certain situations, stimulation of Wnt can promote stem cell self-renewal. Upon activation, Wnt induces the stabilization of β-catenin. Flow cytometry using an antibody against β-catenin demonstrates that Tumor 1 cells express this protein (Figure 5). Immunohistochemistry shows that the β-catenin is located in the cytoplasm and the nucleus, indicating that the protein is active (data not shown). Different Wnt proteins specifically activate different frizzled receptors (44). Since the Wnt signaling pathway appears to play a critical role in proliferation of both normal and breast cancer cell proliferation (14,27), the expression of Wnt pathway genes in Tumor 1 tumorigenic cells and non-tumorigenic cells was examined (Figure 5). To do this, one hundred ESA⁺B38.1⁺CD24^{-/lo}LINEAGE⁻ (tumorigenic) or non-tumorigenic tumor cells were isolated. RT-PCR using nested primers for each of the frizzled proteins was done. These results demonstrate that the tumorigenic cells expressed frizzled 2 and 6, while the non-tumorigenic cells expressed frizzled 2 and 7 (Figure 5). These tests have been repeated twice with identical results. Next, members of the Wnt family expressed by the breast cancer cells were identified. RNA was isolated from 10,000 stem and non-tumorigenic cells. There are more than 20 known members of the Wnt family, making it difficult to analyze expression of particular Wnts in breast cancer tumors. Therefore RT-PCR was performed using degenerate primers that recognize all known Wnt genes and cloned and sequenced the resultant cDNA. Surprisingly, we were able to detect expression of cDNA only by the non-tumorigenic cells (Figure 5). This was confirmed doing RT-PCR at the tencell level. Frizzled 6 expression was detected in nine often tumorigenic samples, and only one of ten non-tumorigenic cell samples. The cDNA was cloned, and sequencing revealed that these cells expressed Wnt 3A, 4, 7A, 7B, 10B, and 11. Wnt signals have been implicated in the growth of both breast cancer cells and normal endothelial cells. While not necessary to understand to practice the present invention, this suggests that the non-tumorigenic cells promote tumor formation both by stimulation of breast cancer stem cells and vessel formation via the Wnt pathway. This model fits very well with known observations that it is much easier to grow breast cancers using pieces of tissue as opposed to individual cells (22).

Figure 5 shows the expression of Wnt (left panel) and Frizzled (right panel). In regard to the left panel, RT-PCR was done using degenerate Wnt primers with RNA isolated from 10,000 cells of the indicated type. + or - indicates whether RT was used. Right panel. RNA was isolated from one hundred breast cancer cells or breast cancer stem cells isolated by flow cytometry as described in figure 1. RT-PCR was done using nested primers to detect the indicated mRNA. Control RT-PCR reactions omitting RT were negative.

To confirm the RT-PCR results for the expression of frizzled proteins, an Affymetrix microarray was probed with cDNA made from Tumor 1, Tumor 2 and Tumor 3 cancer stem cells. All three tumors expressed Frizzled 2 & 6. In addition, Tumors 2 & 3 appeared to express frizzled 4.

**Isolation of normal cells from a tumor.** Efforts were then made to determine whether sufficient normal cells could be isolated from a tumor to do molecular studies with these cells. Normal fibroblast and endothelial cells from a patient's tumor (approximately 3cm in size) were isolated by flow cytometry. 2% of the tumor cells were CD31⁺ endothelial cells and 8% were CD140b⁺ fibroblasts (Figure 6). Nine thousand fibroblasts and two thousand endothelial cells were collected when 1/45 of the tumor was used for flow cytometry. By extrapolation, it would have been possible to isolate approximately 90,000 endothelial cells and 405,000 fibroblasts from the entire tumor.

Figure 6 shows the isolation of normal tumor fibroblasts and endothelial cells. Tumors were dissociated as described in the methods section and tumor cells were stained with cytochrome labeled with antibodies against -CD2, -CD3, -CD16, -CD18 ,-CD45, -CD64, and anti-B38.1-APC (to eliminate hematopoietic cells and tumor cells respectively), anti-CD140b-PE and anti-CD31-FITC. A: the box shows the sorting gate for fibroblasts, which are Lineage CD31⁻ CD140b⁺ cells. B: the box shows the sorting gate for endothelial cells, which are CD31⁺ Lineage⁻ cells.

**Infection of breast cancer stem cells with an adenovirus vector.** Since the xenograft tumors can only be grown briefly in tissue culture, conventional transfection methods are generally not useful for gene expression studies and only viral vectors have the potential to efficiently transduce the breast cancer stem cells. Therefore, the ability of adenovirus vectors to infect T1 breast cancer stem cells was tested. To do this, groups of 10,000 breast cancer stem cells or control MCF-7 cells were infected with 0, 50, 500, or 5,000 LacZ adenovirus particles. Figure 7 shows that we could easily transduce greater than 90% of the stem cells and they were more easily infected with the adenovirus vector than were the control MCF-7 cells. This demonstrates that we can use adenovirus vectors to transduce the stem cells with recombinant genes.

Figures 7 shows infection of breast cancer stem cells with an adenovirus vector. Flow cytometry was used to isolate CD44⁺CD24^{-/low}Lineage⁻ cells. The Tumor 1 stem cells or control MCF-7 cells were infected with 0, or 500, or 5,000 LacZ adenovirus particle/cell. Two days later, the cells were stained with X-gal. Note that the Tumor 1 stem cells were easily infected by the adenovirus vector.

The following data is a description of work that has been done studying hematopoietic stem cells. It illustrates fundamental stem cell properties, and it also demonstrates how the isolation of stem cells enables one to first characterize these cells and then to do molecular and biochemical studies to functionally characterize them.

**Adult stem cell numbers are strictly regulated.** The regulation of hematopoietic stem cell (HSC) homeostasis is not well understood. We screened for genetic polymorphisms that were linked to differences between mouse strains in the numbers of long-term reconstituting HSCs or restricted progenitors in the bone marrow. AKR/J mice had significantly higher frequencies and numbers of both HSCs and restricted progenitors in their bone marrow than C57BL/Ka-Thy-1.1 mice. The C57BL/Ka-Thy-1.1 alleles were partially dominant. A locus on chromosome 17, including the H-2 complex, was significantly linked to the frequency of long-term self-renewing HSCs but showed no evidence of linkage to the frequency of restricted progenitors. Conversely, a chromosome 1 locus exhibited suggestive linkage to restricted progenitor frequencies but was not linked to HSC frequency. This demonstrates that there are distinct genetic determinants of the frequencies of HSCs and restricted progenitors in vivo. The AKR/J chromosome 17 locus was not sufficient to increase HSC frequencies when bred onto a C57BL background. This suggests that to affect HSC frequencies, the product(s) of this locus likely depend on interactions with unlinked modifying loci. The present invention demonstrates that stem cell expansion is under tight genetic regulation in an animal.

**Genomic analysis of hematopoietic stem cells.** Hematopoietic stem cells (HSCs) have self-renewal capacity and multilineage developmental potentials. The molecular mechanisms that control the self-renewal of HSCs are still largely unknown. A systematic approach using bioinformatics and array hybridization techniques to analyze gene expression profiles in HSCs was done. To enrich mRNAs predominantly expressed in uncommitted cell lineages, 54 000 cDNA clones generated from a highly enriched population of HSCs and a mixed population of stem and early multipotent progenitor (MPP) cells were arrayed on nylon membranes (macroarray or high-density array), and subtracted with cDNA probes derived from mature lineage cells including spleen, thymus, and bone marrow. Five thousand cDNA clones with very low hybridization signals were selected for sequencing and further analysis using microarrays on glass slides. Two populations of cells, HSCs and MPP cells, were compared for differential gene expression using microarray analysis. HSCs have the ability to self-renew, while MPP cells have lost the capacity for self-renewal. A large number of genes that were differentially expressed by enriched populations of HSCs and MPP cells were identified. These included transcription factors, signaling molecules, and previously unknown genes.

**Bmi-1 is required for HSC self-renewal.** The gene expression analysis of HSCs allowed us to identify genes potentially important for self-renewal. After analysis of the gene expression data, we began mechanistic studies to identify important stem cell regulatory genes. A central issue in stem cell biology is to understand the mechanisms that regulate self-renewal of HSCs, which is required for hematopoiesis to persist for the lifetime of the animal. We found that adult and E14.5 fetal mouse and adult human hematopoietic stem cells express the proto-oncogene *bmi-1.* The number of fetal liver HSCs, as measured by flow cytometry, was normal in loss of function *bmi-1* mice, and the *bmi-1*^{-/-}HSCs were able to migrate normally towards a chemokine gradient. In post-natal *bmi-1*^{*-*/*-*} mice, the number of HSCs, but not early progenitor cells was markedly reduced. Both fetal liver and bone marrow cells obtained from *bmi-1*^{*-*/*-*} mice were able to contribute only transiently to hematopoiesis when transplanted into lethally irradiated recipients. There was no detectable self-renewal of adult hematopoietic stem cells, indicating a cell autonomous defect in *bmi-1*^{*-*/*-*} mice. This study indicates that expression of *bmi-1* is essential for the generation of self-renewing adult hematopoietic stem cells. See the manuscript by Park et al., "Bmi-1 is required for maintenance of adult self-renewing hematopoietic stem cells" Nature (2003).

**Summary:** The xenograft model developed by this laboratory has made possible the analysis of human breast cancer cells at the cellular level. Although cancer cell lines have proven useful for many studies, the cell lines are adapted to the unique conditions imposed by tissue culture and many of their properties clearly differ from the cancer cells in patients' tumors^{91,109}. Recently, the size of primary breast cancer tumors prior to resection has markedly decreased. This has made biological and biochemical studies using patient samples difficult. It is contemplated that the xenograft model described in the preliminary results ameliorates this problem. Preliminary results suggest that the xenograft tumors appear to recapitulate the phenotypic and biological diversity seen in the original patients' tumors. Although there may be some differences in the mouse and human tumors due to environmental factors, the NOD/SCID model described here is the best available model of human breast cancer. Results demonstrate that breast cancer cells reliably engraft in this xenograft model and in the early passages reflect the cellular and biological diversity found in the original human tumor. These tests also show that different populations of cancer cells may differ in their ability to form tumors.

### Example 2

### Characterizing the Wnt/β-catenin Pathway in Human Breast Cancer Tumors

This examples describes how one could characterize the Wnt/β-catenin pathway in human breast cancer tumors using the xenograft model described above. The Wnt/β-catenin pathway plays a role in the proliferation and self-renewal of normal stem cells. Although a significant percentage of human breast cancers appear to have constitutive activation of this critical pathway, unlike colon cancer, it has not been definitively established what role this pathway plays in the pathology of this disease in humans⁸⁴⁻⁸⁹. The xenograft model described above may be used to characterize the biological consequences of this pathway in human breast cancer tumors. These tests are done using cancer cells directly after removal from patients and early passage xenograft tumors.

**The function of the Wnt/frizzled/β-catenin signaling pathway in multiple patients' tumors.** Rationale: Almost 90% of colon cancers contain mutations that result in activation of β-catenin. The most common mutations are in the APC gene, which is involved in targeting β-catenin for degradationn, or mutations in the β-catenin protein itself^{43,111}. These latter mutations prevent degradation. Although the cancer cells in many breast tumors appear to have constitutively acitve of β-catenin⁸⁴, in contrast to colon cancer, mutations in the APC gene or β-catenin itself account for only 6-10% of these cases⁸⁴⁻⁸⁹. Examination of the Wnt/β-catenin signaling pathway in breast cancer cells should lead to new insights into the pathogenesis of this disease. There are a large number of Wnt proteins that are thought to differentially bind to different Frizzled receptors^{43,65,69,78,112}. Only a subset of Wnts, and by inference Frizzled receptors, can activate β-catenin. Normally, β-catenin is bound to E-cadherin at the cell membrane. Cytoplasmic β-catenin forms a complex with the APC and Axin proteins and facilitates β-catenin phosphorylation by GSK3β^{87,88,111}. The phosphorylated β-catenin is then degraded via the ubiquitin degradation pathway. However, upon activation of frizzled receptors by a Wnt, β-catenin is stabilized. The protein then translocates to the nucleus where it forms a complex with the LGLS/BCL9, PYGO and TCF proteins to activate transcription^{113,114}. We believe that our xenograft model and cellular assays are unique and powerful tools for understanding this critical pathway. We analyze 10 tumors that have constitutive β-catenin signaling and 10 that do not. These studies give new insights into the mechanisms by which the Wnt pathway is activated and the consequences of this activation in human breast cancer.

In mice, ectopic expression of various Wnt proteins results in breast tumor formation, while in humans activated β-catenin in breast cancer cells is associated with expression of cyclin D1 and poor prognosis^{78,84,115,116}. However, it is not known whether continuous β-catenin signaling is necessary for tumorigenic breast cancer cells to form tumors. There are several possible roles that constitutive β-catenin signaling can play in human breast cancer. First, it can be necessary for continued proliferation and/or viability of the tumorigenic cancer cells. Next, it can be necessary for the initiation of the tumor, but subsequent mutations bypass the need for β-catenin signaling. Third, it may make the cancer cells more resistant to chemotherapy due to the activation of downstream targets such as cyclin D1. Fourth, constitutive β-catenin signaling accelerates cancer cell growth, but is not necessary for tumorigenicity. Finally, the role of β-catenin signaling in tumor formation might differ in tumors with and without constitutive activation of β-catenin. For example, the former tumors might require β-catenin signaling whereas the latter tumors might require Wnt signals from other tumor cells or they might be independent of β-catenin because they have constitutive activation of downstream targets such as c-myc and/or cyclin D1. The tests described here are designed to distinguish between these possibilities using a novel xenograft model of human cancer. The data shows that the xenograft model virtually recapitulates a human breast tumor. Thus, this model allows us to study the Wnt pathway in de novo human tumors in as physiological conditions as possible.

**Is β-catenin signaling required for tumor formation by cancer cells isolated from multiple pateints?** The tests here determine whether the β-catenin pathway is obligate for breast cancer cell growth or whether activation is not required for tumor formation but does increase the rate of proliferation of the cancer cells. Although the xenograft tumors appear to closely resemble human tumors, over time selection pressure result in tumors that are adapted to the mouse environment. The cancer cells in such tumors differ in some ways with the cancer cells that made up the original human tumors. We identify cancer cells from five different xenograft tumors and five unpassaged tumors that have activated β-catenin (cytoplasmic and/or nuclear expression by immunohistochemistry) and cancer cells from five xenograft tumors and five unpassaged tumors that do not (membrane-associated expression by immunohistochemistry). We select tumors that are heterogeneous for important prognostic features that include estrogen receptor/progesterone receptor (ER/PgR), primary tumor vs. metastatic tumor, wild type vs. mutant p53, and amplification of Her2/neu.

To identify cells that have constitutive activation of β-catenin, we take advantage of the observation that this results in stabilization of β-catenin and accumulation of the protein in the cytoplasm and nucleus. When not activated, β-catenin is associated with the plasma membrane. We therefore analyze the breast cancer cell population from each of the tumors using immunohistochemistry to determine the sub-cellular localization of β-catenin and using flow cytometry to determine the amount of β-catenin expressed by each population of cells. To do this, we use flow cytometry to isolate the Lineage cancer cells from multiple tumors. Viably frozen xenograft or patient tumor cells are used for this analysis. The cancer cells then are stained with an anti-β-catenin-FITC antibody for immunohistochemistry and flow cytometry analysis using the antibody manufacturer's protocol (Transduction Laboratories). Cells with activated β-catenin have cytoplasmic/ nuclear localization and increased levels of the protein.

To determine the role of β-catenin signaling in tumorigenesis, Lineage⁻ cancer cells isolated from each of the 20 tumors are infected with either an adenovirus vector or a lentivirus vector that contains a dominant-negative (dn) TCF4-IRES-GFP minigene or a control GFP virus (for details of virus construction and use, see¹¹⁷). The adenovirus vector express the dnTCF4 transiently for 1-3 weeks, while the lentivirus vector express the dnTCF4 permanently. The dnTCF4 adenovirus has already been made using a dnTCF4 minigene (a gift from Eric Fearon). The dnTCF4 forms a complex with β-catenin thereby inhibiting transcriptional transactivation by the activated β-catenin. Note that the dnTCF4 blocks signaling from all members of the TCF family that mediate β-catenin signaling (Eric Fearon, personal communication). Limiting dilution tests are done to determine the ability of the transduced cells to form colonies *in vitro* and tumors *in vivo.* The tests here are done using cancer cells isolated from either patient or human tumors by flow-cytometry. By eliminating the lineage cocktail to eliminate the normal cells, colony formation in tissue culture and tumor formation in mice by cancer cells can be measured (The possible contributions of normal stromal cells to the growth of tumorigenic cells are analyzed as described below in aim 2B). To determine the role of β-catenin signaling on cancer cell growth and viability, five sets of 1,000, 5, 000, 20,000, 50,000 and 100,000 Lineage⁻ cancer cells from each of the tumors infected with the dnTCF4 viruses (either the adenovirus or lentivirus vectors) and control viruses are cultured *in vitro* in medium containing the Notch ligand Delta and the number of colonies that form are determined. The colonies in a control tissue culture plate are stained with cytokeratin to confirm that they arose from neoplastic cells⁹⁶. Two days after infection the cells are examined with a fluorescent microscope to confirm that greater than 90% of the cells were transduced by the virus. Similarly, *in vivo* limiting dilution tests are done to determine whether the dnTCF4 viruses affect tumor formation by the cancer cells isolated from the different patients. After infection, ten sets of 5, 000, 20,000, 50,000 and 100,000 Lineage⁻ cancer cells are isolated by flow-cytometry and then infected with the dnTCF4 adenovirus or control adenovirus. The infected cells are injected into the breast of NOD/SCID mice. We then determine the number of cancer cells needed to form tumors in each group, the time needed to form tumors in each group, the rate of growth of each group, and the size of the tumors that form in each group. This allow us to determine whether β-catenin is necessary for tumor formation by cancer cells that do or do not have constitutively activated β-catenin.

Subsequent tests depend on the results of the *in vivo* and *in vitro* limiting dilution tests. If inhibition of β-catenin transcriptional transactivation blocks tumor formation or slows tumor growth, then we begin to test whether downstream β-catenin targets such as cyclin D1 or c-myc are required for tumorigenicity^{84,85,118}. To do this, we infect the Lineage- cancer cells isolated by flow-cytometry and infect the cells with either the control or dnTCF4 adenovirus as well as a control gfp vector, a c-myc-IRES-gfp retrovirus vector, a cyclin D1-IRES-rfp retrovirus vector, or both the myc-IRES-gfp and the cyclin D1-IRES-rfp retrovirus vectors. Infected cells are isolated by flow cytometry, and then ten sets of 5,000, 10,000, 20,000, 50,000 or 100,000 Lineage- cancer cells of each test group are injected into mice. The mice are analyzed weekly for the formation of tumors, and the rate of growth of each test group. This allows us to determine whether enforced expression of either c-myc and/or cyclin rescues the cells from inhibition of β-catenin signaling.

If inhibition of β-catenin does not have any discernable effects on tumor formation, we first confirm that both of the dominant-negative viruses are inhibiting expression of the dnTCF4 minigene. If not, we use another method to inhibit the β-catenin pathway. In addition to RNA-i and antisense approaches¹¹⁹⁻¹²², overexpression of Axin (which targets β-catenin for degradation) can be used to inhibit β-catenin^{66,75}. If β-catenin signaling was inhibited and there was minimal or no effect on tumor formation, then we determine whether there are more subtle changes on the cancer stem cells. Expression of cyclin D1, whose expression is induced by β-catenin, has been associated with resistance to chemotherapy. Therefore, we treat mice with Adriamycin (8mg/kg) or Taxol (60mg/kg) five days after the dnTCF4-transduced or control cancer stem cells were injected into mice to determine whether inhibition of β-catenin enhance the efficacy of chemotherapy. The effect on tumor formation and tumor growth rate is determined as described above.

**Expected results**. Although cancer cells in a significant number of breast tumors have a constitutively active β-catenin signaling pathway, it is not known whether this pathway is essential for malignant transformation. If the Wnt/ β-catenin pathway is necessary for the cancer cells to form tumors, then dominant-negative inhibitors block the ability of cancer cells to form tumors. If constitutive β-catenin signaling enhances tumor cell growth after malignant transformation but is not necessary for tumor formation, then the dominant-negative inhibitor slow growth of the tumor cells but not block tumor formation. If oncogenic mutations subsequent to tumor initiation make the cells independent of Wnt signaling, then the dominant-negative inhibitor do not affect tumor formation or growth. Finally, it is possible that constitutive activation of the Wnt pathway contributes to resistance to apoptosis and therefore makes the cells resistant to chemotherapy.

In a model of mouse cancer, a brief inhibition of c-ras or c-myc activity in cancer cells transformed by these genes resulted in a permanent loss of tumorigenicity^{123,124}. If this is also true for β-catenin signaling, then transient inhibition of signaling by the adenovirus inhibit tumor formation. If inhibition of β-catenin signaling inhibits tumorigenicity, but the cells remain viable and restoration of β-catenin signaling enables them to form tumors, then the adenovirus vector slow tumor formation wherease the lentivirus vector inhibit tumor formation. If β-catenin signaling increases the rate of proliferation but is not obligate for tumorigenicity, then both viral vectors delay tumor formation and slow the growth of the tumors. If some tumors rely on β-catenin signaling and others rely on other pathways or have constitutive activation of downstream effectors of β-catenin signaling, then some tumors are affected by the viral vectors while others do not. The tests described in this aim allow us to answer these critical questions using a unique model recapitulates human tumors. These tests for the first time delineate the biological function(s) of β-catenin signaling in de novo human breast cancers.

The lentivirus can be made using other envelopes until one is found that infects the cells efficiently^{125,126}.

Note that with the lentivirus vector, infection efficiency may only be in the range of 30-70%. This would mean that a significant number of tumor cells would remain that could form tumors. However if inhibition of β-catenin signaling inhibits tumor formation, then the resultant tumors would not express gfp. Flow cytometry is used to measure gfp-expressing cells in the tumors infected with the dnTCF4 and control viruses. The tumors arising from the dnTCF4 group have a marked decrease in such cells if β-catenin signaling does play a role in tumor formation.

**Does inhibition of β-catenin signaling alter the phenotype of tumorigenic breast cancer cells?** One of the informative markers useful for the separation of tumrorigenic and non-tumorigenic breast cancer cells is CD44. Interestingly, CD44 is one of the target genes that is transcriptionally upregulated by β-catenin and epithelial stem cells, but not their differentiated progeny, are felt to express this marker^{83,127}. We contemplate that inhibition of β-catenin signaling result in the differentiation of the tumorigenic breast cancer cells and cause them to lose expression of CD44. We further contemplate that the CD44⁻ non-tumorigenic cancer cells do not have active β-catenin. To test this, we use flow-cytometry to isolate ESA⁺CD44⁺CD24^{-/low}Lineage-cancer cells from Tumor 1, Tumor 2 and Tumor 3 and infect them with the dnTCF4 adenovirus or a control adenovirus. The cells are cultured in tissue culture medium containing soluble Delta. We have found that this medium allows the tumorigenic cells to grow in tissue culture for 1-3 weeks. The cells are monitored for growth in vitro over a 3-week period. In addition, 1, 3 and 7 days after infection, the dnTCF4 adenovirus or a control adenovirus infected cells are analyzed by flow-cytometry for the expression of ESA, CD44 and CD24.

Next, we determine if there is a difference in β-catenin signaling in the tumorigenic cancer cells, the CD44⁺ cancer cells, or the CD44⁻ cancer cells. To do this, we use flow-cytometry to isolate ESA⁺CD44⁺CD24^{-/low}Lineage- tumorigenic cancer cells, CD44⁺ cancer cells, and CD44⁻ non-tumorigenic cancer cells from tumor 1, tumor 2 and tumor 3. Each population of cells are stained with an anti-β-catenin antibody that has been conjugated with APC. Each population of cells are analyzed by fluorescent microscopy to determine whether the β-catenin is membrane bound (not constitutively active), and by flow-cytometry to determine the amount of the protein in the cells. The level of β-catenin is associated with activity. In addition, we use commercially available antibodies that recognize phosphorylated and unphosphorylated β-catenin. The phosphorylated form is marked for degradation while the unphosphorylated form is active^{128,129}. These tests allow us to determine whether CD44 expression and β-catenin signaling are linked in patients' cancer cells.

CD44 is one of the best markers that allows one to distinguish tumorigenic cancer cells from non-tumorigenic cancer cells. Since CD44 is transcriptionally activated by β-catenin, then inhibition of β-catenin signaling result in downregulation of CD44.

**Does the differential expression of the frizzled proteins affect breast cancer stem cell fate in Tumor 1?** Data suggest that in Tumor 1, the tumorigenic stem cells express frizzled 2 and 6, whereas the non-tumorigenic neoplastic cells express Wnt 3, 4, 7A, 7B, 10B, and 11. This suggests a paracrine system in this particular tumor where the non-tumorigenic cells might drive the proliferation of the cancer stem cells. Preliminary data also suggest that in Tumor 1, the tumorigenic stem cells express frizzled 2 and 6, whereas the non-tumorigenic neoplastic cells express frizzled 2 and 7. It is possible that differential expression of frizzled genes plays a role in cancer cell fate decisions. The other possibility is that differential expression of these genes is a function of differentiation or immortality but does not directly regulate cell fate decisions in this tumor. This sub-aim distinguish between these possibilities.

Tumor 1 tumorigenic cells express frizzled 6 and non-tumorigenic cancer cells express frizzled 7. It is possible that frizzled 6 enhances and frizzled 7 inhibits the proliferation or self-renewal of the cancer cells. To test this possibility, *in vitro* and *in vivo* clonogenic assays are done. Tumor 1 tumorigenic and non-tumorigenic cancer cells are infected with a lentivirus vector that expresses either frizzled 6-IRES-GFP or frizzled 7-IRES-GFP. A lentivirus is used rather than an adenovirus since the former virus can infect and stably transduce a high proportion of primary cells, whereas adenovirus transduction is often transient. It is conceivable that expression of frizzled 6 confers the ability to self renew to the cancer cells. If so, infection of stem cells and/or non-tumorigenic cells with a lentivirus vector containing a frizzled 6-IRES-GFP minigene may enhance tumorigenicity of the stem cell or allow the previously non-tumorigenic cells to form tumors. Conversely, enforced expression of frizzled 7 may inhibit tumorigenicity. After infection with either the frizzled or control virus, limiting dilution tests are done to determine whether enforced expression of each gene alters the ability of each population of cancer cells to form tumors.

These tests allow us to determine whether enforced expression of frizzled 6 increases stem cell proliferation and/or self-renewal or expression of frizzled 7 inhibits tumorigenic cancer cell proliferation and/or self-renewal. To test this possibility, we isolate the tumorigenic ESA⁺CD44⁺CD24^{-/low}Lineage⁻ cancer cells and the other Lineage⁻, non-tumorigenic cancer cells are isolated by flow-cytometry from each of the tumors. First, immunohistochemistry are done using the anti-β-catenin antibody to determine whether there is a difference in the amount of active β-catenin in the tumorigenic and non-tumorigenic cells. Next, we determine the amount of phosphorylated (inactivated) and non-phosphorylated (active) β-catenin the tumorigenic and non-tumorigenic cells¹²⁸.

Next, *in vitro* assays are designed to determine the affects of each gene on colony formation by tumorigenic and non-tumorigenic cancer cells in tissue culture. After isolation by flow cytometry, each population of cells are infected with an identical MOI of either the frizzled 6 /GFP, frizzled 7/GRP or a control GFP virus. Triplicate cultures of 100, 500, 1,000 and 5,000 cells are placed in tissue culture medium. The total number of GFP⁺ colonies as well as the total number of colonies and the number of GFP⁺ colonies are counted on days 3, 7, 14, 21 and 28. At the end of 21 days, we attempt to pass the cells to determine whether expression of the particular frizzled gene affects self-renewal.

The influence of enforced expression of each frizzled gene on the ability of the neoplastic cells to form tumors in the NOD/SCID mice are determined. Normally, 200 Tumor 1 cells are required to form a tumor. Therefore, the frizzled 6, frizzled 7 or a control GFP lentivirus are used to infect 50, 100, 500, 1,000, 5,000, and 10,000 tumorigenic cancer cells or non-tumorigenic cancer cells. The cells are injected into the immunodeficient mice. The number of cells needed to form tumors and the rate of tumor growth are monitored. After the tumors have reached one centimeter in size, they are excised and analyzed by flow cytometry for expression of GFP. By comparing the percentage of cells infected by the GFP virus and frizzled /GFP virus, we are able to estimate the efficiency of infection and the affect of the latter virus on proliferation. These tests are replicated three times.

**Predicted Results:** In Tumor 1, different populations of cells express different frizzled proteins, and the non-tumorigenic cells appear to preferentially express Wnt proteins. This suggests that certain populations of non-tumorigenic cells promote tumor formation through Wnts. If β-catenin signaling is downregulated in the non-tumorigenic cells and active in the tumorigenic subset, this is detected by the immunohistochemistry analysis of the expression patterns of phosphorylated & unphosphorylated β-catenin in the non-tumorigenic and tumorigenic cancer cells respectively. If there is no affect of the particular frizzled/GFP virus, then a similar percentage of cells would express GFP in each group and there is no difference in the number of cells needed to form a tumor. If the particular frizzled virus decreases or increases tumorigenicity or proliferation, then tumors infected with frizzled/GFP virus would have fewer or more GFP⁺ cells and/or would require more or fewer cells to form tumors, respectively.

If necessary, a Feline Leukemia Virus lentivirus based vector system is used. This latter vector efficiently transduces non-replicating cells, and results in prolonged expression of transgenes. We can infect cells using a tet-inducible dnTCF-IRES-GFP lentivirus flanked by gene insulators or a control GFP lentivirus. 1-2 days prior to harvesting tumors, the transgene is activated. GFP⁺ breast cancer stem cells are harvested and transplanted into the breasts of NOD/SCID mice. We continue to induce the expression of the transgene in the mice, and we are able to monitor them for the ability of the cells to form tumors.

**The β-catenin signaling pathway differs in the cancer cells isolated from cancers with and without constitutively activated β-catenin**. Rationale: Unlike colon cancer, mutations in the β-catenin signaling pathway have been detected in only a minority of breast cancer cells. However, these studies have concentrated only on APC and β-catenin. In this aim, we closely examine the β-catenin pathway in each of the tumors that were analyzed at the biological level in specific aim 1A.

**Does the Wnt pathway differ in cancer cells isolated from differen**t **tumors?** In these experiments, we characterize the Wnt/β-catenin pathway in each of the tumors. To do this, we use RT-PCR to amplify the coding sequence of β-catenin, each of the frizzled proteins, the low-density lipoprotein-related Wnt receptors, APC, TCF family members, Axin, and Bcl-9 expressed by the cancer cells from each of the 10 tumors with constitutive activation of β-catenin. RT-PCR products of the expressed genes are sequenced to determine whether there are mutations in any of the genes. Any possible mutant genes are confirmed by repeated sequencing of an independent RT-PCR sample. If mutations are found, we determine whether the mutations result in the constitutive activation of the Wnt/β-catenin pathway. To do this, the mutated gene-IRES-GFP are cloned into the pCDNA3 eukaryotic expression vector. For example, if we find a mutant frizzled 2, then HEK 293 cells (which do not have activated B-catenin¹³⁰) are transfected with the mutant frizzled 2-IRES-GFP expression vector or a control IRES-GFP vector. Cells are stained with an anti-β-catenin-PE antibody and fluorescent microscopy is done to determine whether the mutant frizzled 6 causes cytoplasmic/nuclear localization of β-catenin, indicating activation of signaling. This assay allow us to determine whether mutation of components of the β-catenin pathway result in aberrant signaling in human breast cancer stem cells.

**Expected results.** Although constitutively active β-catenin is seen in the cancer cells in a significant number of breast cancer tumors, the mechanism is not known. There are differences in the signaling pathway in different tumor cells that are detected by these studies. If a mutation in a Wnt receptor or β-catenin modifier is present, then the sequencing studies detect this difference. If autocrine stimulation is present, then we see expression of one of the Wnt ligands by the cancer cells.

**Does Wnt expression by different populations of tumor cells in some tumors drive breast cancer cell growth?** Perhaps more so than any other type of cancer, a breast cancer tumor contains a heterogeneous population of normal cells including mesenchymal (stromal) cells, inflammatory cells, and endothelial cells that interact with malignant cells to modulate tumor growth and invasion. The purpose is to begin to understand the role of the Wnt pathway in such interactions. We contemplate that normal stromal elements including mesenchymal and endothelial cells produce different Wnts that influence tumor cell proliferation and invasion. Just unpassaged tumors are analyzed since the xenograft tumors would be expected to have infiltrating normal mouse stromal cells and analysis of the mouse cells would be too complicated. Purification of these cells by flow-cytometry allow both molecular and biological analysis of these cells without first placing the cells in tissue culture. This is particularly important since the normal cells are known to change expression of genes when cultured *in vitro.*

The normal stromal cells are thought to play a role in the proliferation of breast cancer cells. It is also likely that the cell-cell interactions between cancer cells contribute to tumor growth. Wnt signaling is one of the major pathways that normal tissue cells use to talk to each other. Therefore, it is important to understand how this pathway is regulated in tumors. Specific Wnt proteins can activate specific frizzled receptors. Some frizzled receptors signal through β-catenin, while others signal through different pathways. To understand how the various populations of tumor cells within a tumor might talk to the tumorigenic breast cancer cells through this pathway, we must first determine which frizzled and Wnt genes are expressed by the normal cells and the cancer cells from multiple patients' tumors. Therefore, we identify the Wnt pathway genes that are expressed by each population of normal cells and the cancer cells isolated from the 5 patients' tumor samples that have constitutive β-catenin signaling and the cancer cells from 5 patients' tumors that do not have constitutive activation of this protein.

Since our evidence suggests that there are differences in the expression of Wnt and frizzled genes in the different populations of cancer cells, it is important to isolate the different phenotype subsets of cells in the cancer to do these tests. This is because the apparently tumorigenic population of cells is a minority population, and the genes that these cells express might otherwise be missed in the analyses. Therefore, flow-cytometry is used to isolate tumorigenic and non-tumorigenic breast cancer cells, as well as normal endothelial cells and fibroblasts from the patients' original tumor. This is done as described in preliminary results and aim 1. RNA is isolated from pools of 35,000 of each population of cells and then linear amplification is done to make sufficient probe for the microarray analysis¹³¹⁻¹³⁵. To determine which frizzled and Wnt genes are expressed by the each population of cells found in each tumor, we probe an affymetrix microarray chip (3 chips for each cell type) that includes the Wnt and frizzled genes (the newly released U133 chip has the majority of these genes).

Results are confirmed by quantitative RT-PCR of the different populations of cancer cells isolated from the primary tumors with and without activated β-catenin in the cancer cells. Real time RT-PCR is done to determine the level of expression of each of the frizzled and Wnt genes by different populations of normal and neoplastic tumor cells. To do this, we make PCR primers for detection each of these genes. Each set of primers span at least one exon so RT-PCR can be used to detect expression of the mRNA in different populations of tumor cells. Flow-cytometry is used to isolate the tumorigenic population of cells identified in each of the tumors. Real-time PCR then is used to measure the expression of each of the Wnt pathway-related RNAs by each respective cell population identified in the microarray analysis (reviewed in ¹³⁶). To do the real-time PCR gene expression analysis, mRNA is purified from 3x10⁴ cells (isolated by flow-cytometry). Part of the RNA is used to directly measure RNA amount by the Ribogreen RNA quantitation method (Molecular Probes, Eugene, OR), and part used to measure rRNA and GAPDH expression (a control housekeeping gene) via the Taqman real-time RT-PCR assay. Taken together, these control measurements allow us to normalize expression of the genes of interest between the different populations of cells¹³⁶. Although fewer cells may be used in this assay, analysis of RNA isolated from 3x10⁴ cells should result in a more accurate measurement of gene expression.

Each frizzled receptor expressed by the different populations of cancer cells from each tumor is analyzed for the ability to activate β-catenin and transform cells when stimulated by each of the different Wnt genes that are expressed by different populations of cells within a tumor. Two biological systems are used for these studies. First, we use HEK 293 cells transfected with each individual frizzled identified in this screen to test the ability of the identified Wnts to activate β-catenin through the frizzled proteins expressed by the tumorigenic cells. Next, we use a mammary epithelial cell line to determine whether a particular Wnt or frizzled gene is able to transform the cell line.

To measure the biochemical functions of the different Wnt and Frizzled proteins expressed by the breast cancer cells, we use a transient transfection assay as described by Gazit et al.¹³⁰. In this assay, HEK 293T cells are transiently transfected with a frizzled minigene or a control minigene and aTCF-luciferase or control reporter minigene. To test the ability of a particular Wnt protein to stimulate β-catenin signaling, a second group of HEK 293T cells are transfected with each of the Wnt genes expressed by the various populations of tumor cells. The frizzled-transfected cells are mixed with the Wnt-transfected cells to measure paracrine activation of a particular frizzled receptor expressed by the breast cancer stem cells activates β-catenin when stimulated by a particular Wnt protein expressed by one of the various populations of tumor cells.

The C57MG cell line is used to determine whether activation of particular frizzled receptors by particular Wnts causes morphological transformation¹³⁷. These cells undergo morphologic transformation when exposed to Wnt-1, Wnt-2, Wnt-3A, Wnt-6 and Wnt-7A, but not Wnt4, Wnt-5A, Wnt-5B and Wnt-7B. These data suggest that the non-transforming Wnts signal differently than the transforming Wnts, or that they signal through different receptors not expressed by the C57MG cells. Therefore, to fully characterize the functions of the different frizzled and Wnt proteins expressed by the cancer cells in the patients' tumors, we must first determine which frizzled genes are expressed by the C57MG cells. The cells are transfected with minigenes that express any frizzled genes expressed by tumorigenic breast cancer cells but not expressed by the C57MG cells. Next, cells are cultured in the presence of lethally irradiated fibroblasts or HEK 293T cells transfected with individual Wnt genes that were expressed by the different populations of tumor cells. The cells are analyzed for morphological transformation as described by Shimizu¹³⁸.

Next, we characterize the *in vivo* response of cancer cells from the different patients' tumors to different Wnts made by the tumor cells. The Wnt proteins are often found in the extracellular matrix and difficult to prepare in soluble forms. Therefore, we make control HEK 293 cell lines that express each of the Wnts made by the various types of tumor cells present in 2 patients' tumors. To do this, we first analyze HEK 293 cells to determine whether they constitutively make any of the Wnt proteins. Next, we stably transfect the HEK 293 cells with each of the Wnts made by the patients' tumor cells. To determine the affect of Wnt stimulation in the breast cancer cells by each of its ligands *in vivo*, 0,10, 50, 100, 200, 500 and 1,000 Tumor 1 stem cells are mixed with 500,000 lethally irradiated control 293 cells or 293 cells transfected with one or more relevant Wnt minigenes and then injected into immunodeficient mice. Each injection is done in five mice. The mice then be monitored weekly for tumor formation. If a particular Wnt stimulates self renewing cell division, then either fewer cells are needed to initiate a tumor and/or tumors form more quickly. Conversely, if the ligand induces commitment to differentiation, then more cells are required to form a tumor and/or tumors take longer to form.

**Expected results.** The interaction of cancer cells with the normal stromal cells in tumors is thought to be critical for tumor formation and metastasis³³. The Wnt pathway is one of the central pathways by which cells in normal tissues communicate⁶⁵. It is therefore likely that such communications are maintained to some extent in tumors. The models described in this proposal for the first time enable such studies to be conducted using patients' tumor cells. If the stromal cells indeed promote tumor growth through Wnt signaling, then the various populations of stromal cells make specific Wnts that provide a proliferative signal for the tumorigenic cancer cells.

To minimize these problems, all tests are done in triplicate with different numbers of cells. Expression of a control RNA of a known quantity is used to construct a standard curve to analyze the data(reviewed in ¹³⁶). If necessary, new PCR primers are made, or RT is done with gene specific primers recognizing a different part of the mRNA (oligo dT primers are used for the RT reaction initially).

**Summary:** These tests for the first time describe in comprehensive detail the molecular mechanisms by which the β-catenin pathway is activated *in vivo* in tumorigenic populations of breast cancer cells obtained directly from multiple patients' tumors, and the biological consequences of this activation in de novo breast cancer cells.

### Example 3

### Localizatoin of β-catenin in Tumorigenic Cells

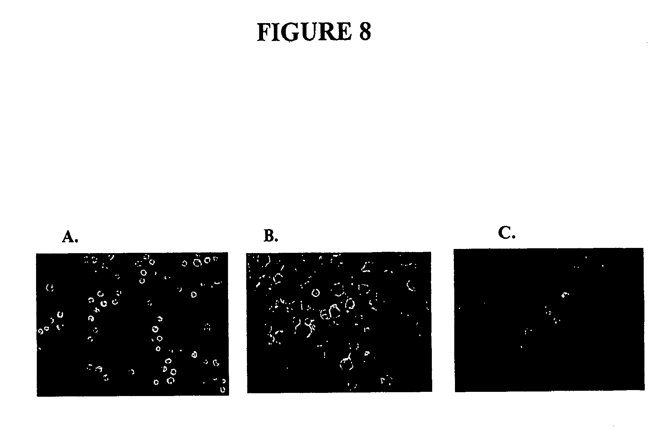

In normal hematopoietic cells, nuclear β-catenin is found only in the stem cell compartment. Reya *et al*. further demonstrate that β-catenin signaling is necessary for normal stem cells to self-renew. A recently completed analysis of the subcellular localization of β-catenin in tumorigenic and non-tumorigenic tumor 1 breast cancer cells further supports this notion. Normally, the subcellular distribution of β-catenin is heterogeneous in cancer cells. In some cells, the protein is located primarily in the outer membrane, while in others primarily in the nucleus. The subcellular distribution of the protein differs in the tumorigenic and non-tumorigenic cancer cells. The β-catenin is primarily located in the cytoplasm of the non-tumorigenic cancer cells, while it is primarily in the nucleus of the tumorigenic cells (Fig. 8). Since upon activation by a Wnt signal, β-catenin translocates from the cell membrane to the nucleus to activate downstream target genes, this data supports the hypothesis that Wnt signaling plays a role in the self-renewal of breast cancer stem cells.

Figure 8 shows subcellular localization of β-catenin. A FITC labeled anti-β-catenin antibody was used to stain (**A**) colon cancer cells, which have a constitutively activated β-catenin, (**B**) non-tumorigenic T1 breast cancer cells, and (**C**) tumorigenic breast cancer cells. The tumorigenic and non-tumorigenic cancer cells were isolated by flow cytometry as described in the PNAS manuscript by Al-Hajj *et al*. Note that the β-catenin is located primarily in the nucleus of the colon cancer cells and the breast cancer stem cells, but it is primarily located on the surface of the non-tumorigenic cells.

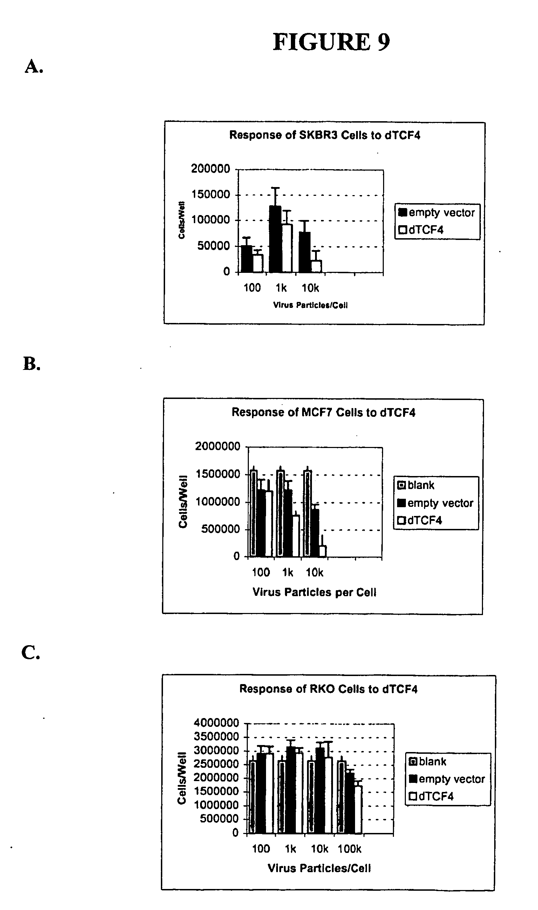

To begin to understand the biological consequences of β-catenin signaling in breast cancer, we have tested our dominant negative TCF-4 (dTCF4) adenovirus vector in several cell lines. This adenovirus acts to inhibit β-catenin signaling. Two different breast cancer cell lines, SKBR3 and MCF7, and a gastrointestinal tract cancer cell line, RKO, were infected with the dTCF4 adenovirus or a control adenovirus (empty vector). Four days after infection, the number of viable cells in each group was determined. As shown in Figure 9, the breast cancer cells infected with the dTCF4 adenovirus, but not the control adenovirus, died. These data show that the Wnt pathway does play a role in human breast cancer.

Figure 9 shows inhibition of β-catenin signaling in cancer cells. Triplicate cultures of SKBR3 cells (A), MCF7 cells (B) and RKO cells (C) were infected with either an control adenovirus (empty vector) or an adenvovirus vector that expresses a dominant-negative TCF4 minigent (dTCF4). With increasing virus concentrations, SKBR3 cells and MCF7 cells, but not RKO cells, lost viablility. Note that the virus titers resulting in cell death were those needed to efficiently infect most of the target cells with a control GFP virus (data not shown). This experiment has been repeated with similar results.
The observation that β-catenin is located primarily in the nucleus in the tumorigenic but not the non-tumorigenic cancer cells taken together with the observation that inhibition of β-catenin signaling affects the viability of some breast cancer cell lines shows that like normal stem cells, Wnt signals may play a role in the self-renewal of cancer stem cells.

### Example 4

### Identifying Stem Cell Cancer Markers

This Example describes how various stem cell cancer markers were identified using microarray screens. The results of these screens were processed and the names of the differentially expressed genes are reported in Tables 4-8 (see above).

In order to generate gene expression profiles, human breast tumorigenic cells which were initially isolated. A series of samples were accumulated from human breast tumors or normal tissues. These were generated as follows. Three passaged breast tumors---breast tumor cells from patient 1, 2, 3 were engrafted on miceach tumor was engrafted on three mice to make the triplicate tumors. The breast tumorigenic cells were then isolated from these tumors. Two or three unpassaged breast tumors from three patients SUM, PE13, PE15 were labeled and sorted into tumorigenic cells (TG) or non-tumorigenic cells (NTG). Both PE15-TG and PE15-NTG were triplicate. Two or three normal breast samples were from breast reduction patients. Breast epithelial cells (Breast) were isolated with flow cytometry and used for microarray. Two or three normal colon samples were collected freshly from colon patients. Colon epithelial cells (Colon) were isolated with flow cytometry and used for microarray. Two or three normal stem cell samples (normal bone marrow) were collected from bone marrow donors. Hematopoietic stem cells (HSC) were isolated with flow cytometry. Probes were made from the following were made from the various cells types for use in the mircoarray analysis.

In order to perform the various microarray screens Affymetrix HG-U133 gene chips were used. The normalized gene expression intensity was used to generate the data that was collected in a number of large tables. The results in these tables was processed and used to generate Tables 4, 5, 6, 7a, 7b, 7c, 7d and 8, which present the names of the genes found to be differentially expressed. For tables 4-6, candidate cancer markers were sorted by identifying genes whose expression was greater or less than 1.5 fold in unpassaged breast tumorigenic cells comparing to non-tumorigenic cells or the normal stem cells (HSC). Table 6 shows only those genes found to be down regulated in UPTG vs. UPNTG. Table 5 shows only those genes found to be up regulated in UPTG vs. HSC. Table 4 shows only those genes found to be up regulated in UPTG vs UPNTG. For tables 7a, 7b, 7c and 7d, cancer markers were were generated from the larger tables by standard T-test. These tables were sorted based on T-score is <0.01 and ratio is more than 2 fold. Table 7a shows only those genes found to be up regulated in UPTG vs. HSC. Tables 7b shows only those genes found to be down regulated in UPTG vs. HSC. Table 7c shows only those genes found to be up regulated in PTG vs HSC. Table 7d shows only those genes found to be down regulated in PTG vs HSC.

### References

1. Akashi, K. & Weissman, I. L. Developmental Biology of Hematopoiesis (ed. Zon, L. I.) (Oxford Univ. Press, New York, 2001).
2. Baum, C. M., Weissman, I. L., Tsukamoto, A. S., Buckle, A. M. & Peault, B. Isolation of a candidate human hematopoietic stem-cell population. Proc Natl Acad Sci USA 89, 2804-8 (1992).
3. Morrison, S., Hemmati, H., Wandycz, A. & Weissman, I. The Purification and Characterization of Fetal Liver Hematopoietic Stem Cells. Proc. Natl. Acad. Sci. USA 92, 10302-10306 (1995).
4. Morrison, S., Prowse, K., Ho, P. & Weissman, I. Telomerase activity in hematopoietic cells is associated with self-renewal potential. Immunity 5, 207-16(1996).
5. Morrison, S. J., Uchida, N. & Weissman, I. L. The biology of hematopoietic stem cells. Annu. Rev. Cell Dev. Biol. 11, 35-71 (1995).
6. Morrison, S. J., Wandycz, A. M., Hemmati, H. D., Wright, D. E. & Weissman, I. L. Identification of a lineage of multipotent hematopoietic progenitors. Development 124, 1929-39 (1997).
7. Morrison, S. J. & Weissman, I. L. The long-term repopulating subset of hematopoietic stem cells is deterministic and isolatable by phenotype. Immunity 1, 661-73 (1994).
8. Spangrude, G. J., Heimfeld, S. & Weissman, I. L. Purification and characterization of mouse hematopoietic stem cells. Science 241, 58-62 (1988).
9. Uchida, N. et al. Direct isolation of human central nervous system stem cells. Proceedings of the National Academy of Sciences of the United States of America 97, 14720-5 (2000).
10. Morrison, S. et al. Transient Notch activation initiates an irreversible switch from neurogenesis to gliogenesis by neural crest stem cells. Cell 101, 499-510 (2000).
11. Morrison, S. J., Shah, N. M. & Anderson, D. J. Regulatory mechanisms in stem cell biology. Cell 88, 287-98 (1997).
12. Baum, C., Uchida, N., Peault, B. & Weissman, I. L. Bone Marrow Transplantation (eds. Forman, S. J., Blume, K. G. & Thomas, E. D.) (Blackwell Scientific Publications, Boston, 1994).
13. Negrin, R. S. et al. Transplantation of highly purified CD34+Thy-1+ hematopoietic stem cells in patients with metastatic breast cancer. Biol Blood Marrow Transplant 6, 262-71 (2000).
14. Voena, C. et al. Qualitative and quantitative polymerase chain reaction detection of the residual myeloma cell contamination after positive selection of CD34+ cells with small- and large-scale Miltenyi cell sorting system. Br J Haematol 117, 642-5 (2002).
15. Michallet, M. et al. Transplantation with selected autologous peripheral blood CD34+Thyl+ hematopoietic stem cells (HSCs) in multiple myeloma: impact of HSC dose on engraftment, safety, and immune reconstitution. Exp Hematol 28, 858-70 (2000).
16. Tricot, G. et al. Collection, tumor contamination, and engraftment kinetics of highly purified hematopoietic progenitor cells to support high dose therapy in multiple myeloma. Blood 91, 4489-95 (1998).
17. Barbui, A. et al. Negative selection of peripheral blood stem cells to support a tandem autologous transplantation programme in multiple myeloma. British Journal of Haematology. 116, 202-210 (2002).
18. Al-Hajj, M., Wicha, M., Morrison, S. J. & Clarke, M. F. Prospecitve identification and isolation of breast cancer tumorigenic cells. Proc Natl Acad Sci USA (2002).
19. Park, C. H., Bergsagel, D. E. & McCulloch, E. A. Mouse myeloma tumor stem cells: a primary cell culture assay. J Natl Cancer Inst 46, 411-22 (1971).
20. Bruce, W. R. & Gaag, H. A quantitative assay for the number of murine lymphoma cells capable of proliferation in vivo. Nature 199, 79-80 (1963).
21. Wodinsky, I., Swiniarski, J. & Kensler, C. J. Spleen colony studies of leukemia L1210.I. Growth kinetics of lymphocytic L1210 cells in vivo as determined by spleen colony assay. Cancer Chemother. Rep. 51, 415-421 (1967).
22. Bergsagel, D. E. & Valeriote, F. A. Growth characteristics of a mouse plasma cell tumor. Cancer Res 28, 2187-96 (1968).
23. Southam, C. & Brunschwig, A. Quantitative studies of autotransplanation of human cancer. Cancer 14, 971-978 (1961).
24. Hamburger, A. W. & Salmon, S. E. Primary bioassay of human tumor stem cells. Science 197, 461-3 (1977).
25. Reya, T., Morrison, S. J., Clarke, M. F. & Weissman, I. L. Stem cells, cancer, and cancer stem cells. Nature 414, 105-11 (2001).
26. Lagasse, E. & Weissman, I. L. bcl-2 inhibits apoptosis of neutrophils but not their engulfment by macrophages. J Exp Med 179, 1047-52 (1994).
27. Morrison, S. J. et al. A Genetic Determinant That Specifically Regulates the Frequency of Hematopoietic Stem Cells. J. Immunol. 168, 635-642 (2002).
28. Christensen, J. L. & Weissman, I. L. Flk-2 is a marker in hematopoietic stem cell differentiation: a simple method to isolate long-term stem cells. Proc Natl Acad Sci USA 98, 14541-6 (2001).
29. Weissman, I. L. Translating stem and progenitor cell biology to the clinic: barriers and opportunities. Science 287, 1442-6 (2000).
30. Osawa, M., Hanada, K., Hamada, H. & Nakauchi, H. Long-term lymphohematopoietic reconstitution by a single CD34-low/negative hematopoietic stem cell. Science 273, 242-5 (1996).
31. Domen, J. & Weissman, I. L. Hematopoietic stem cells need two signals to prevent apoptosis; BCL-2 can provide one of these, Kitl/c-Kit signaling the other. J Exp Med 192, 1707-18 (2000).
32. Miller, C. L. & Eaves, C. J. Expansion in vitro of adult murine hematopoietic stem cells with transplantable lympho-myeloid reconstituting ability. Proc Natl Acad Sci USA 94, 13648-53 (1997).
33. Hanahan, D. & Weinberg, R. A. The hallmarks of cancer. Cell 100, 57-70 (2000).
34. Phillips, R. L., Reinhart, A. J. & Van Zant, G. Genetic control of murine hematopoietic stem cell pool sizes and cycling kinetics. Proc Natl Acad Sci U SA 89, 11607-11 (1992).
35. Muller-Sieburg, C. E., Cho, R. H., Sieburg, H. B., Kupriyanov, S. & Riblet, R. Genetic control of hematopoietic stem cell frequency in mice is mostly cell autonomous. Blood 95, 2446-8 (2000).
36. Domen, J., Cheshier, S. H. & Weissman, I. L. The Role of Apoptosis in the Regulation of Hematopoietic Stem Cells: Overexpression of BCL-2 Increases Both Their Number and Repopulation Potential. J. Exp. Med. 191, 253-264 (2000).
37. Domen, J., Gandy, K. L. & Weissman, I. L. Systemic overexpression of BCL-2 in the hematopoietic system protects transgenic mice from the consequences of lethal irradiation. Blood 91, 2272-82 (1998).
38. Traver, D., Akashi, K., Weissman, I. L. & Lagasse, E. Mice defective in two apoptosis pathways in the myeloid lineage develop acute myeloblastic leukemia. Immunity 9, 47-57 (1998).
39. Mucenski, M. L. et al. A functional c-myb gene is required for normal murine fetal hepatic hematopoiesis. Cell 65, 677-89 (1991).
40. Clarke, M. F., Kukowska-Latallo, J. F., Westin, E., Smith, M. & Prochownik, E. V. Constitutive expression of a c-myb cDNA blocks Friend murine erythroleukemia cell differentiation. Molecular & Cellular Biology 8, 884-92 (1988).
41. Danish, R. et al. c-myb effects on kinetic events during MEL cell differentiation. Oncogene 7, 901-7 (1992).
42. Prochowinik, E. & Kukowska, J. Deregulated expression of c-myc by murine erythroleukaemia cells prevents differentiation. Nature 322, 848-50 (1986).
43. Taipale, J. & Beachy, P. A. The Hedgehog and Wnt signalling pathways in cancer. Nature 411, 349-54 (2001).
44. Bhardwaj, G. et al. Sonic hedgehog induces the proliferation of primitive human hematopoietic cells via BMP regulation. Nat Immunol 2, 172-80 (2001).
45. Shivdasani, R., Mayer, E. & Orkin, S. Absence of blood formation in mice lacking the T-cell leukaemia oncoprotein tal-1/SCL. Nature 373, 432-4 (1995).
46. Porcher, C. et al. The T cell leukemia oncoprotein SCL/tal-1 is essential for development of all hematopoietic lineages. Cell 86, 47-57 (1996).
47. Buske, C. et al. Deregulated expression of HOXB4 enhances the primitive growth activity of human hematopoietic cells. Blood 100, 862-868 (2002).
48. Antonchuk J, S. G., Humphries RK. Related Articles. HOXB4-induced expansion of adult hematopoietic stem cells ex vivo. Cell 109, 39-45 (2002).
49. Cheng, T. et al. Hematopoietic Stem Cell Quiescence Maintained by p21cip1/waf1. Science 287, 1804-1808. (2000).
50. van Lohuizen, M., Frasch, M., Wientjens, E. & Berns, A. Sequence similarity between the mammalian bmi-1 proto-oncogene and the Drosophila regulatory genes Psc and Su(z)2. Nature 353, 353-355 (1991).
51. van der Lugt, N. M. et al. Posterior transformation, neurological abnormalities, and severe hematopoietic defects in mice with a targeted deletion of the bmi-1 proto-oncogene. Genes & Development 8, 757-769 (1994).
52. Artavanis-Tsakonas, S., Rand, M. D. & Lake, R. J. Notch signaling: cell fate control and signal integration in development. Science 284, 770-776 (1999).
53. Berry, L., Westlund, B. & Schedl, T. Germ-line tumor formation caused by activation of glp-1, a Caenorhabditis elegans member of the Notch family of receptors. Development 124, 925-936 (1997).
54. Shelly, L. L., Fuchs, C. & Miele, L. Notch-1 inhibits apoptosis in murine erythroleukemia cells and is necessary for differentiation induced by hybrid polar compounds. J Cell Biochem. 73, 164-175 (1999).
55. Varnum-Finney, B. et al. Pluripotent, cytokine-dependent, hematopoietic stem cells are immortalized by constitutive Notch1 signaling. Nat Med 6, 1278-81 (2000).
56. Gallahan, D. & Callahan, R. The mouse mammary tumor associated gene INT3 is a unique member of the NOTCH gene family (NOTCH4). Oncogene 14, 1883-1890 (1997).
57. Zagouras, P., Stifani, S., Blaumueller, C., Carcangiu, M. & Artavanis-Tsakonas, S. Alterations in Notch signaling in neoplastic lesions of the human cervix. Proceedings of the National Academy of Sciences of the United States of America 92, 6414 (1995).
58. Leethanakul, C. et al. Distinct pattern of expression of differentiation and growth-related genes in squamous cell carcinomas of the head and neck revealed by the use of laser capture microdissection and cDNA arrays. Oncogene 19, 3220-4 (2000).
59. Liu, Y. et al. Epitheial expression and chromosomal location of human TLE genes: implications for notch signaling and neoplasia. Genomics 31, 58-64 (1996).
60. Capobianco, A. J., Zagouras, P., Blaumueller, C. M., Artavanis-Tsakonas, S. & Bishop, J. M. Neoplastic transformation by truncated alleles of human NOTCH1/TAN1 and NOTCH2. Mol. Cell Biol. 17, 6265-6273 (1997).
61. Ellisen, L. W. et al. TAN-1, the human homolog of the Drosophila notch gene, is broken by chromosomal translocations in T lymphoblastic neoplasms. Cell 66, 649-661 (1991).
62. Imatani, A. & Callahan, R. Identification of a novel NOTCH-4/INT-3 RNA species encoding an activated gene product in certain human tumor cell lines. Oncogene 19, 223-231 (2000).
63. Jehn, B. M., Bielke, W., Pear, W. S. & Osborne, B. A. Cutting edge: protective effects of notch-1 on TCR-induced apoptosis. J Immunol. 162, 635-638 (1999).
64. Weizen, S. et al. Activation of Notch-1 signaling maintains the neoplasitc phenotype in human Ras-transformed cells. Nature Medicine 8, 979-986 (2002).
65. Cadigan, K. M. & Nusse, R. Wnt signaling: a common theme in animal development. Genes & Development 11, 3286-305 (1997).
66. Spink, K. E., Polakis, P. & Weis, W. I. Structural basis of the Axin-adenomatous polyposis coli interaction. Embo J 19, 2270-9 (2000).
67. Austin, T. W., Solar, G. P., Ziegler, F. C., Liem, L. & Matthews, W. A role for the Wnt gene family in hematopoiesis: expansion of multilineage progenitor cells. Blood 89, 3624-35 (1997).
68. Tsukamoto, A. S., Grosschedl, R., Guzman, R. C., Parslow, T. & Varmus, H.E. Expression of the int-1 gene in transgenic mice is associated with mammary gland hyperplasia and adenocarcinomas in male and female mice. Cell 55, 619-25 (1988).
69. Nusse, R. et al. A new nomenclature for int-1 and related genes: the Wnt gene family. Cell 64, 231 (1991).
70. Reya, T. et al. Wnt signaling regulates B lymphocyte proliferation through a LEF-1 dependent mechanism. Immunity 13, 15-24 (2000).
71. Wu, C., Zeng, Q., Blumer, K. J. & Muslin, A. J. RGS proteins inhibit Xwnt-8 signaling in Xenopus embryonic development. Development 127, 2773-2784 (2000).
72. VanDenBerg, D. J., Sharma, A. K., Bruno, E. & Hoffman, R. Role of members of the Wnt gene family in human hematopoiesis. Blood 92, 3189-3202 (1998).
73. Gat, U., DasGupta, R., Degenstein, L. & Fuchs, E. De Novo hair follicle morphogenesis and hair tumors in mice expressing a truncated beta-catenin in skin. Cell 95, 605-14(1998).
74. Chan, E. F., Gat, U., McNiff, J. M. & Fuchs, E. A common human skin tumour is caused by activating mutations in beta-catenin. Nat Genet 21, 410-3 (1999).
75. Hedgepeth, C. M., Deardorff, M. A., Rankin, K. & Klein, P. S. Regulation of Glycogen Synthase Kinase 3beta and Downstream Wnt Signaling by Axin. Mol. Cell. Biol. 19, 7147-7157 (1999).
76. Korinek, V. et al. Depletion of epithelial stem-cell compartments in the small intestine of mice lacking Tcf-4. Nat Genet 19, 379-83 (1998).
77. Zhu, A. J. & Watt, F. M. beta-catenin signalling modulates proliferative potential of human epidermal keratinocytes independently of intercellular adhesion. Development 126, 2285-98 (1999).
78. Nusse, r. The Wnt gene family in tumorigenesis and in normal development. Journal of Steroid Biochemistry & Molecular Biology 43, 9-12 (1992).
79. Weeraratna, A. T. et al. Wnt5 signaling directly affects cell motility and invasion of metastatic melanoma. Cancer Cell 1, 279-88 (2002).
80. Saitoh, T., Mine, T. & Katoh, M. Up-regulation of WNT8B mRNA in human gastric cancer. International Journal of Oncology 20, 343-8 (2002).
81. Saitoh, T., Mine, T. & Katoh, M. Frequent Up-regulation of WNT5A mRNA in primary gastric cancer. International Journal of Molecular Medicine. 9, 515-9 (2002).
82. Kirikoshi, H., Inoue, S., Sekihara, H. & Katoh, M. Expression of WNT10A in human cancer. International Journal of Oncology 19, 997-1001 (2001).
83. van de Wetering, M. et al. The beta-catenin/TCF-4 complex imposes a crypt progenitor phenotype on colorectal cancer cells. Cell 111, 241-50 (2002).
84. Lin, S. Y. et al. Beta-catenin, a novel prognostic marker for breast cancer: its roles in cyclin D1 expression and cancer progression. Proc Natl Acad Sci U S A 97, 4262-6 (2000).
85. Wong, S. C. et al. Expression of frizzled-related protein and Wnt-signalling molecules in invasive human breast tumours. J Pathol 196, 145-53 (2002).
86. Candidus, S., Bischoff, P., Becker, K. F. & Hofler, H. No evidence for mutations in the alpha- and beta-catenin genes in human gastric and breast carcinomas. Cancer Res 56, 49-52 (1996).
87. Sorlie, T., Bukholm, I. & Borresen-Dale, A. L. Truncating somatic mutation in exon 15 of the APC gene is a rare event in human breast carcinomas. Mutations in brief no. 179. Online. Hum Mutat 12, 215 (1998).
88. Jonsson, M., Borg, A., Nilbert, M. & Andersson, T. Involvement of adenomatous polyposis coli (APC)/beta-catenin signalling in human breast cancer. Eur J Cancer 36, 242-8 (2000).
89. Schlosshauer, P. W. et al. APC truncation and increased beta-catenin levels in a human breast cancer cell line. Carcinogenesis 21, 1453-6 (2000).
90. Hoffman, R. M. Orthotopic metastatic mouse models for anticancer drug discovery and evaluation: a bridge to the clinic. Invest New Drugs 17, 343-359 (1999).
91. Brown, J. M. NCI's anticancer drug screening program may not be selecting for clinically active compounds. Oncol Res. 9, 213-215 (1997).
92. Ikeda, H. et al. Changes in phenotype and proliferative potential of human acute myeloblastic leukemia cells in culture with stem cell factor. Exp. Hematol. 21, 1686-1694 (1993).
93. Weidmann, E. et al. Establishment and characterization of a new, factor-independent acute myeloid leukemia line designated Ei501. Leukemia 11, 709-713 (1997).
94. Leglise, M. C., Dent, G. A., Ayscue, L. H. & Ross, D. W. Leukemic cell maturation: phenotypic variability and oncogene expression in HL60 cells: a review. Blood Cells 13, 319-337 (1988).
95. Dorrell, C., Gan, O.I., Pereira, D.S., Hawley, R. G. & Dick, J. E. Expansion of human cord blood CD34(+)CD38(-) cells in ex vivo culture during retroviral transduction without a corresponding increase in SCID repopulating cell (SRC) frequency: dissociation of SRC phenotype and function. Blood 95, 102-110 (2000).
96. Ethier, S. P., Mahacek, M. L., Gullick, W. J., Frank, T. S. & Weber, B. L. Differential isolation of normal luminal mammary epithelial cells and breast cancer cells from primary and metastatic sites using selective media. Cancer Res 53, 627-35 (1993).
97. Krasna, L. et al. Large expansion of morphologically heterogeneous mammary epithelial cells, including the luminal phenotype, from human breast tumours. Breast Cancer Res Treat 71, 219-35 (2002).
98. Furley, A. J. et al. Divergent molecular phenotypes of KG1 and KGl a myeloid cell lines. Blood 68, 1101-1107 (1986).
99. Sakakibara, T. et al. Growth and metastasis of surgical specimens of human breast carcinomas in SCID mice. Cancer J. Si. Am 2, 291-300 (1996).
100. Al-Hajj, M., Wicha, M., Morrison, S. J. & Clarke, M. F. Prospecitve identification and isolation of breast cancer tumorigenic cells. Proc Natl Acad Sci USA (2003).
101. Heppner, G. H. Tumor heterogeneity. Cancer Res 44, 2259-65 (1984).
102. Fialkow, P. J. Human tumors studied with genetic markers. Birth Defects Orig Artic Ser 12, 123-32 (1976).
103. Weisenthal, L. & Lippman, M. E. Clonoggenic and nonclonogenic in vitro chemosensitivity assays. Cancer Treatment Reports 69, 615-632 (1985).
104. Lapidot, T. et al. A cell initiating human acute myeloid leukaemia after transplantation into SCID mice. Nature 17, 645-648 (1994).
105. Bonnet, D. & Dick, J. Human acute myeloid leukemia is organized as a hierarchy that originates from a primitive hematopoietic cell. Nature Medicine 3, 730-737 (1997).
106. Larochelle, A. et al. Identification of primitive human hematopoietic cells capable of repopulating NOD/SCID mouse bone marrow: implications for gene therapy. Nat Med 2, 1329-37 (1996).
107. Ahrens, T. et al. Soluble CD44 inhibits melanoma tumor growth by blocking cell surface CD44 binding to hyaluronic acid. Oncogene 20 (2001).
108. Kufe, D. et al. Biological behavior of human breast carcinoma-associated antigens expressed during cellular proliferation. Cancer Research 43, 851-857 (1983).
109. Porter, E. H. & Berry, R. J. The efficient design of transplantable tumor assays. Br J Cancer 17 (1964).
110. Packeisen, J., Kaup-Franzen, C. & Knieriem, H. Detection of surface antigen 17-1A in breast and colorectal cancer. Hybridoma 18, 37-40 (1999).
111. Webster, M. T. et al. Sequence variants of the axin gene in breast, colon, and other cancers: an analysis of mutations that interfere with GSK3 binding. Genes Chromosomes Cancer 28, 443-53 (2000).
112. Nusse, R. WNT targets. Repression and activation. Trends in Genetics 15, 1-3 (1999).
113. Korinek, V. et al. Two members of the Tcf family implicated in Wnt/beta-catenin signaling during embryogenesis in the mouse. Mol Cell Biol 18, 1248-56 (1998).
114. Kramps, T. et al. Wnt/wingless signaling requires BCL9/legless-mediated recruitment of pygopus to the nuclear beta-catenin-TCF complex. Cell 109, 47-60 (2002).
115. Nusse, R. & Varmus, H. E. Many tumors induced by the mouse mammary tumor virus contain a provirus integrated in the same region of the host genome. Cell 31, 99-109 (1982).
116. Nusse, R. Insertional mutagenesis in mouse mammary tumorigenesis. Current Topics in Microbiology & Immunology 171, 43-65 (1991).
117. Clarke, M. F. et al. A recombinant bcl-x s adenovirus selectively induces apoptosis in cancer cells but not in normal bone marrow cells. Proc.Natl.Acad.Sci. U.S.A 92, 11024-11028 (1995).
118. Yu, Q., Geng, Y. & Sicinski, P. Specific protection against breast cancers by cyclin D1 ablation. Nature 411, 1017-21 (2001).
119. Paul, C., Good, P., Winer, I. & Engelke, D. Effective expression of small interfering RNA in human cells. Nature Biotechnology 29, 505-508 (2002).
120. Caplen, N. J., Parrish, S., Imani, F., Fire, A. & Morgan, R. A. Specific inhibition of gene expression by small double-stranded RNAs in invertebrate and vertebrate systems. Proc.Natl.Acad.Sci.U.S.A 98, 9742-9747'(2001).
121. Martinez, J., Patkaniowska, A., Urlaub, H., Luhrmann, R. & Tuschi, T. Single-Stranded Antisense siRNAs Guide Target RNA Cleavage in RNAi. Cell 110, 563-574 (2002).
122. Sazani, P. et al. Nuclear antisense effects of neutral, anionic and cationic oligonucleatide analogs. Nucleic Acids Research 29, 3965-74 (2001).
123. Jain, M. et al. Sustained loss of a neoplastic phenotype by brief inactivation of MYC. Science 297, 102-4 (2002).
124. Chin, L. et al. Essential role for oncogenic Ras in tumour maintenance. Nature 400, 468-72 (1999).
125. Hughes, S. M., Moussavi-Harami, F., Sauter, S. L. & Davidson, B. L. Viral-mediated gene transfer to mouse primary neural progenitor cells. Mol Ther 5, 16-24 (2002).
126. Wang, G. et al. Apical barriers to airway epithelial cell gene transfer with amphotropic retroviral vectors. Gene Ther 9, 922-31 (2002).
127. Liu, A. et al. Cell-cell interaction in prostate generegulation and cytodifferentiation. Proc.Natl.Acad.Sci. U.S.A 94, 10705-10 (1997).
128. van Noort, M., Meeldijk, J., van der Zee, R., Destree, O. & Clevers, H. Wnt signaling controls the phosphorylation status of beta-catenin. J Biol Chem 277, 17901-5 (2002).
129. van Noort, M., van de Wetering, M. & Clevers, H. Identification of two novel regulated serines in the N terminus of beta-catenin. Exp Cell Res 276, 264-72 (2002).
130. Gazit, A. et al. Human frizzled 1 interacts with transforming Wnts to transduce a TCF dependent transcriptional response. Oncogene 18, 5959-66 (1999).
131. Ramalho-Santos, M., Yoon, S., Matsuzaki, Y., Mulligan, R. C. & Melton, D. A. "Stemness": Transcriptional Profiling of Embryonic and Adult Stem Cells. Science 298, 597-600 (2002).
132. Ivanova, N. B. et al. A Stem Cell Molecular Signature. Science, 1073823 (2002).
133. Akashi, K. et al. Transcriptional accessibility for genes of multiple tissues and hematopoietic lineages is hierarchically controlled during early hematopoiesis. Blood 101, 383-9 (2003).
134. Terskikh, A. V. et al. From hematopoiesis to neuropoiesis: evidence of overlapping genetic programs. Proc Natl Acad Sci U S A 98, 7934-9 (2001).
135. Park, I. K. et al. Differential gene expression profiling of adult murine hematopoietic stem cells. Blood 99, 488-498 (2002).
136. Bustin, S. A. Absolute quantification of mRNA using real-time reverse transcription polymerase chain reaction assays. J Mol. Endocrinol. 25, 169-193 (2000).
137. Wong, G. T., Gavin, B. J. & McMahon, A. P. Differential transformation of mammary epithelial cells by Wnt genes. Mol Cell Biol 14, 6278-86 (1994).
138. Shimizu, H. et al. Transformation by Wnt family proteins correlates with regulation of beta-catenin. Cell Growth Differ 8, 1349-58 (1997).

All publications and patents mentioned in the above specification are herein incorporated by reference. Various modifications and variations of the described method and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be within the scope of the following claims.

Additional aspects of the invention are set out in the statements below.
1. A method of detecting solid tumor stem cells, comprising;
   a) providing a tissue sample from a subject, and
   b) detecting at least one stem cell cancer marker from Tables 4-8 in said tissue sample under conditions such that the presence or absence of solid tumor stem cells in said tissue sample is determined.
2. The method of statement 1, wherein said detecting comprises determining an expression level for said at least one stem cell cancer marker.
3. The method of statement 1, wherein said detecting comprises detecting mRNA expression of said at least one stem cell cancer marker.
4. The method of statement 1, wherein said detecting comprises detecting polypeptide expression of said at least one stem cell cancer marker.
5. The method of statement 1, wherein said subject comprises a human subject.
6. The method of statement 1, wherein said tissue sample comprises tumor tissue.
7. The method of statement 1, further comprising the step of c) providing a prognosis to said subject.
8. The method of statement 1, wherein said at least one stem cell cancer marker is from Table 8.
9. The method of statement 1, wherein said at least one stem cell cancer marker is selected from the group consisting of: Bmi-1, eed, easyh1, easyh2, mf2, yy1, smarcA3, smarcA5, smarcD3, smarcE1, mllt3, FZD1, FZD2, FZD3, FZD4, FZD6, FZD7, FZD8, FZD9, FZD10, WNT2, WNT2B, WNT3, WNT5A, WNT10B, WNT16, AXIN1, BCL9, MYC, and (TCF4).
10. A method for reducing the size of a solid tumor comprising contacting cells of a solid tumor with a biologically effective amount of a composition comprising at least one agent directed against at least one stem cell cancer marker shown in Tables 4-8.
11. The method of statement 10, wherein said biologically effective amount is an amount sufficient to cause cell death of or inhibit proliferation of solid tumor stem cells in said solid tumor.
12. The method of statement 10, wherein said at least one agent is an antibody, peptide or small molecule.
13. The method of statement 12, wherein the antibody, peptide or small molecule is directed against the extracellular domain of said at least one stem cell cancer marker.
14. The method of statement 10, wherein said at least one stem cell cancer marker is selected from the group consisting of: Bmi-1, eed, easyh1, easyh2, mf2, yy1, smarcA3, smarcA5, smarcD3, smarcE1, mllt3, FZD1, FZD2, FZD3, FZD4, FZD6, FZD7, FZD8, FZD9, FZD10, WNT2, WNT2B, WNT3, WNT5A, WNT10B, WNT16, AXIN1, BCL9, MYC, and (TCF4).
15. A method for killing or inhibiting the proliferation of solid tumor stem cells comprising contacting said solid tumor stem cells with a biologically effective amount of a composition comprising at least one agent targeted to at least one stem cell cancer marker shown in Tables 4-8.
16. The method of statement 15, further comprising identifying the death of or the prevention of the growth of said solid tumor stem cells following said contacting.
17. The method of statement 16, wherein said cell death is apoptosis.
18. The method of statement 15, wherein said at least one stem cell cancer marker is selected from the group consisting of: Bmi-1, eed, easyh1, easyh2, rnf2, yy1, smarcA3, smarcA5, smarcD3, smarcE1, mllt3, FZD1, FZD2, FZD3, FZD4, FZD6, FZD7, FZD8, FZD9, FZD10, WNT2, WNT2B, WNT3, WNT5A, WNT10B, WNT16, AXIN1, BCL9, MYC, and (TCF4).
19. The method of statement 15, wherein said solid tumor stem cells express cell surface marker CD44, ESA, or B3 8. 1.
20. A method of distinguishing tumorigenic from non-tumorigenic cancer cells, comprising: detecting the presence of β-catenin in a cancer cell such that the localization of β-catenin in the cancer cell is determined to be primarily nuclear or primarily cytoplasmic.
21. The method of statement 20, further comprising identifying the cancer cell as tumorigenic if the β-catenin localization is primarily nuclear, or identifying the cancer cell as non-tumorigenic if the β-catenin localization is primarily cytoplasmic.

## Claims

1. A composition comprising at least two antibodies, wherein each of the antibodies binds and modulates the activity of at least one of FZD1, FZD2, FZD3, FZD4, FZD6, FZD7, FZD8, FZD9 or FZD10, for use in a method of treating cancer by reducing the size of a solid tumor.

2. A composition according to claim 1, wherein the two antibodies bind to and modulate the activity of more than one of FZD1, FZD2, FZD3, FZD4, FZD6, FZD7, FZD8, FZD9 or FZD10.

3. A composition according to claim 1 or claim 2, wherein the antibodies are for sequential use in the method.

4. A composition according to any of the preceding claims, wherein at least one antibody is an antibody fragment.

5. A composition according to any of the preceding claims, wherein at least one antibody is bispecific.

6. A composition according to any of the preceding claims, wherein at least one antibody is humanised.

7. A composition according to any of the preceding claims, wherein at least one antibody is monoclonal.

8. A composition according to any of the preceding claims, wherein at least one antibody is conjugated to a cytotoxic agent.

9. A composition according to any of the preceding claims, further comprising a chemotherapeutic agent.

10. A composition according to any of the preceding claims, wherein at least one antibody binds and modulates the activity of FZD2.

11. A composition according to any of the preceding claims, wherein at least one antibody binds and modulates the activity of FZD6.

12. A composition according to any of the preceding claims, wherein at least one antibody binds and modulates the activity of FZD7.

13. A composition according to any of the preceding claims, wherein at least one antibody binds and modulates the activity of FZD8.

14. A composition according to any of the preceding claims, wherein at least one antibody binds and modulates the activity of FZD4.

15. A composition according to any of the preceding claims, wherein the cancer is small cell lung cancer, non small cell lung cancer, adenocarcinoma of the lung, squamous carcinoma of the lung, pancreatic cancer, ovarian cancer, breast cancer, colorectal cancer, prostate cancer or head and neck cancer.
